# FASCICULE DE BREVET EUROPEEN

(11) **EP 1 807 697 B1**
(45) Date de publication et mention de la délivrance du brevet: **21.10.2009**
(21) Numéro de dépôt: 05812049.4
(22) Date de dépôt: 24.10.2005
(51) Int. Cl.: G01N 33/53, C12N 15/65, C12N 15/81, C12Q 1/68

(54) **SYSTÈME DOUBLE-HYBRIDE BASÉ SUR LA MISE SOUS SILENCE DE GÈNES PAR INTERFÉRENCE TRANSCRIPTIONNELLE.**
DOPPELTES HYBRIDSYSTEM AUF DER BASIS VON GEN-SILENCING DURCH TRANSKRIPTIONELLE INTERFERENZ
DOUBLE HYBRID SYSTEM BASED ON GENE SILENCING BY TRANSCRIPTIONAL INTERFERENCE

(30) Priorité: 25.10.2004 FR 0411372
(43) Date de publication de la demande: 18.07.2007
(73) Titulaire: Aventis Pharma S.A., 92160 Antony (FR)
(72) Inventeur: FOURNIER, Alain, F-92290 CHATENAY-MALABRY (FR); BEHRENS, Ralf, 21407 DEUTSCH EVERN (DE); BANDIERA, Silvio, F-75012 PARIS (FR)
(74) Mandataire: Bouvet, Philippe
(86) Numéro de dépôt international: PCT/FR2005/002646
(87) Numéro de publication internationale: WO 2006/045941

(56) Documents cités:
- WO-A-02/101011
- POORKAJ P ET AL: "Single-Step Conversion of P1 and P1 Artificial Chromosome Clones into Yeast Artificial Chromosomes" GENOMICS, ACADEMIC PRESS, SAN DIEGO, US, vol. 68, no. 1, 15 août 2000 (2000-08-15), pages 106-110, XP004437869 ISSN: 0888-7543
- KIM BYUNG-MOON ET AL: "A general method for selection and screening of coiled coils on the basis of relative helix orientation" JOURNAL OF THE AMERICAN CHEMICAL SOCIETY, vol. 124, no. 28, 17 juillet 2002 (2002-07-17), pages 8237-8244, XP002328328 ISSN: 0002-7863
- CAUSIER B ET AL: "Analysing protein-protein interactions with the yeast two-hybrid system" PLANT MOLECULAR BIOLOGY, NIJHOFF/JUNK, THE HAGUE, NL, vol. 50, no. 6, décembre 2002 (2002-12), pages 855-870, XP002328327 ISSN: 0167-4412

## Description

La présente invention concerne un nouveau système double-hybride basé sur la mise sous silence de gènes par interférence transcriptionnelle. Elle est également relative à l'utilisation de ce système pour l'identification de composés biologiquement actifs agissant sur l'interaction protéine-protéine.

Le système double-hybride (Y2H : « Yeast Two hybrid system ») a été utilisé de manière extensive chez la levure pour l'identification d'interactions protéine-protéine (cf. notamment : Chien, C. T. *et al.,* 1991, Fields, S., et O. Song, 1989, Cagney, G. *et al.,* 2000, 6. Causier, B., et B. Davies, 2002, Coates, P. J., et P. A. Hall. 2003).

La technique du double-hybride repose sur le fait qu'un domaine de liaison à 1'ADN et un domaine d'activation de la transcription contenu dans des protéines chimériques distinctes peuvent activer la transcription d'un gène lorsque ces deux domaines sont suffisamment proches l'un de l'autre. Une version couramment utilisée du système double-hybride chez la levure tire profit des propriétés de la protéine GAL4p de la levure *Saccharomyces cerevisiae* (Fields, S., et O. Song, 1989).

La protéine GAL4p active entre autres la transcription du gène *GAL1* nécessaire à l'utilisation du galactose par la levure et est constituée de deux domaines : un domaine N-terminal qui lie spécifiquement une séquence d'ADN (UAS_{G} pour upstream activated sequence for the yeast *GAL* genes) et un domaine C-terminal contenant une région acide permettant l'activation transcriptionnelle.

Ce premier système double-hybride fait intervenir trois acteurs : deux protéines de fusion et une construction dans laquelle un gène rapporteur est placé sous le contrôle du promoteur du gène *GAL1* (Pgal1). Une première protéine fusionnée au domaine de liaison à l'ADN (DBD pour DNA binding domaine) de la protéine GAL4p est exprimée chez une levure *S. cerevisiae* portant la construction décrite ci-dessus. Une deuxième protéine fusionnée au domaine d'activation de la transcription (AD pour activation domaine) de GAL4p est co-exprimée dans cette même levure. En cas d'interaction des deux protéines de fusion, le rapprochement du domaine d'activation de la transcription GAL4-AD et du domaine de liaison à l'ADN permet la reconstitution d'un facteur de transcription GAL4p fonctionnel, ce qui permet l'induction de l'expression du gène rapporteur placé sous le contrôle de Pgall.

Le choix comme gène rapporteur d'un gène essentiel à la viabilité de la levure (comme par exemple le gène *HIS3*) permet un criblage aisé des souches pour lesquelles le gène rapporteur est exprimé et donc pour lesquelles une interaction entre les deux protéines hybrides a lieu. Les souches pour lesquelles une telle interaction n'a pas lieu ne peuvent pas activer Pgal1 et ne peuvent pas croître si le gène rapporteur est un gène essentiel à la viabilité de la levure.

Plus récemment, il a été développé des systèmes dits double-hybrides inverse (rY2H : « reverse Yeast Two hybrid system »). Dans ces systèmes, l'activation du gène rapporteur est induite en l'absence d'une interaction protéine-protéine, ce qui est utile notamment pour l'identification de protéines mutantes ayant perdu la capacité d'interagir avec leur partenaire ainsi que pour l'identification de molécules inhibant spécifiquement l'interaction protéine-protéine.

Young *et al* ont ainsi mis au point un test de criblage à haut débit basé sur un système double-hybride inverse afin d'identifier des modulateurs de canaux calciques (Young, K. *et al.,* 1998). Ce système rY2H exploite les propriétés du gène *CYH2.* Le gène *CYH2* code une protéine ribosomale L29, un composant de la sous-unité ribosomale 60S, qui confère une sensibilité vis-à-vis du cycloheximide aux cellules (Kaufer, N. F. *et al.,* 1983). Une cassette rapporteuse Pgal1-*CYH2* est introduite dans une souche de levure résistante au cycloheximide présentant un gène *CYH2* endogène muté. L'allèle *CYH2* de type sauvage étant dominant, la croissance de la cellule rapporteuse est inhibée sur un milieu contenant du cycloheximide lorsque l'interaction entre les deux protéines se produit (et donc lorsque la cassette rapporteuse est transcrite). Une interruption de l'interaction atténue l'effet toxique du cycloheximide et permet à la cellule rapporteuse de croître sur un milieu contenant du cycloheximide (Leanna, C. A., and M. Hannink. 1996).

Toutefois, des problèmes associés à la stabilité des ARNm et de la protéine ainsi que des différences au niveau de l'activité des protéines CYH2p de type sauvage et mutées rendent ce système complexe à utiliser et assez peu sensible pour la détection de dissociation d'interactions protéine-protéine (interaction Y2H inverses).
Ce système présente en outre le désavantage d'être relativement difficile à utiliser pour obtenir une sensibilité maximale car sa réponse vis-à-vis d'interactions Y2H inverses dépend de plusieurs molécules. Ainsi la compétition entre CYH2p de type sauvage et mutant au niveau de l'incorporation dans les ribosomes affecte la sensibilité du rapporteur. En outre, des fluctuations au niveau de la capture du cycloheximide et de sa réactivité sur les ribosomes influencent très probablement la capacité de réponse du système.

Un autre système double-hybride inverse, basé sur l'utilisation du gène rapporteur *URA3* en combinaison avec son substrat protoxique : l'acide 5-fluoroorotique (5-FOA) a été également décrit (Huang, J., et S. L. Schreiber. 1997, Vidal, M., *et al.,* 1996, WO9632503). Dans ce système, l'induction de l'expression de la protéine URA3p inhibe la croissance cellulaire car le 5-FOA est alors transformé en son analogue toxique : le 5-fluoro-UTP. Une inactivation d'*URA3* permet au contraire une croissance cellulaire car le 5-FOA n'est alors plus métabolisé et les cellules utilisent l'uracile fourni dans le milieu. Ce système a été utilisé pour identifier des protéines mutantes ayant perdu leur capacité à interagir avec leur partenaire normal (Burke, T. W. *et al.,* 2001 ; Daros, J. A. *et al.,* 1999 ; Puthalakath, H., *et al.,* 1999).

La configuration et le mécanisme de ce système sont très similaires à ceux du système double-hybride inverse décrit ci-dessus (Young, K. *et al.,* 1998). Par conséquent, ce système présente les inconvénients relevés ci-dessus. En outre le système basé sur l'utilisation de 5-FOA présente le désavantage de générer des faux positifs liés au système lui même. En effet, la croissance cellulaire est liée à l'inactivation de l'*URA3* de type sauvage. Par conséquent, des composés inhibant la protéine URA3p permettent une croissance cellulaire. Ces composés génèrent donc un signal faux positif lors de l'utilisation du système pour l'identification de molécules inhibant spécifiquement l'interaction protéine-protéine.

Ce système présente également le désavantage de l'utilisation de 5-FOA qui est un agent chimique relativement coûteux.

Enfin, un système à relais comprenant une cascade de deux gènes rapporteurs et baptisé « Split-Hybrid » a également été décrit (WO9526400 et WO9813502). Ce système rY2H a été utilisé pour identifier des mutations dans la protéine CREB qui interrompent son association avec CBP (Crispino, J. D. *et al*., 1999). Dans ces systèmes, une interaction double-hybride active l'expression d'une première protéine rapporteuse qui à son tour contrôle l'expression d'un deuxième gène rapporteur utilisé pour la sélection de croissance. Ce système est toutefois complexe à utiliser et assez peu sensible pour la détection d'interactions Y2H inverses.

Les systèmes décrits ci-dessus dépendent donc de l'activité de plusieurs molécules (deux gènes rapporteurs, un gène rapporteur et une substance toxique, un gène rapporteur sauvage et un muté). Ceci augmente leur complexité de mise en oeuvre notamment lorsqu'une sensibilité maximale est recherchée. Il serait souhaitable de disposer d'un système à la fois plus simple, plus économique tout en disposant d'une bonne sensibilité.

### Description générale de l'invention

La présente invention concerne un nouveau système double-hybride basé sur la mise sous silence de gène par interférence transcriptionnelle. Le système double-hybride selon la présente invention est simple, ne requiert pas d'addition au milieu de substances toxiques et peut être utilisé sans qu'une intégration dans le génome ne soit nécessaire.

Un premier objet de l'invention concerne une cellule comportant une construction d'ADN d'interférence, ladite construction comprenant :
- Un gène rapporteur placé sous le contrôle d'un premier promoteur,
- Un ou plusieurs promoteur(s) inductible(s), dit(s) promoteur(s) d'interférence, choisi(s) et positionné(s) de manière à ce que son (leur) activation entraîne une interférence transcriptionnelle du premier promoteur, conduisant à une diminution détectable de l'expression du gène rapporteur,
ladite cellule exprimant en outre :
- Une première protéine chimère (Y-AD) constituée d'un domaine d'activation de la transcription (AD) fusionné à une protéine Y susceptible d'interagir avec une protéine partenaire X,
- Une deuxième protéine chimère (X-DBD) constituée d'un premier domaine liant l'ADN (DBD) fusionné à un deuxième domaine constitué par une protéine X susceptible d'interagir avec la protéine Y, l'interaction des deux protéines chimères X-DBD et Y-AD aboutissant à la constitution d'un facteur de transcription fonctionnel activant le ou les promoteur(s) d'interférence.

Selon un mode de mise en oeuvre, la présente invention a pour objet une cellule telle que décrite ci-dessus dont le promoteur régulant l'expression du gène rapporteur est un promoteur inductible, dont la protéine Y est susceptible d'interagir avec deux protéines partenaires X et Z, la cellule exprimant en outre une troisième protéine chimère (Z-DBD) constituée d'un domaine liant l'ADN (DBD), fusionné à un deuxième domaine constitué par une protéine Z susceptible d'interagir avec la protéine Y, l'interaction des deux protéines chimères Z-DBD et Y-AD aboutissant à la constitution d'un facteur de transcription fonctionnel activant l'expression du gène rapporteur.

Dans un mode de mise en oeuvre préféré, la présente invention a pour objet une cellule telle que définie ci-dessus dont le promoteur inductible régulant l'expression du gène rapporteur comprend une séquence susceptible d'interagir avec le domaine liant l'ADN (DBD) de la protéine chimère (Z-DBD).

L'invention a également pour objet une cellule telle que définie ci-dessus dont le promoteur régulant l'expression du gène rapporteur est un promoteur constitutif.

L'invention concerne également une cellule telle que décrite ci-dessus **caractérisée en ce qu**'il s'agit d'une cellule hôte transformée ou transfectée par la construction d'ADN d'interférence et par des constructions d'ADN codant pour les protéines chimères, l'ensemble de ces constructions étant portées par un ou plusieurs vecteurs non intégratifs.

Un autre aspect de l'invention concerne une cellule telle que définie ci-dessus **caractérisée en ce qu**'il s'agit d'une cellule hôte transformée ou transfectée par des constructions d'ADN codant pour les protéines chimères, ces constructions étant portées par un ou plusieurs vecteurs non intégratifs, et en ce que la construction d'ADN d'interférence est intégrée au génome de la cellule.

L'invention concerne également une cellule telle que définie ci-dessus dont la construction d'ADN d'interférence et les constructions d'ADN codant pour les protéines chimères sont intégrées au génome de la cellule.

Dans un mode de mise en oeuvre préféré, la présente invention concerne une cellule telle que décrite ci-dessus dont la construction d'ADN d'interférence est intégrée à un locus exempt d'activités de transcription génomiques perturbatrices.

L'invention concerne également une cellule telle que définie ci-dessus **caractérisée en ce qu**'elle est choisie dans le groupe constitué par les cellules de mammifères, d'insectes, de plantes et de levures.

L'invention a également pour objet une cellule telle que décrite ci-dessus **caractérisée en ce qu**'il s'agit de cellules de levures.

Dans un mode de mise en oeuvre préféré, l'invention est relative à une cellule telle que définie ci-dessus **caractérisée en ce qu**'il s'agit de levures de l'espèce *Saccharomyces cerevisiae, Schizosaccharomyces pombe, Kluyveromyces lactis, Pichia pastoris, Saccharomyces carlsbergensis* ou *Candida albicans.*

L'invention concerne aussi une cellule telle que définie ci-dessus avec au moins un promoteur d'interférence positionné en aval du gène rapporteur et du premier promoteur et dans une orientation opposée à ce dernier (DI).

L'invention concerne en outre une cellule telle que décrite ci-dessus avec au moins un promoteur d'interférence positionné en aval du gène rapporteur et du premier promoteur et dans la même orientation que ce dernier (nDI).

L'invention concerne également une cellule telle que définie ci-dessus avec au moins un promoteur d'interférence positionné en amont du gène rapporteur et du premier promoteur et dans la même orientation que ce dernier (UI).

Un autre aspect de l'invention concerne une cellule telle que décrite ci-dessus avec au moins un promoteur d'interférence positionné de part et d'autre du premier promoteur et du gène rapporteur, le ou les promoteurs d'interférences situés en aval du premier promoteur et du gène rapporteur présentant une orientation convergente par rapport au premier promoteur et le ou les promoteurs d'interférence positionnés en amont du premier promoteur et du gène rapporteur présentant une orientation identique à celle du premier promoteur (UDI).

L'invention concerne en outre une cellule telle que décrite ci-dessus avec au moins un promoteur d'interférence positionné de part et d'autre du premier promoteur et du gène rapporteur, le ou les promoteurs d'interférences situés en aval du premier promoteur et du gène rapporteur présentant une orientation en tandem par rapport au premier promoteur et le ou les promoteurs d'interférence positionnés en amont du premier promoteur et du gène rapporteur présentant une orientation identique à celle du premier promoteur (nUDI).

Dans un mode de mise en oeuvre préféré, l'invention concerne une cellule telle que définie ci-dessus et dont le gène rapporteur est un gène essentiel à la survie de la cellule.

L'invention est également relative à une cellule telle que décrite ci-dessus et dont le gène rapporteur est un gène indispensable au métabolisme primaire, à la division cellulaire, à la synthèse protéique, à la synthèse d'ADN ou la synthèse d'ARN.

L'invention a également pour objet une cellule telle que définie ci-dessus dont le gène rapporteur n'est pas à lui seul essentiel à la survie de la cellule, mais est essentiel à la survie de la cellule lorsque sa transcription est inhibée en association avec un ou plusieurs gènes rapporteurs du même type, dont l'expression est contrôlée ou non par le système d'interférence transcriptionnelle.

Dans un mode de mise en oeuvre préféré, l'invention concerne une cellule telle que décrite ci-dessus dont le ou les promoteurs inductibles d'interférence comprennent une séquence susceptible d'interagir avec le domaine liant l'ADN (DBD) de la protéine chimère (X-DBD).

L'invention concerne également une cellule telle que définie ci-dessus dont le ou les promoteurs inductibles d'interférence comprennent une séquence susceptible d'interagir avec une protéine possédant un domaine de liaison à l'ADN (DBD) choisie dans le groupe constitué par : GAL4 UAS, LexAop, cIop et TetRop et que le domaine de liaison à l'ADN de la protéine chimère X-DBD est le DBD correspondant (respectivement GAL4, LexA, cI ou TetR).

Un autre aspect de l'invention concerne une cellule telle que décrite ci-dessus dont le domaine d'activation de la transcription (AD) de la protéine chimère Y-AD est choisi dans le groupe constitué par les domaines d'activation de la transcription des protéines suivant : B42, VP16 et GAL4p.

Dans un mode de mise en oeuvre préféré, l'invention a pour objet une cellule telle que définie ci-dessus dont la construction d'ADN d'interférence est bordée à ses extrémités par un ou plusieurs terminateurs de transcription unidirectionnels ou bidirectionnels.

Un autre aspect de l'invention concerne un procédé d'identification d'un composé inhibant l'interaction d'une première protéine X, avec une deuxième protéine Y comprenant les étapes suivantes :
a) cultiver des cellules telles que définies ci-dessus,
b) incuber lesdites cellules en présence du composé à tester,
c) comparer l'expression du gène rapporteur en présence et en l'absence dudit composé, une augmentation de l'expression du gène rapporteur étant l'indication que le composé à tester est un inhibiteur de l'interaction de la protéine X avec la protéine partenaire Y, exprimées par les cellules cultivées.

L'invention concerne en outre l'utilisation de cellules telles que définies ci-dessus pour l'identification de composés inhibant l'interaction protéine-protéine.

L'invention a également pour objet l'utilisation de cellules telles que décrite ci-dessus pour le criblage de banques d'ADNc ou de banques de peptides afin d'identifier des peptides ou des facteurs protéiques abrogeant spécifiquement une interaction protéine-protéine.

Un autre aspect de l'invention concerne un kit pour la mise en place d'un système double-hybride comprenant:
- Une première construction d'ADN comprenant :
   - Un gène rapporteur placé sous le contrôle d'un premier promoteur,
   - Un ou plusieurs promoteurs inductibles, choisi(s) et positionné(s) de manière à ce que son (leur) activation entraîne une interférence transcriptionnelle du premier promoteur, conduisant à une diminution détectable de l'expression du gène rapporteur,
- Une deuxième construction d'ADN codant pour :
   - Une première protéine chimère (Y-AD) constituée d'un domaine d'activation de la transcription (AD) fusionné à une protéine partenaire Y susceptible d'interagir avec une protéine partenaire X,
- Une troisième construction d'ADN codant pour :
   - Une deuxième protéine chimère (X-DBD) constituée d'un premier domaine liant l'ADN (DBD) fusionné à un deuxième domaine constitué par une protéine X susceptible d'interagir avec la protéine Y, l'interaction des deux protéines chimères X-DBD et Y-AD aboutissant à la constitution d'un facteur de transcription fonctionnel activant le ou les promoteurs d'interférence lorsque les deux protéines chimères sont exprimées dans une cellule hôte.

L'invention concerne également un kit tel que défini ci-dessus **caractérisé en ce que** le promoteur régulant l'expression du gène rapporteur est un promoteur inductible, en ce que la protéine Y est susceptible d'interagir avec deux protéines partenaires X et Z, et en ce que le dit kit comprend une quatrième construction d'ADN codant pour une troisième protéine chimère (Z-DBD) constituée d'un domaine liant l'ADN (DBD), fusionné à un deuxième domaine constitué par une protéine Z susceptible d'interagir avec la protéine Y, l'interaction des deux protéines chimères Z-DBD et Y-AD aboutissant à la constitution d'un facteur de transcription fonctionnel activant l'expression du gène rapporteur.

Un autre aspect de l'invention concerne un procédé d'identification d'un composé inhibant l'interaction d'une première protéine X, avec une deuxième protéine Y, mais n'inhibant pas ou moins l'interaction entre la protéine Y et une troisième protéine Z comprenant les étapes suivantes :
a) cultiver des cellules telles que définies ci-dessus,
b) incuber lesdites cellules en présence du composé à tester,
c) comparer l'expression du gène rapporteur en présence et en l'absence dudit composé, une augmentation de l'expression du gène rapporteur étant l'indication que le composé à tester est un inhibiteur de l'interaction de la protéine X, avec la protéine partenaire Y, mais que ce produit n'inhibe pas ou moins l'interaction entre la protéine Y et la protéine Z.

L'invention concerne enfin un vecteur d'intégration de levure contenant deux fragments homologues aux régions amont et aval, de la phase ouverte de lecture du gène *URA3* de *S. cerevisiae* et permettant une intégration par recombinaison homologue au niveau du locus *URA3* d'une séquence insérée entre ces deux fragments.

### Description détaillée de l'invention

La présente invention concerne un nouveau système double-hybride basé sur la mise sous silence de gène par interférence transcriptionnelle. Le système double-hybride selon la présente invention est simple, ne requiert pas d'addition au milieu de substances toxiques et peut être utilisé sans qu'une intégration dans le génome ne soit nécessaire.

L'interférence transcriptionnelle est définie comme la perturbation de l'activité d'un premier promoteur lorsqu'un deuxième promoteur est activé. Ainsi, l'activité de transcription d'un promoteur réduit une transcription initiée au niveau d'un autre promoteur. L'interférence de transcription a été décrite par exemple chez la levure (Greger, I. H., and N. J. Proudfoot. 1998, Springer, C.O *et al.,* 1997, Peterson, J. A., and A. M. Myers, 1993, Puig, S. et al., 1999, Martens *et al*., 2004).

Un premier objet de l'invention concerne des cellules porteuses d'une ou plusieurs constructions permettant l'expression d'un gène rapporteur en l'absence d'une interaction protéine-protéine particulière alors que lorsque cette interaction a lieu, il est constaté une diminution détectable de l'expression dudit gène rapporteur.

Plus particulièrement, les cellules selon la présente invention comporte une ou plusieurs constructions d'ADN, dites constructions d'interférence, ladite ou lesdites constructions comprenant :
- Un gène rapporteur placé sous le contrôle d'un premier promoteur,
- Un ou plusieurs promoteur(s) inductible(s), dit(s) promoteur(s) d'interférence, choisi(s) et positionné(s) de manière à ce que son (leur) activation entraîne une interférence transcriptionnelle du premier promoteur, conduisant à une diminution détectable de l'expression du gène rapporteur,
ladite cellule exprimant en outre :
- Une première protéine chimère (Y-AD) constituée d'un domaine d'activation de la transcription (AD) fusionné à une protéine Y susceptible d'interagir avec une protéine partenaire X,
- Une deuxième protéine chimère (X-DBD) constituée d'un premier domaine liant l'ADN (DBD) fusionné à un deuxième domaine constitué par une protéine X susceptible d'interagir avec la protéine Y, l'interaction des deux protéines chimères X-DBD et Y-AD aboutissant à la constitution d'un facteur de transcription fonctionnel activant le ou les promoteur(s) d'interférence.

Dans un premier mode de mise en oeuvre, les cellules selon la présente invention sont des cellules hôte transformées ou transfectées par la construction d'ADN comprenant le gène rapporteur (construction d'interférence) et elles sont également transformées ou transfectées par deux constructions d'ADN codant pour chacune des deux protéines chimères. Les trois constructions peuvent être portées par le même vecteur d'ADN ou se trouver sur deux ou trois vecteurs différents. Toutes les combinaisons sont envisageables : les trois constructions sur le même vecteur, les trois constructions sur trois vecteurs différents, les deux constructions codant pour les deux protéines chimères sur le même vecteur alors que la troisième construction se trouve sur un deuxième vecteur, une construction codant pour une des deux protéines chimères et construction d'interférence sur le même vecteur alors que la construction codant pour la deuxième protéine chimère trouve sur un deuxième vecteur. Dans ce premier mode de mise en oeuvre, les vecteurs sont des vecteurs non intégratifs.

Dans un second mode de mise en oeuvre de l'invention, la construction d'interférence est intégrée au génome des cellules et ces dernières sont transformées ou transfectées par deux constructions d'ADN codant pour chacune des deux protéines chimères. Les constructions d'ADN codant pour les protéines chimères peuvent être portées par le même vecteur d'ADN ou se trouver sur deux vecteurs différents, ce ou ces vecteurs étant des vecteurs non intégratifs.

Dans un troisième mode de mise en oeuvre de l'invention, la construction d'interférence ainsi que les constructions d'ADN codant pour chacune des deux protéines chimères sont intégrées au génome des cellules. Il est également possible que seule une des deux constructions codant pour une des deux protéines chimères soit intégrée au génome et que la deuxième construction soit portée par un vecteur non intégratif. Enfin, dans un dernier mode de mise en oeuvre, la construction d'interférence et une des deux constructions codant pour une des deux protéines chimères sont portées par des vecteurs non intégratifs alors que la construction codant pour la deuxième protéines chimères est intégrée au génome des cellules.

Un gène rapporteur est habituellement défini comme un gène codant pour un produit aisément détectable. Dans un mode de mise en oeuvre préférentiel, le gène rapporteur utilisé dans le cadre de la présente invention est un gène dont l'expression est essentielle à la survie de la cellule. Il peut s'agir par exemple d'un gène indispensable au métabolisme primaire, à la division cellulaire, à la synthèse protéique (comme les ribosomes), à la synthèse d'ADN ou la synthèse d'ARN etc...

A titre d'exemple de ce type de gène on peut citer tous les gènes décrits comme essentiels pour la survie de la levure, c'est à dire environ 1/6^{ème} des gènes que constituent son génome (Winzeler E. A *et al.,* 1999).

Ainsi il peut s'agir d'un gène impliqué dans le métabolisme primaire et indispensable à ce dernier ou un gène indispensable à la division cellulaire. Plus particulièrement, il s'agit d'un gène codant pour une enzyme impliquée dans la biosynthèse d'un métabolite essentiel pouvant être ajouté ou retiré du milieu de culture.

A titre d'exemple de ce type de gène on peut citer les gènes *HIS3, URA3, LEU2, LYS2, TRP1, ADE2, MET15 et ARG4* largement utilisés chez la levure *S. cerevisiae* car facilement complémentable par apport de l'acide aminé ou de la base d'acide nucléique correspondante dans le milieu de culture des levures. D'autres marqueurs basé sur le métabolisme peuvent être utilisés, on peut citer à titre d'exemple le gène *HIS5* (gènes de la levure *S. pombe*), les gènes *URA3* et *LEU2* (gènes la levure *K. lactis*), *URA3* (gène de la levure *C. albicans*), *LEU2* (gène de la levure *A. gossypii*).

Il peut également s'agir de gènes impliqués dans le métabolisme primaire et non essentiels à ce dernier, ou des gènes non essentiels à la division cellulaire lorsqu'ils sont utilisés seuls. Par contre si leur transcription est inhibée en association avec un ou plusieurs gènes rapporteurs du même type, l'association des effets peut conduire à l'inhibition d'une fonction essentielle à la survie de la levure. Il peut s'agir par exemple d'un ensemble de gènes dont les rôles associés conduisent à une fonction indispensable au métabolisme primaire, à la division cellulaire, à la synthèse protéique (comme les ribosomes), à la synthèse d'ADN ou la synthèse d'ARN etc...

A titre d'exemple de ce type de gène on peut citer tous les gènes décrits comme non essentiels pour la survie de la levure (c'est-à-dire environ 5/6^{ème} des gènes que constituent son génome (Winzeler E. A *et al*., 1999)) qui en association avec un ou plusieurs gènes du même type deviennent alors essentiels.

Toutefois, d'autres gènes rapporteurs peuvent également être utilisés dans le cadre de la présente invention. Il peut être cité à titre d'exemple, le gène codant pour l'enzyme CAT (chloramphenicol acetyltransferase), le gène *luc* codant pour la luciférase, les gènes codant pour une protéine fluorescentes comme la GFP (pour « Green Flourescent Protein »), CFP (pour « Cyan Flourescent Protein »), YFP (pour « Yellow Flourescent Protein ») ou la RFP (« Red Fluorescent Protein »), la béta-galactosidase, la béta-lactamase, la béta-glucuronidase.

Il peut également s'agir de marqueur conférant une résistance à un produit toxique : le gène kan du transposon Tn903 (conférant une résistance à la généticine (G418)), le gène *ble* du transposon Tn5 (conférant une résistance à la phléomycine), le gène *CYHr* (conférant une résistance au cycloheximide), *pat* (gène de *S. viridochromogenes* conférant une résistance au bialaphos), *nat1* (gène de *S. noursei* conférant une résistance à la nurseothricine), *hph* (gène de *E.coli,* conférant une résistance à l'hygromycine B). Tous ces gènes sont fonctionnels notamment chez la levure (Gutherie and Fink, 2002).

Le gène rapporteur est placé sous le contrôle d'un premier promoteur. Dans un mode de réalisation préférée, ce premier promoteur est un promoteur constitutif. Un promoteur constitutif, comme il est communément admis, est un promoteur permettant une expression relativement indépendante des conditions environnantes.

A titre d'exemple, chez la levure le gène rapporteur peut être exprimé sous le contrôle du promoteur mutant constitutif ADH1(700) (promoteur du gène *ADH1* (Padh1) muté) (Ruohonen *et al.,* 1995). Toutefois, d'autres promoteurs constitutifs peuvent également être utilisés chez la levure dans le cadre de la présente invention. Il peut être cité à titre d'exemple le promoteur TPI (Alber and Kawasaki, 1982), TEF1, TDH3, KEX2 (Nacken *et al.,* 1996) et ACT1 (Ernst, 1986).

En ce qui concerne les cellules de mammifères, on peut citer à titre d'exemple le promoteur CMV (pour human cytomegalovirus (CMV) immediate-early enhancer/promoter) (Foecking and Hofstetter, 1986) (Kronman *et al.,* 1992), le promoteur EF-1 α (Mizushima and Nagata, 1990), le promoteur SV40 (pour Simian Virus 40 early enhancer/promoter) (Das et al., 1985), le promoteur UB (promoteur du gène humain de l'ubiquitin C (hUbC)) (Nenoi *et al*., 1996; Schorpp *et al.,* 1996), le promoteur RSV LTR (pour Rous Sarcoma Virus Long Terminal Repeat) du virus du sarcome de Rous (Yamamoto et al., 1980).

Le ou les promoteurs dits « promoteur d'interférence » sont des promoteurs inductibles. Ce ou ces derniers sont choisis et positionnés de manière à ce que leur activation entraîne une interférence transcriptionnelle du premier promoteur, conduisant à une diminution détectable de l'expression du gène rapporteur.

Les promoteurs d'interférences pouvant être mis en oeuvre dans le cadre de la présente invention sont les promoteurs inductibles dont l'activation est induite par un facteur de transcription interagissant via un domaine de liaison à l'ADN (DBD) avec une séquence dudit promoteur (séquence d'ADN lié par le DBD), ledit facteur de transcription étant capable d'initier la transcription gouvernée par le promoteur. Ainsi les promoteurs d'interférences pouvant être mis en oeuvre dans le cadre de la présente invention sont tous les promoteurs convenant à la mise en place d'un système double-hybride chez l'organisme considéré.

De façon préféré, les promoteurs d'interférence selon la présente invention comprennent une séquence susceptible d'interagir avec une protéine possédant un domaine de liaison à l'ADN (DBD) (séquence d'ADN lié par le DBD (UAS)). Plus particulièrement, le ou les promoteurs inductibles d'interférence comprennent une séquence susceptible d'interagir avec le domaine liant l'ADN (DBD) de la protéine chimère (X-DBD).

Il s'agit par exemple des promoteurs Pgal1, Pgal10, Pga15 Pga180, Pmel1, Pga12, Pgal7, Pgal3, Pgcy1, Plth1, Ppcl10 et Pfur4 (Ren *et al.,* 2000; Svetlov and Cooper, 1995), inductibles par le facteur de transcription GAL4p (dans une cellule sauvage, le facteur de transcription GAL4p est impliqué notamment dans l'expression des gènes induits par le galactose). De manière préférée, le promoteur inductible mise en oeuvre est le promoteur Pgal1.

Cependant, d'autres modes de mises en oeuvres de l'invention utilisant d'autres systèmes d'expression inductible rentrent également dans le cadre de la présente invention. La table 1 présente un certain nombre de systèmes inductibles déjà utilisés dans des systèmes double-hybrides. Les promoteurs inductibles de ces systèmes décrits dans la littérature constituent également des promoteurs d'interférences au sens de la présente invention. Afin d'obtenir un système double-hybride fonctionnelle, toutes les combinaisons possibles entre les DBDs et les ADs dans la table 1 sont envisageables.

**Table 1 :**

| **Séquence d'ADN lié par le DBD (UAS, opérateur)** | **Domaine de liaison à l'ADN (DBD)** | **Domaine d'activation de la transcription (AD)** | **Référence bibliographique** |
|---|---|---|---|
| LexAop | LexA | B42 | (Gyuris et al., 1993) |
| LexAop | LexA | VP16 | (Vojtek and Hollenberg, 1995) |
| GAL4 UAS | GAL4 | GAL4 | (Elledge and Spottswood, 1991; Fields and Song, 1989) |
| cIop | cI | B42 | (Serebriiskii et al., 1999) |
| TetRop | TetR | B42 | (Xu et al., 1997) |

Le ou les promoteurs d'interférence inductible sont positionnés de manière à ce que leur activation entraîne une interférence transcriptionnelle du premier promoteur, conduisant à une diminution détectable de l'expression du gène rapporteur.

Le ou les promoteurs d'interférence peuvent ainsi être positionnés en aval du premier promoteur et du gène rapporteur, il s'agit alors d'interférence aval car l'activation du promoteur d'interférence situé en aval interfère avec l'activité du premier promoteur situé plus en amont.

Dans le cadre d'une interférence aval, le promoteur d'interférence peut cependant présenter deux orientations possibles. Une première orientation correspond à une orientation convergente par rapport au premier promoteur, cette configuration a été dénommée interférence aval (ou DI pour « Downstream Interference »). Le promoteur d'interférence peut également présenter une orientation en tandem par rapport au premier promoteur, cette deuxième configuration a été dénommée interférence aval non-sens (ou nDI pour «non-sense Downstream Interference ») qui montre également une inhibition de l'expression du gène rapporteur (cf la partie exemple).

Le ou les promoteurs d'interférence peuvent également être positionnés en amont du premier promoteur et du gène rapporteur, il s'agit alors d'interférence amont car l'activation du promoteur d'interférence situé en amont interfère avec l'activité du premier promoteur situé plus en aval. Le promoteur d'interférence présente alors une orientation identique à celle du premier promoteur (les deux promoteurs ont donc une configuration en tandem), cette configuration a été dénommée interférence amont (ou UI pour « Upstream Interference »).

Une autre configuration possible est la combinaison des deux configurations décrites ci-dessus (système UI et DI). Dans ce cas, au moins un promoteur d'interférence inductible est positionné de part et d'autre du premier promoteur et du gène rapporteur. Il s'agit alors d'interférence amont-aval car l'activation du ou des promoteur(s) d'interférence situé(s) en amont interfère(nt) avec l'activité du premier promoteur situé plus en aval et l'activation du ou des promoteur(s) d'interférence situé(s) en aval interfère(nt) avec l'activité du premier promoteur situé plus en amont.

Le ou les promoteurs d'interférences situés en aval du premier promoteur et du gène rapporteur présentent une orientation convergente par rapport au premier promoteur (cf. le système DI ci-dessus). Le ou les promoteurs d'interférence positionné en amont du premier promoteur et du gène rapporteur présente une orientation identique à celle du premier promoteur (les deux promoteurs ont donc une configuration en tandem) (cf. système UI ci-dessus).

Cette configuration a été dénommée interférence amont-aval (ou UDI pour « Upstream-Downstream Interference »).

Une autre configuration possible est la combinaison de deux configurations décrites ci-dessus (système UI et nDI). Dans ce cas, au moins un promoteur d'interférence inductible est positionné de part et d'autre du premier promoteur et du gène rapporteur. Il s'agit alors d'interférence amont-aval car l'activation du ou des promoteur(s) d'interférence situé(s) en amont interfère(nt) avec l'activité du premier promoteur situé plus en aval et l'activation du ou des promoteur(s) d'interférence situé(s) en aval interfère(nt) avec l'activité du premier promoteur situé plus en amont.

Le ou les promoteurs d'interférences situés en aval du premier promoteur et du gène rapporteur présentent une orientation en tandem par rapport au premier promoteur (cf. le système nDI ci-dessus). Le ou les promoteurs d'interférence positionné(s) en amont du premier promoteur et du gène rapporteur présente une orientation identique à celle du premier promoteur (cf. système UI ci-dessus), les trois promoteurs ont donc une configuration en tandem.
Cette configuration a été dénommée interférence non sens amont-aval (ou nUDI pour «non-sensé Upstream-Downstream Interference »).

Dans le cas d'une construction de type UI, une activation du promoteur d'interférence conduira à une réduction de l'activité du premier promoteur en raison d'une interférence de transcription. L'activité du promoteur d'interférence en amont pourrait cependant encore permettre de produire un transcrit sens du gène rapporteur.

Afin d'empêcher la traduction de ce transcrit sens fonctionnel, une courte phase ouverte de lecture suivie d'un ou plusieurs (par exemple 2 ou 3) codons stop est préférentiellement insérée entre le promoteur d'interférence et le premier promoteur. Une interférence de transcription dans un système UI produit alors un messager bicistronique à partir duquel seul la première courte phase ouverte de lecture est traduite.

Ceci est également le cas dans une configuration de type UDI et nUDI. En effet, dans le cas d'une construction de type UDI ou nUDI, une activation du ou des promoteurs d'interférence situé(s) en amont du premier promoteur conduira à une réduction de l'activité du premier promoteur en raison d'une interférence de transcription. L'activité du promoteur d'interférence en amont pourrait cependant encore permettre de produire un transcrit sens du gène rapporteur.

Afin d'empêcher la traduction de ce transcrit sens fonctionnel, une courte phase ouverte de lecture suivie d'un ou plusieurs codons stop est préférentiellement insérée entre le promoteur d'interférence et le premier promoteur. Une interférence de transcription dans un système UDI ou nUDI produit alors un messager bicistronique à partir duquel seul la première courte phase ouverte de lecture est traduite.

Préférentiellement, les constructions d'interférences décrites ci-dessus sont bordées à leurs extrémités par des terminateurs de transcription bidirectionnels. Tout terminateur de transcription fonctionnel et bidirectionnel peut être utilisé pour border les constructions selon l'invention. A titre d'exemple il peut être utilisé le terminateur de transcription du gène *CYC1* (Tcyc1) (décrit par Osbome, B. I., et L. Guarente., 1989) ainsi que le terminateur du gène *ADH1* (Tadh1) (décrit par Irniger, S. *et al.,* 1991).

Les terminateurs de transcription bidirectionnels arrêtent la transcription d'une manière bidirectionnelle. Ceci empêche l'extension de la transcription dirigée par le premier promoteur ou le ou les promoteurs d'interférence au-delà de la construction et protège également le système de l'éventuelle influence d'activités de transcription situées en dehors de la construction.

Dans le même but, il est également possible de combiner des terminateurs unidirectionnels dans une configuration qui bloque la transcription dans les deux sens (en combinant deux terminateurs unidirectionnels bloquant la transcription dans une orientation divergente ou convergente afin d'obtenir un terminateur bidirectionnel). Il peut être cité à titre d'exemple de terminateurs unidirectionnels les terminateurs suivants : Tpgk1(Picard et al., 1990), Ttef (de *A. gossypii*) (Steiner and Philippsen, 1994), This3 (de *S. kluyveri*) (Weinstock and Strathern, 1993).

Le promoteur d'interférence selon la présente invention est un promoteur inductible et est choisi et positionné de manière à ce que son activation entraîne une interférence transcriptionnelle du premier promoteur, conduisant à une diminution détectable de l'expression du gène rapporteur.

Le promoteur inductible est activé de manière conditionnelle. Dans le cadre de la présente invention, il est activé par un facteur de transcription lorsque celui-ci est actif. Selon la présente invention, le facteur transcription activant le promoteur inductible est constitué par un ensemble de deux protéines chimère (X-DBD et Y-AD) reconstituant un facteur de transcription actif capable d'activer le promoteur inductible lorsqu'une interaction a lieu entre les deux protéines chimères X-DBD et Y-AD. Comme il a été précisé plus haut, les cellules selon la présente invention expriment deux protéines chimères :
- Une première protéine chimère (Y-AD) constituée d'un domaine d'activation de la transcription (AD) fusionné à une protéine partenaire Y, susceptible d'interagir avec une protéines partenaires X,
- Une deuxième protéine chimère (X-DBD) constituée d'un premier domaine liant l'ADN (DBD) fusionné à un deuxième domaine constitué par une protéine X, susceptible d'interagir avec la protéine Y,
l'interaction des deux protéines chimères X-DBD et Y-AD aboutissant à la constitution d'un facteur de transcription fonctionnel activant le ou les promoteur d'interférence.

Le domaine liant l'ADN (DBD) de la deuxième protéine chimère X-DBD est donc choisi en fonction du promoteur inductible de manière à lier l'ADN au niveau dudit promoteur. De façon préféré, le domaine liant l'ADN (DBD) de cette protéine chimère est choisi afin d'interagir avec une séquence présente dans ledit promoteur (séquence d'ADN lié par le DBD (UAS) (upstream activating sequence)).

Le domaine d'activation de la transcription de la première protéine chimère Y-AD permet d'activer la transcription du promoteur inductible dans la cellule. Ainsi, le ou les promoteurs d'interférences (promoteurs inductibles) sont induits par le facteur de transcription fonctionnel constitué par l'ensemble des deux protéines chimères Y-AD et X-DBD (interagissant entre elles), ce facteur de transcription fonctionnel interagissant *via* son domaine de liaison à l'ADN (DBD) avec une séquence du promoteur et étant capable d'initier la transcription gouverné par le promoteur via son domaine d'activation de la transcription (AD).

Ceci constitue le principe même de la technique double-hybride et l'homme du métier est à même de choisir de multiples couples promoteur/facteur de transcription permettant la mise en oeuvre de la présente invention.
Une version couramment utilisée du système double-hybride chez la levure tire profit des propriétés de la protéine GAL4p de la levure *Saccharomyces cerevisiae* (Fields, S., et O. Song, 1989) et du promoteur Pgal1. Toutefois les autres systèmes double-hybrides décrits, notamment ceux décrits en table 1 sont également utilisable dans le cadre de la présente invention.

Les protéines partenaires X et Y fusionnées au domaine liant l'ADN (DBD) et au domaine d'activation de la transcription (AD) respectivement peuvent être définies comme tout couple de protéines qui fusionnées au DBD et AD respectivement, interagissent dans la cellule (même si cela n'est pas le cas dans leur organisme d'origine) pour aboutir à la constitution d'un facteur de transcription fonctionnel (X-DBD/Y-AD) activant le ou les promoteurs inductibles tel que décrit ci-dessus. Il peut s'agir de protéines complètes ou de fragments de ces dernières. Ainsi, peuvent être utilisées dans le cadre de la présente invention, les protéines chimères générant un signal double-hybride. C'est à dire les protéines chimères dont les deux protéines partenaires (ou fragment des protéines partenaires) interagissent entre elles avec une affinité suffisante dans la cellule utilisée, pour constituer un facteur de transcription fonctionnel activant le ou les promoteurs inductibles, ledit facteur de transcription liant l'ADN via le domaine de liaison à de la première protéine chimère et activant le ou les promoteur(s) par le domaine d'activation de la transcription (AD) de la deuxième protéine chimérique.

Les cellules selon l'invention peuvent porter la construction d'ADN et les constructions codant pour les protéines chimériques soit de manière intégrée soit sur un vecteur non intégré. Il a été constaté que les meilleurs résultats sont obtenus avec une construction d'ADN d'interférence intégrée.

Préférentiellement, la construction d'ADN d'interférence est intégrée dans le génome de la cellule hôte. Les constructions d'interférence sont avantageusement ciblées vers un locus exempt d'activités de transcription génomiques potentiellement perturbatrices, afin de protéger les systèmes d'interférence de l'éventuelle influence d'activités transcriptionnelles initiées en dehors de la cassette rapporteuse.

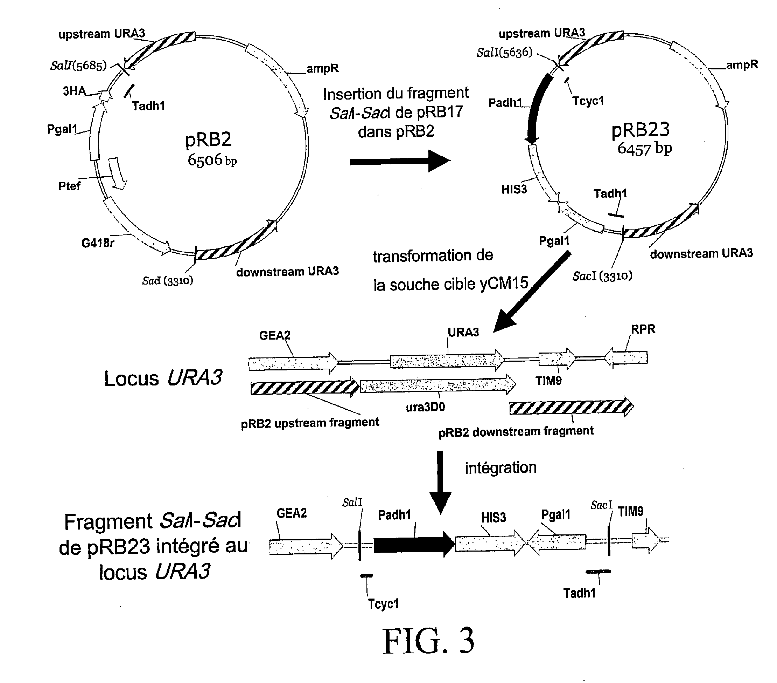

Pour cela, il a été construit un nouveau vecteur d'intégration de levure pRB2. Ce vecteur d'intégration cible les constructions d'interférence vers un locus exempt d'activités de transcription génomiques potentiellement perturbatrices (cf. figure 3).

Il a également été montré que les systèmes d'interférence selon la présente invention sur vecteur non intégrés sont génétiquement stables, ce qui rend non nécessaire la construction de souches présentant des cassettes rapporteuses intégrées. Cette caractéristique est très intéressante pour un criblage de médicament puisque l'on peut ainsi évaluer facilement de nombreuses souches présentant différentes mutations pour augmenter la perméabilité de petites molécules.

Les cellules utilisables pour la mise en oeuvre du système double-hybride objet de la présente invention consiste en toute cellule capable d'exprimer les protéines chimères requises audit système double-hybride décrit ci-dessus et pouvant être transformée ou transfectée par/avec la construction d'ADN d'interférence décrite ci-dessus. Ces hôtes cellulaires sont des hôtes eucaryotes ou procaryotes, il peut s'agir de cellules de mammifères, d'insectes, de plantes ou plus préférentiellement de levures.

Dans un mode de réalisation préféré, les cellules selon la présente invention sont choisies dans le règne des *Fungi.* Préférentiellement, les organismes du règne des *Fungi* sont choisis dans le phylum des *Ascomycetes*, de manière plus préférée, ils sont choisis dans le sous-phylum des *Saccharomycotina*, de manière encore plus préférée, ils sont choisis dans la classe des *Saccharomycetes* ou des *Schizosaccharomycetes*, de manière encore plus préférée ils sont choisis dans l'ordre des *Saccharomycetales* ou des *Schizosaccharomycetales*, de manière encore plus préférée ils sont choisis dans la famille des *Saccharomycetaceae* ou des *Schizosaccharomycetaceae*, de manière encore plus préférée ils sont choisis dans le genre *Saccharomyces* ou *Schizosaccharomyces*, de manière tout à fait préférée les organismes du règne des Fungi selon l'invention appartiennent à l'espèce *Saccharomyces cerevisiae* ou *Schizosaccharomyces pombe*. Les levures des espèces *Kluyveromyces lactis, Pichia pastoris*, *Saccharomyces carlsbergensis* ou *Candida albicans* sont également des cellules entrant dans le cadre de la présente invention.

Contrairement aux systèmes décrits jusqu'à présent, les systèmes doubles hybrides selon la présente invention ne nécessitent pas l'ajout de substances toxiques ou non toxiques. Ceci est un avantage car elles ont probablement des effets supplémentaires sur la physiologie cellulaire et elles peuvent interférer avec la détection de certaines interactions Y2H inverses. L'utilisation d'une substance toxique ou non reste toutefois possible. Il peut par exemple être intéressant d'utiliser une substance inhibant le produit du gène rapporteur dans le cas de l'utilisation d'un gène rapporteur indispensable à la survie, par exemple d'un gène indispensable au métabolisme primaire, à la division cellulaire, à la synthèse protéique (comme les ribosomes), à la synthèse d'ADN ou la synthèse d'ARN etc... En effet, si le gène rapporteur est encore suffisamment exprimé malgré l'interférence de transcription et qu'une absence totale de pousse des cellules est recherchée, une substance inhibant le produit d'un tel gène rapporteur peut être utilisée pour augmenter la sensibilité du système double-hybride. Ainsi par exemple, si le gène rapporteur est le gène *HIS3,* la croissance cellulaire de souches de levure d'interférence (par exemple *S. cerevisiae*) peut être évaluée sur milieu dépourvu d'histidine. Un inhibiteur compétitif de la protéine HIS3p (le 3-amino-1,2,4-triazole (3-AT)) peut éventuellement être ajouté au milieu de culture afin d'observer des différences au niveau du niveau d'activité du gène rapporteur *HIS3*. Ceci peut permettre d'atteindre une plus grande sensibilité et de calibrer le système (cf exemple 6).

Si toutefois le gène rapporteur est encore suffisamment exprimé malgré l'interférence de transcription et qu'une absence totale de pousse des cellules est recherchée, une possibilité est d'utiliser en association d'autres gènes rapporteurs controlés ou non par les systèmes d'interférence décrits ci-dessus (UI, DI, nDI, UDI et nUDI) qui a pour effet d'augmenter la sensibilité du système double-hybride. L'inhibition même partielle de l'expression de ces gènes rapporteurs conduit, par addition des effets, à une inhibition complète de la croissance. Ainsi par exemple, si un des gènes rapporteurs est le gène *HIS3* et l'un des autres est le gène *URA3,* tous deux contrôlés par le système d'interférence, la croissance de la levure d'interférence (par exemple *S. cerevisiae*) peut être évaluée sur milieu dépourvu d'histidine et d'uracile. Alors que sur un milieu dépourvu uniquement d'histidine ou d'uracile on pourrait avoir encore une croissance de la levure ayant une interférence de transcription partielle des gènes rapporteurs, sur un milieu dépourvu à la fois d'uracile et d'histidine, la croissance peut être complètement abolie.

Le système double-hybride selon la présente invention peut avoir de nombreuses applications. Ainsi, il est particulièrement approprié pour le criblage de molécules présentant une activité d'inhibition d'une interaction protéine-protéine.

L'identification d'interactions protéine-protéine impliquées dans les pathologies est hautement intéressante puisque ces interactions procurent des cibles potentielles pour le développement de nouveaux médicaments. Une fois qu'une interaction protéine-protéine impliquée dans une maladie a été identifiée, il est souvent très intéressant de disposer de molécules synthétiques, ou naturelles, dissociant spécifiquement cette interaction protéine-protéine afin d'une part de valider la pertinence de cette interaction dans des systèmes biologiques complexes et de disposer de molécules candidates pour le développement d'un médicament (cf. Vidal, M., and H. Endoh, 1999).

Les systèmes double-hybrides selon la présente invention procurent des systèmes génétiques simples pour un criblage d'inhibiteur d'une interaction protéine-protéine donnée. Ainsi dans les systèmes doubles hybrides selon la présente invention, les molécules dissociant l'interaction protéine-protéine provoquent l'expression du gène rapporteur ce qui permet leur identification.

Il peut être pertinent de choisir un gène rapporteur dont l'expression est essentielle à la survie de la cellule. Ainsi, en permettant une ré-expression de ce gène essentiel, les molécules inhibant l'interaction protéine-protéine peuvent par conséquent être facilement identifiées en suivant la croissance cellulaire.

Ainsi, le criblage d'inhibiteur de l'interaction protéine-protéine recherché peut être réalisé par exemple dans des analyses de diffusion sur gélose à débit élevé (Young, K. *et al.,* 1998), qui permettent d'évaluer des interactions vis-à-vis d'un gradient de concentration de chaque candidat inhibiteur.

Ainsi, la présente invention a pour objet un procédé d'identification d'un composé inhibant l'interaction d'une première protéine X, avec une deuxième protéine Y, comprenant les étapes suivantes :
a) cultiver des cellules telles que décrites ci-dessus,
b) incuber lesdites cellules en présence du composé à tester,
c) comparer l'expression du gène rapporteur en présence et en l'absence dudit composé, une augmentation de l'expression du gène rapporteur étant l'indication que le composé à tester est un inhibiteur de l'interaction de la protéine X avec la protéine partenaire Y, exprimées par les cellules cultivées.

Une autre application du système d'interférence selon la présente invention est le criblage de banques d'ADNc et de banques de peptides pour identifier des peptides ou des facteurs protéiques qui abrogent spécifiquement une interaction protéine-protéine étudiée (Zutshi, R. *et al.,* 1998).

Une fois qu'une interaction protéine-protéine pertinente au niveau d'une maladie a été identifiée et validée, il est intéressant de caractériser la structure et la régulation de l'interaction observée. L'identification de mutations au niveau de chaque partenaire d'une paire de protéines en interaction, qui interrompent l'interaction est utile non seulement pour rechercher les composants structurels d'une interaction, mais également comme moyen de générer des outils génétiques comme des mutants négatifs transdominants pour la caractérisation de la fonction *in vivo* (Serebriiskii, I. G., *et al.,* 2001). Ceci est particulièrement important pour des protéines qui présentent de multiples partenaires d'interaction. Les systèmes d'interférence selon la présente invention peuvent être utilisés dans un criblage par mutagenèse pour une identification de mutations interrompant une interaction. Des banques de partenaires d'interaction ayant subi une mutagenèse doivent être générés et peuvent être criblés dans un format à débit élevé concernant un rétablissement de croissance cellulaire (Gutherie, C., et G. R. Fink (ed.), 2002). Par cette approche, on peut rapidement identifier un ensemble de protéines mutantes déficientes au niveau de l'interaction, notamment des mutants présentant des propriétés dominantes négatives. L'ensemble de mutants peut également aider à définir les surfaces de la protéine impliquées dans l'interaction protéine-protéine et aider un biologiste structurel à améliorer la structure de candidats inhibiteurs lors d'études de relation structure-affinité.

La présente invention a également pour objet un kit pour la mise en place d'un système double-hybride tel que décrit ci-dessus comprenant:
- Une construction d'ADN d'interférence telle que définie ci-dessus,
- Une deuxième construction d'ADN codant pour :
   - Une première protéine chimère (Y-AD) constituée d'un domaine d'activation de la transcription (AD) fusionné à une protéine partenaire Y susceptible d'interagir avec une protéine partenaire X,
- Une troisième construction d'ADN codant pour :
   - Une deuxième protéine chimère (X-DBD) constituée d'un premier domaine liant l'ADN (DBD) fusionné à un deuxième domaine constitué par une protéine X susceptible d'interagir avec la protéine Y, l'interaction des deux protéines chimères X-DBD et Y-AD aboutissant à la constitution d'un facteur de transcription fonctionnel activant le ou les promoteur d'interférence lorsque les deux protéines chimères sont exprimées dans une cellule hôte.
Les trois constructions d'ADN du kit décrit ci-dessus peuvent être présentes sur la même molécule d'ADN, ou sur deux ou trois molécules d'ADN différentes. Les trois constructions peuvent ainsi être portées par le même vecteur d'ADN ou se trouver sur deux ou trois vecteurs différents. Les deux constructions codant pour les deux protéines chimères peuvent par exemple se trouver sur le même vecteur alors que la troisième construction se trouve sur un deuxième vecteur.

La présente invention procure ainsi un ensemble de systèmes génétiques simples pour l'identification de mutations et de molécules qui induisent l'interruption d'interactions protéine-protéine. La présente invention est un outil utile pour la caractérisation d'interactions protéine-protéine et également pour le développement de nouveaux médicaments.

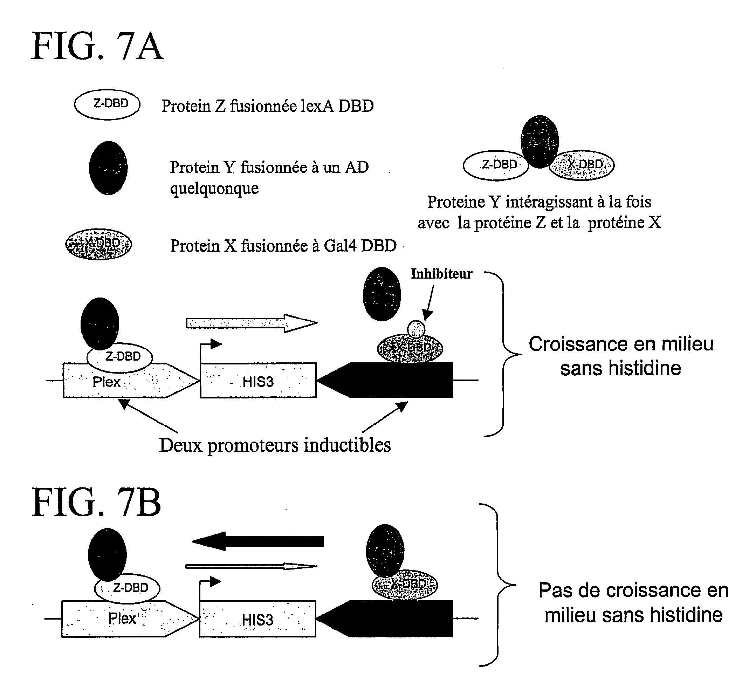

La présente invention peut également s'avérer très utile en cas d'interaction d'une protéine Y avec deux autres protéines X et Z et que l'on souhaite identifier des molécules inhibant l'interaction entre Y et X mais n'inhibant pas l'interaction entre Y et Z. Des cellules permettant une telle sélection portent par exemple la construction génétique présentée à la figure 7 et exprimant en outre au moins trois protéines chimères telles que représentées à la figure 7. Ces cellules permettent d'identifier des inhibiteurs spécifiques d'une interaction entre une protéine X et une protéine Y, si la protéine Y interagit également avec une autre protéine Z.

L'invention a donc également pour objet des cellules comportant une construction d'ADN, dite construction d'interférence, ladite construction comprenant :
- Un gène rapporteur placé sous le contrôle d'un premier promoteur inductible,
- Un où plusieurs promoteur(s) inductible(s), dit(s) promoteur(s) d'interférence, choisi(s) et positionné(s) de manière à ce que son (leur) activation entraîne une interférence transcriptionnelle du premier promoteur inductible, conduisant à une diminution détectable de l'expression du gène rapporteur lorsque l'expression de ce dernier est induite,
ladite cellule exprimant en outre :
- Une première protéine chimère (Y-AD) constituée d'un domaine d'activation de la transcription (AD) fusionné à une protéine Y, susceptible d'interagir avec deux protéines partenaires X et Z,
- Une deuxième protéine chimère (X-DBD) constituée d'un premier domaine liant l'ADN (DBD) fusionné à un deuxième domaine constitué par une protéine X, susceptible d'interagir avec la protéine Y, l'interaction des deux protéines chimères X-DBD et Y-AD aboutissant à la constitution d'un facteur de transcription fonctionnel activant le ou les promoteur d'interférence,
- Une troisième protéine chimère (Z-DBD) constituée d'un domaine liant l'ADN (DBD), fusionné à un deuxième domaine constitué par une protéine Z susceptible d'interagir avec la protéine Y, l'interaction des deux protéines chimères Z-DBD et Y-AD aboutissant à la constitution d'un facteur de transcription fonctionnel activant l'expression du gène rapporteur.

Dans un premier mode de mise en oeuvre, les cellules selon la présente invention sont des cellules hôte transformées ou transfectées par la construction d'ADN comprenant le gène rapporteur (construction d'interférence) et elles sont également transformées ou transfectées par trois constructions d'ADN codant pour chacune des trois protéines chimères. Les quatre constructions peuvent être portées par le même vecteur d'ADN ou se trouver sur deux ou trois vecteurs différents. Toutes les combinaisons sont envisageables : les quatre constructions sur le même vecteur, les quatre constructions sur quatre vecteurs différents, les trois constructions codant pour les trois protéines chimères sur le même vecteur alors que la quatrième construction (construction d'interférence) se trouve sur un deuxième vecteur, une construction codant pour une des trois protéines chimères et la construction d'interférence sur le même vecteur alors que la construction codant pour les deux autres protéines chimères se trouve sur un deuxième vecteur etc... Dans ce premier mode de mise en oeuvre, les vecteurs sont des vecteurs non intégratifs.

Dans un second mode de mise en oeuvre, la construction d'interférence est intégrée au génome des cellules et ces dernières sont transformées ou transfectées par trois constructions d'ADN codant pour chacune des trois protéines chimères. Les constructions d'ADN codant pour les protéines chimères peuvent être portées par le même vecteur d'ADN ou se trouver sur des vecteurs différents, ce ou ces vecteurs étant des vecteurs non intégratifs.

Dans un troisième mode de mise en oeuvre de l'invention, la construction d'interférence ainsi que les constructions d'ADN codant pour les protéines chimères sont intégrées au génome des cellules. Il est également possible que seule une ou deux des trois constructions codant pour une ou deux des trois protéines chimères soit intégrée au génome et que la ou les constructions soit portée par un vecteur non intégratif alors que la construction d'interférence est intégrée au génome. Enfin ,dans un dernier mode de mise en oeuvre, la construction d'interférence et une ou deux des constructions codant pour une ou deux des trois protéines chimères sont portées par des vecteurs non intégratifs alors que la construction codant pour la troisième protéine chimères est intégrée au génome des cellules.

Le gène rapporteur est tel que défini ci-dessus. Le gène rapporteur est placé sous le contrôle d'un promoteur inductible. De façon préféré, le promoteur inductible régulant l'expression du gène rapporteur comprend une séquence susceptible d'interagir avec une protéine possédant un domaine de liaison à l'ADN (DBD) (séquence d'ADN lié par le DBD (UAS)). Plus particulièrement, le promoteur inductible régulant l'expression du gène rapporteur comprend une séquence susceptible d'interagir avec le domaine liant l'ADN (DBD) de la protéine chimère (Z-DBD).

Le ou les promoteurs dit « promoteurs d'interférence » sont également des promoteurs inductibles. Ce ou ces derniers sont choisis et positionnés de manière à ce que leur activation entraîne une interférence transcriptionnelle du premier promoteur, conduisant à une diminution détectable de l'expression du gène rapporteur lorsque l'expression de ce dernier est induite.

Les promoteurs d'interférences pouvant être mis en oeuvre sont les promoteurs inductibles tels que définis ci-dessus. De façon préféré, les promoteurs d'interférences pouvant être mis en oeuvre comprennent une séquence susceptible d'interagir avec une protéine possédant un domaine de liaison à l'ADN (DBD) (séquence d'ADN lié par le DBD (UAS)). Plus particulièrement, les promoteurs inductibles d'interférence comprennent une séquence susceptible d'interagir avec le domaine liant l'ADN (DBD) de la protéine chimère (X-DBD).

Le ou les promoteurs d'interférence peuvent être positionnés comme décrit ci-dessus. Ainsi, ils peuvent être positionnés en aval du premier promoteur et du gène rapporteur, il s'agit alors d'interférence aval. Le ou les promoteurs d'interférence peuvent également être positionnés en amont du premier promoteur et du gène rapporteur, il s'agit alors d'interférence amont.

Dans le cadre d'une interférence aval, le promoteur d'interférence peut cependant présenter deux orientations possibles. Une première orientation correspond à une orientation convergente par rapport au premier promoteur, cette configuration est dénommée interférence aval (ou DI pour « Downstream Interference »). Le promoteur d'interférence peut également présenter une orientation en tandem par rapport au premier promoteur, cette deuxième configuration a été dénommée interférence aval non-sens (ou nDI pour « non-sense Downstream Interference »).

Le ou les promoteurs d'interférence peuvent également être positionnés en amont du premier promoteur et du gène rapporteur. Le promoteur d'interférence présente alors une orientation identique à celle du premier promoteur (les deux promoteurs ont donc une configuration en tandem), cette configuration a été dénommée interférence amont (ou UI pour « Upstream Interference »).

Une autre configuration possible est la combinaison des deux configurations décrites ci-dessus (système UI et DI). Dans ce cas, au moins un promoteur d'interférence (inductible) est positionné de part et d'autre du premier promoteur inductible et du gène rapporteur. Il s'agit alors d'interférence amont-aval car l'activation du ou des promoteur(s) d'interférence situé(s) en amont interfère(nt) avec l'activité du premier promoteur situé plus en aval et l'activation du ou des promoteur(s) d'interférence situé(s) en aval interfère(nt) avec l'activité du premier promoteur situé plus en amont.

Le ou les promoteurs d'interférences situés en aval du premier promoteur et du gène rapporteur présentent une orientation convergente par rapport au premier promoteur (cf. le système DI ci-dessus). Le ou les promoteurs d'interférence positionnés en amont du premier promoteur et du gène rapporteur présentent une orientation identique à celle du premier promoteur (les deux promoteurs ont donc une configuration en tandem) (cf. système UI ci-dessus).

Cette configuration a été dénommée interférence amont-aval (ou UDI pour « Upstream-Downstream Interference »).

Une autre configuration possible est la combinaison de deux configurations décrites ci-dessus (système UI et nDI). Dans ce cas, au moins un promoteur d'interférence inductible est positionné de part et d'autre du premier promoteur inductible et du gène rapporteur. Il s'agit alors d'interférence amont-aval car l'activation du ou des promoteur(s) d'interférence situé(s) en amont interfère(nt) avec l'activité du premier promoteur situé plus en aval et l'activation du ou des promoteur(s) d'interférence situé(s) en aval interfère(nt) avec l'activité du premier promoteur situé plus en amont.

Le ou les promoteurs d'interférences situés en aval du premier promoteur et du gène rapporteur présentent une orientation en tandem par rapport au premier promoteur (cf. le système nDI ci-dessus). Le ou les promoteurs d'interférence positionnés en amont du premier promoteur et du gène rapporteur présentent une orientation identique à celle du premier promoteur (cf. système UI ci-dessus), les trois promoteurs ont donc une configuration en tandem.
Cette configuration a été dénommée interférence non sens amont-aval (ou nUDI pour « non-sense Upstream-Downstream Interference »).

Dans le cas d'une construction de type UI, UDI et nUDI, une courte phase ouverte de lecture suivie d'un ou plusieurs (par exemple 2 ou 3) codons stop est préférentiellement insérée entre le promoteur d'interférence et le premier promoteur, comme décrit ci-dessus.

Préférentiellement, les constructions d'interférences sont bordées à leurs extrémités par des terminateurs de transcription bidirectionnels tel que décrit ci-dessus.

Le promoteur d'interférence est un promoteur inductible qui est activé de manière conditionnelle. Dans le cadre de la présente invention, il est activé par un facteur de transcription lorsque celui-ci est actif Selon la présente invention, le facteur de transcription activant le promoteur inductible est constitué par un ensemble de deux protéines chimère (X-DBD et Y-AD) reconstituant un facteur de transcription actif capable d'activer le promoteur inductible lorsqu'une interaction a lieu entre les deux protéines chimères X-DBD et Y-AD.

Comme il a été précisé plus haut, dans ce mode particulier de réalisation de la présente invention, les cellules expriment trois protéines chimères. Elles expriment ainsi :
- Une première protéine chimère (Y-AD) constituée d'un domaine d'activation de la transcription (AD) fusionné à une protéine partenaire Y, susceptible d'interagir avec deux protéines partenaires X et Z,
- Une deuxième protéine chimère (X-DBD) constituée d'un premier domaine liant l'ADN (DBD) fusionné à un deuxième domaine constitué par une protéine X, susceptible d'interagir avec la protéine Y, l'interaction des deux protéines chimères X-DBD et Y-AD aboutissant à la constitution d'un facteur de transcription fonctionnel activant le ou les promoteur d'interférence,
- Une troisième protéine chimère (Z-DBD) exprimée est constituée d'un domaine liant l'ADN (DBD), fusionné à un deuxième domaine constitué par une protéine Z susceptible d'interagir avec la protéine Y, l'interaction des deux protéines chimères Z-DBD et Y-AD aboutissant à la constitution d'un facteur de transcription fonctionnel activant l'expression du gène rapporteur.

Le domaine liant l'ADN (DBD) de la deuxième protéine chimère est choisi en fonction du promoteur d'interférence de manière à lier l'ADN au niveau dudit promoteur. De façon préféré, le domaine liant l'ADN (DBD) de la deuxième protéine chimère est choisi afin d'interagir avec une séquence présente dans ledit promoteur (séquence d'ADN lié par le DBD (UAS) (upstream activating sequence)). Le ou les promoteurs inductibles d'interférence comprennent une séquence susceptible d'interagir avec le domaine liant l'ADN (DBD) de la protéine chimère (X-DBD).

Le domaine d'activation de la transcription de la première protéine chimère (domaine AD de Y-AD) permet d'activer la transcription du promoteur d'interférence dans la cellule. Ainsi, le ou les promoteurs d'interférences (promoteurs inductibles) sont induits par le facteur de transcription fonctionnel constitué les deux protéines chimères X-DBD et Y-AD interagissant entre elles. Ce facteur de transcription fonctionnel interagit *via* son domaine de liaison à l'ADN (DBD) avec une séquence du promoteur d'interférence et est capable d'initier la transcription gouverné par ce promoteur via son domaine d'activation de la transcription (AD).

Ceci constitue le principe même de la technique double-hybride et l'homme du métier est à même de choisir de multiples couples promoteur/facteur de transcription permettant la mise en oeuvre de la présente invention, comme précisé ci-dessus.

Le domaine liant l'ADN (DBD) de la troisième protéine chimère (Z-DBD) est choisi en fonction du promoteur inductible régulant l'expression du gène rapporteur, de manière à lier l'ADN au niveau dudit promoteur. De façon préféré, le domaine liant l'ADN (DBD) de la protéine chimère Z-DBD est choisi afin d'interagir avec une séquence présente dans le promoteur régulant l'expression du gène rapporteur (séquence d'ADN lié par le DBD (UAS) (upstream activating sequence)).

Le promoteur inductible régulant l'expression du gène rapporteur et le ou les promoteurs d'interférences sont des promoteurs différents. Notamment le promoteur inductible régulant l'expression du gène rapporteur est choisi de tel manière à ne pas lier la protéine chimère X-DBD (ce promoteur ne comporte donc pas de séquence d'ADN lié par le DBD (UAS) (upstream activating sequence) de X-DBD)). De plus, le ou les promoteurs inductibles d'interférence sont choisis de manière à ce qu'ils ne lient pas la protéine chimère Z-DBD (ce promoteur ne comporte donc pas de séquence d'ADN lié par le DBD (UAS) (upstream activating sequence) de Z-DBD)).

Le domaine d'activation de la transcription de la première protéine chimère (domain AD de Y-AD) permet d'activer la transcription du promoteur régulant l'expression du gène rapporteur dans la cellule. Ainsi, le promoteur régulant l'expression du gène rapporteur est induit par le facteur de transcription fonctionnel constitué les deux protéines chimères Z-DBD et Y-AD interagissant entre elles. Ce facteur de transcription fonctionnel interagit via son domaine de liaison à l'ADN (DBD) avec une séquence du promoteur régulant l'expression du gène rapporteur et est capable d'initier la transcription gouverné par ce promoteur via son domaine d'activation de la transcription (AD).

Ceci constitue le principe même de la technique double-hybride et l'homme du métier est à même de choisir de multiples couples promoteur/facteur de transcription permettant la mise en oeuvre de la présente invention, comme précisé ci-dessus.

Ainsi en cas d'interaction entre les protéines Y et X, le promoteur d'interférence est activé ce qui conduit à une interférence dans la transcription du gène rapporteur. Si l'interaction entre Y et X est perturbé (par exemple par l'action d'un inhibiteur chimique inhibant l'interaction entre Y et X), le promoteur d'interférence sera moins ou pas activé conduisant ainsi à une expression accrue du gène rapporteur.

Les protéines partenaires X, Y et Z fusionnées au domaine liant l'ADN (DBD) et au d'un domaine d'activation de la transcription (AD) peuvent être définies comme tout groupe de protéines pour lesquels il existe une interaction entre Y et X et entre Z et Y et qui lorsque Y est fusionné à AD et Z et X sont fusionnés à des DBD différents, interagissent dans la cellule (même si cela n'est pas le cas dans leur organismes d'origine) pour aboutir à la constitution de deux facteurs de transcription fonctionnels (X-DBD/Y-AD et Z-DBD/Y-AD respectivement) activant les promoteurs inductibles tel que décrit ci-dessus. Il peut s'agir de protéines complètes ou de fragments de ces dernières. Ainsi, peuvent être utilisées dans le cadre de la présente invention, les protéines chimères générant un signal double-hybride. C'est à dire les protéines chimères dont les deux protéines partenaires (ou fragment des protéines partenaires) interagissent entre elles avec une affinité suffisante dans la cellule utilisée, pour constituer un facteur de transcription fonctionnel activant le ou les promoteurs inductibles, ledit facteur de transcription liant l'ADN via le domaine de liaison à de la première protéine chimère et activant le ou les promoteur par le domaine d'activation de la transcription (AD) de la deuxième protéine chimérique.

Lorsque la construction d'ADN d'interférence est intégrée dans le génome de la cellule hôte, cette construction est avantageusement ciblée vers un locus exempt d'activités de transcription génomiques potentiellement perturbatrices, comme décrit ci-dessus.

Les cellules utilisables dans le cadre de ce mode de réalisation de l'invention (trois protéines chimères) sont celles décrites ci-dessus.

Comme il a été précisé ci-dessus, le système double-hybride selon la présente invention peut avoir de nombreuses applications. Ainsi, il est particulièrement approprié pour le criblage de molécules présentant une activité d'inhibition d'une interaction protéine-protéine. Dans le cadre du mode de réalisation de l'invention à trois protéines chimères, les cellules décrites ci-dessus sont particulièrement intéressantes en ce qui concerne l'identification des molécules inhibant l'interaction entre les protéines Y et X mais n'inhibant pas l'interaction entre Y et Z. Ainsi dans ces cellules, les molécules dissociant l'interaction protéine-protéine entre Y et X provoquent l'expression du gène rapporteur ce qui permet leur identification.

La présente invention a donc également pour objet un procédé d'identification d'un composé inhibant l'interaction d'une première protéine X, avec une deuxième protéine Y, mais n'inhibant pas ou moins l'interaction entre la protéine Y et une troisième protéine Z comprenant les étapes suivantes :
a) cultiver des cellules telles que décrites ci-dessus (mode de réalisation à trois protéines chimérique),
b) incuber lesdites cellules en présence du composé à tester,
c) comparer l'expression du gène rapporteur en présence et en l'absence dudit composé, une augmentation de l'expression du gène rapporteur étant l'indication que le composé à tester est un inhibiteur de l'interaction de la protéine X, avec la protéine partenaire Y, mais que ce produit n'inhibe pas ou moins l'interaction entre la protéine Y et la protéine Z.

Une autre application du système d'interférence à trois protéines chimères est le criblage de banques d'ADNc et de banques de peptides pour identifier des peptides ou des facteurs protéiques qui abrogent spécifiquement l'interaction entre la protéine X et la protéine Y étudiée (Zutshi, R. *et al.,* 1998), mais qui n'affecte pas l'interaction entre la protéine X et la protéine Z.

Une autre application est l'identification des mutations dans la protéine Y, qui interrompent l'interaction entre la protéine Y et la protéine X, mais qui n'affecte pas l'interaction entre la protéine Y et la protéine Z. Ceci est utile non seulement pour rechercher les composants structurels d'une interaction particulière, mais également comme moyen de générer des outils génétiques comme des mutants négatifs transdominants pour la caractérisation de la fonction *in vivo* (Serebriiskii, I. G., *et al.,* 2001) (voir ci-dessus).

La présente invention a également pour objet un kit pour la mise en place d'un système double-hybride tel que décrit ci-dessus comprenant:
- Une construction d'ADN telle que définie ci-dessus,
- Une deuxième construction d'ADN codant pour :
   - Une première protéine chimère (Y-AD) constituée d'un domaine d'activation de la transcription (AD) fusionné à une protéine partenaire Y, susceptible d'interagir avec deux protéines partenaires X et Z,
- Une troisième construction d'ADN codant pour :
   - Une deuxième protéine chimère (X-DBD) constituée d'un premier domaine liant l'ADN (DBD) fusionné à un deuxième domaine constitué par une protéine X, susceptible d'interagir avec la protéine Y, l'interaction des deux protéines chimères X-DBD et Y-AD aboutissant à la constitution d'un facteur de transcription fonctionnel activant le ou les promoteur d'interférence,
- Une quatrième construction d'ADN codant pour :
   - Une troisième protéine chimère (Z-DBD) constituée d'un domaine liant l'ADN (DBD), fusionné à un deuxième domaine constitué par une protéine Z susceptible d'interagir avec la protéine Y, l'interaction des deux protéines chimères Z-DBD et Y-AD aboutissant à la constitution d'un facteur de transcription fonctionnel activant l'expression du gène rapporteur.
Les quatre constructions d'ADN du kit décrit ci-dessus peuvent être présentes sur la même molécule d'ADN, ou sur deux ou trois ou quatre molécules d'ADN différentes. Les quatre constructions peuvent ainsi être portées par le même vecteur d'ADN ou se trouver sur deux, trois ou quatre vecteurs différents. Les trois constructions codant pour les trois protéines chimères peuvent par exemple se trouver sur le même vecteur alors que la quatrième construction se trouve sur un deuxième vecteur.

La présente invention est un outil utile pour la caractérisation d'interactions protéine-protéine et mais également pour le développement de nouveaux médicaments.

### Légende des figures

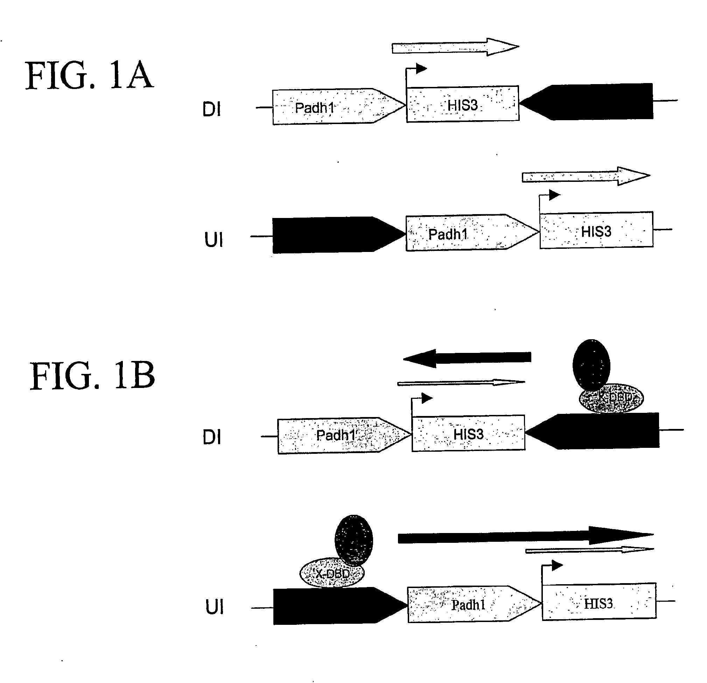

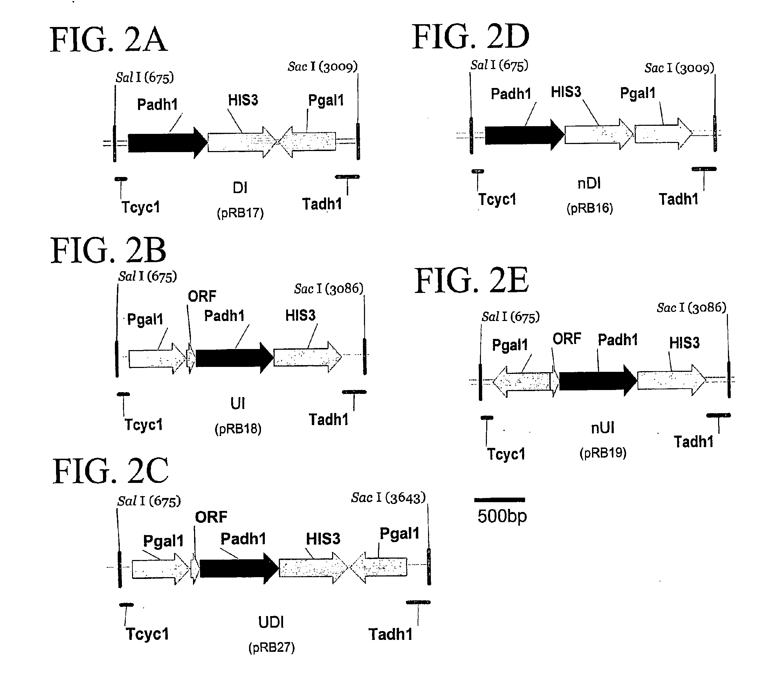

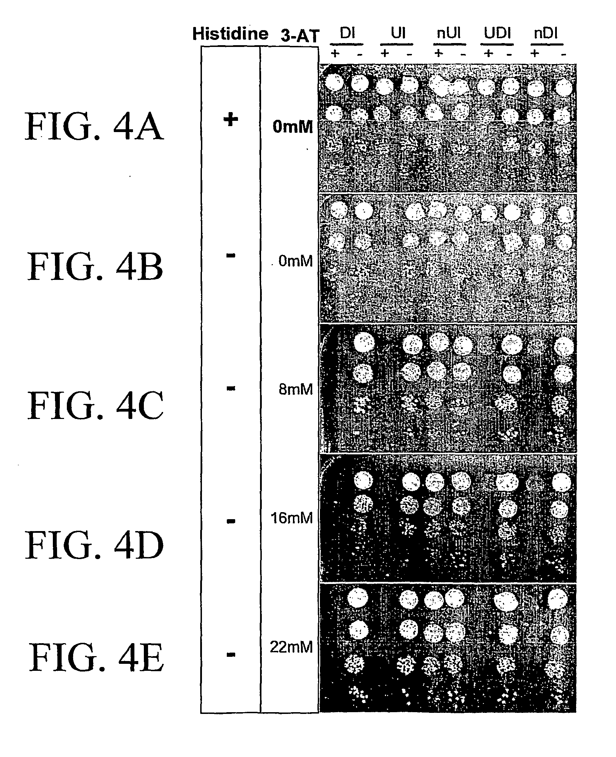

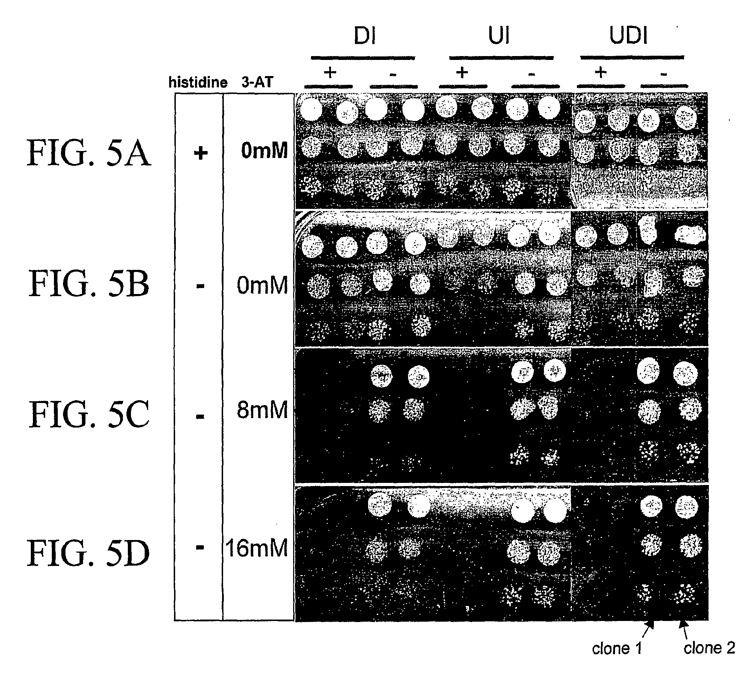

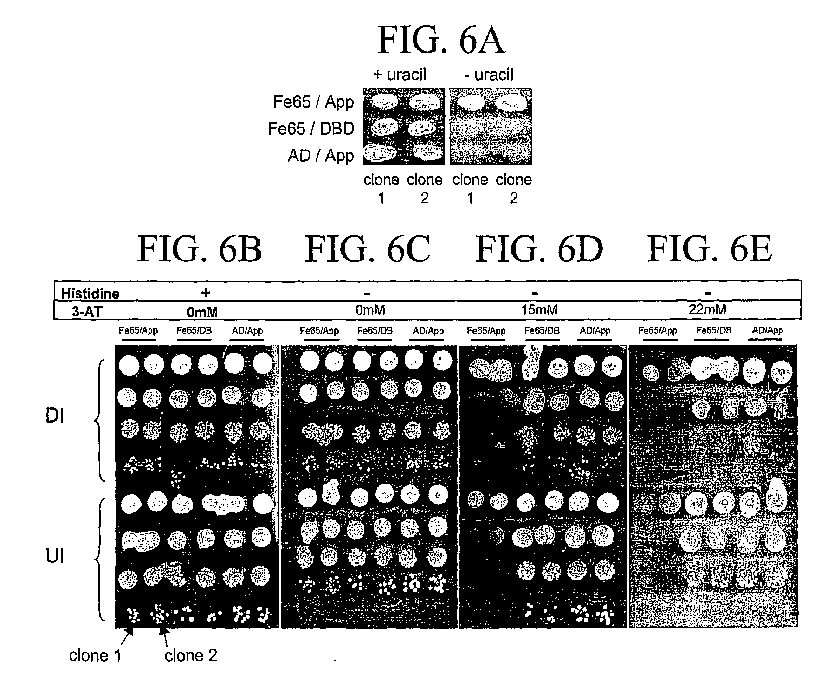

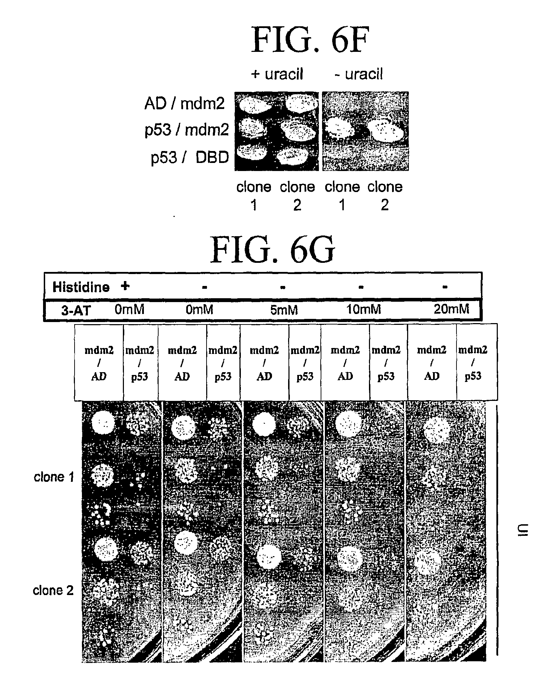

Figure 1 : Systèmes rY2H basés sur une interférence de transcription.
   Présentation schématique illustrant l'application d'une interférence de transcription pour la détection d'interactions Y2H inverses. En absence d'une interaction Y2H, le gène rapporteur *HIS3* est normalement exprimé et les cellules peuvent croître sur un milieu sans histidine (Fig. 1A). La présence d'une interaction Y2H réduit le niveau de transcription de *HIS3*, ce qui provoque une réduction de la croissance cellulaire de la souche rapporteuse auxotrophe pour l'histidine (Fig. 1B). L'ORF de *HIS3* est symbolisé par le rectangle de couleur clair, la petite flèche noire marquant la position du codon de départ. La flèche grise au-dessus, de l'ORF de *HIS3* symbolise la transcription initiée au niveau de Padh1 alors que la flèche noire représente la transcription de Pgall. L'épaisseur des flèches symbolise l'activité de transcription des promoteurs. Les flèches pointent dans la direction de la transcription. Des protéines en interaction X et Y utilisées pour le GAL4-DBD (X-DBD) et le GAL4-AD (Y-AD) sont représentées sous forme ovoïde. Padh1 : promoteur de *ADH1*; Pgal1 : promoteur de *GAL1*; HIS3 : ORF de *HIS3*; DI : interférence aval; UI : interférence amont. Figure non représentée à l'échelle.
Figure 2 :
   Constructions d'interférence de transcription.
      Cinq constructions différentes ont été mises en oeuvre pour la mise en place du systèmes rY2H basés sur une interférence de transcription ; les cinq constructions sont représentées à l'échelle (Fig. 2A à 2E). Les sites de restriction *Sal*I et *Sac*I et leurs positions sur les vecteurs correspondants pBluescript sont indiqués. Les noms des plasmides exemplifiés (voir exemple 4 et Table 2) sont indiqués. Abréviations : Padh1 : promoteur de *ADH1*; Pgal1 : promoteur de *GAL1*; HIS3 : ORF de *HIS3*; Tadh1 : terminateur de *ADH1*; Tcyc1 : terminateur de *CYC1*; ORF : courte phase ouverte de lecture suivie de deux codons stop; DI : interférence aval (2A); UI : interférence amont (2B); UDI : interférence amont-aval (2C); nDI : interférence aval non sens (2D); nUI : interférence amont non sens (2E).
Figure 3 :
   Intégration génomique de constructions d'interférence.
      Exemple illustrant l'intégration génomique de constructions d'interférence de transcription. Le vecteur d'intégration pRB23 a été construit en insérant le fragment *Sal*I-*Sac*I de pRB17 dans pRB2. pRB23 est alors utilisé pour transformer la souche cible yCM15 où il s'intègre au niveau du locus *URA3* par recombinaison homologue. Les séquences de vecteur homologues au locus d'*URA3* (amont/aval d'*URA3)* sont représentées en hachuré. Les ORFs adjacents au gène d'*URA3* sont représentés (*GEA2, TIM9* et *RPR)* et la délétion modèle *ura3D0* est indiquée (Brachmann, C. B., *et al.,* 1998). Abréviations : Padh1 : promoteur de *ADH1*; Pgal1 : promoteur de *GAL1*; HIS3 : ORF de *HIS3*; Tadh1 : terminateur de *ADH1*; Tcyc1 : terminateur de CYC1; 3HA : séquences tag codées sur pRB2; Ptef : promoteur de *TEF*; G418r : ORF codant pour une résistance au G418; ampR : gène de résistance à l'ampicilline.
Figure 4 :
   Evaluation fonctionnelle de systèmes d'interférence intégrés.
      Les souches portant des copies intégrées des systèmes d'interférence (yRB48 (DI), yRB49 (UI), yRB50 (nUI), yRB83 (UDI) et yRB82 (nDI)) sont transformées avec les séries de plasmides pCL1/pFL39 (+) et pTAL20/pDBT (-), respectivement, et la croissance cellulaire des transformants est étudiée sur milieu pourvu (Fig. 4A) ou dépourvu (Fig. 4B à E) d'histidine par une méthode d'évaluations en goutte. On prépare des séries de dilutions d'un facteur 10 pour chaque transformant. Un inhibiteur compétitif de la protéine HIS3p (le 3-amino-1,2,4-triazole (3-AT)) est éventuellement ajouté au milieu de culture à des concentrations croissantes comme indiqué (0 mM à 22 mM) (Fig. 4B à E). Abréviations : DI : souche d'interférence en aval; UI : souche d'interférence en amont; nDI : souche d'interférence aval non sens; nUI: souche d'interférence amont non sens; UDI : souche d'interférence amont-aval.
Figure 5 :
   Evaluation fonctionnelle de systèmes d'interférence basés sur des plasmides (non intégrées au niveau énomique) :
      La souche yCM15 est co-transformé indépendamment avec des versions basées sur des plasmides des systèmes d'interférence (pRB20 (DI), pRB21 (UI) ou pRB28 (UDI)) et les séries de plasmides pCL1/pFL39 (+) et pTAL20/pDBT (-), respectivement. La croissance cellulaire de chacun de ces triples transformants est étudiée sur boîtes de gélose avec (Fig. 5A) et sans (Fig. 5B à D) histidine et pour les boîtes sans histidine, sans ou avec des concentrations croissantes en 3-AT (0 mM à 16 mM) (Fig. 5B à D). On prépare des séries de dilution d'un facteur 10 et on évalue deux clones indépendants pour chaque souche transformée. Abréviations : DI : plasmide d'interférence aval; UI : plasmide d'interférence amont; UDI : plasmide d'interférence amont-aval.
Figure 6 :
   Interférence de transcription induite par des interactions Y2H.
Figure 6A et 6F :
   Les interactions protéine-protéine précédemment identifiées entre les protéines Fe65 et App et p53 et mdm2 induisent suffisamment une construction d'interférence de transcription pour inhiber la croissance cellulaire de levure.
   Dans un premier temps l'activité des constructions est vérifiée (Fig. 6A et 6F). Pour cela, la souche reportrice Y187 est transformée par les couples de plasmides suivants : pRB34 + pRB35 (Fe65 / App), pRB34 + pDBT (Fe65 / DBD) et pTAL20 + pRB35 (AD / App) (Fig. 6A) et pDBT-mdm2 + pVP 1 6-p53Nt (p53 / mdm2), pVP 1 6-p53Nt + pDBT (p53 / DBD) et pTAL20 + pDBT-mdm2 (AD / mdm2) (Fig. 6F). Deux clones de chacun de ces transformants sont cultivés sur milieu minimum avec et sans uracile ((Fig. 6A et 6F).
   Les souches yRB48 (système DI) et yRB49 (système UI) sont ensuite co-transformées chacune par le couple de plasmides pRB34 (Fe65) et pRB35 (App) permettant l'expression des deux protéines hybrides AD-Fe65 et DBD-AppCt. Les souches yRB48 (système DI) et yRB49 (système UI) sont également co-transformées chacune par les couples de plasmides pRB34 + pDBT (Fe65 / DBD) et pTAL20 + pRB35 (AD / App) comme contrôles.
   La croissance cellulaire de deux clones de chacun de ces doubles transformants est étudiée sur boîtes de gélose avec et sans histidine et pour les boîtes sans histidine, sans ou avec des concentrations croissantes en 3-AT (Fig. 6B à 6E).
   La souche yRB49 (système UI) est également co-transformées par le couple de plasmides pDBT-mdm2 + pVP16-p53Nt (p53 / mdm2) permettant l'expression des deux protéines hybrides DBD-mdm2 et VP16-p53(1-55). La souche yRB49 (système UI) est également co-transformées par le couple de plasmides pDBT-mdm2 + pTAL20 (AD / mdm2) comme contrôles. Deux clones co-transformés par les deux plasmides sont sélectionnés pour chacun de ces deux couples de plasmides.
   La croissance cellulaire de chacun des deux clones de ces doubles transformants est étudiée sur boîtes de gélose avec et sans histidine et pour les boîtes sans histidine, sans ou avec des concentrations croissantes en 3-AT, comme indiqué sur la figure.
Figure 7 :
   Systèmes rY2H basés sur une interférence de transcription selon la présente invention permettant l'identification de molécules inhibant l'interaction entre deux protéines Y et X mais n'inhibant pas l'interaction entre la protéine Y et une troisième protéine Z
      Présentation schématique illustrant l'application d'une interférence de transcription pour la détection d'interactions Y2H inverses permettant l'identification de molécules inhibant l'interaction entre deux protéines Y et X mais n'inhibant pas l'interaction entre la protéine Y et une troisième protéine Z. En l'absence d'une interaction Y2H entre la protéine X et Y, le gène rapporteur *HIS3* est normalement exprimé et les cellules peuvent croître sur un milieu sans histidine (Fig. 7A). Ceci est le cas lorsqu'un inhibiteur de l'interaction de la protéine X, avec la protéine partenaire Y est présent. La présence d'une interaction Y2H entre X-DBD et Y-AD réduit le niveau de transcription de *HIS3*, ce qui provoque une réduction de la croissance cellulaire de la souche rapporteuse auxotrophe pour l'histidine (Fig. 7B). L'ORF de *HIS3* est symbolisé par le rectangle de couleur clair, la petite flèche noire marquant la position du codon de départ. La flèche grise au-dessus de l'ORF de *HIS3* symbolise la transcription initiée au niveau de Plex alors que la flèche noire représente la transcription de Pgal1. L'épaisseur des flèches symbolise l'activité de transcription des promoteurs. Les flèches pointent dans la direction de la transcription. Des protéines en interaction X / Y et Z / Y utilisées sont représentées sous forme ovoïde. Plex : promoteur de LexA; Pgall : promoteur de *GAL1*; HIS3 : ORF de *HIS3*; Figure non représentée à l'échelle.
Figure 8 :
   Détermination des conditions expérimentales pour le criblage d'inhibiteurs de l'interaction Fe65/App.
Figure 8A :
   Détermination de la quantité de cellules :
      La souche contenant le système d'interférence transcriptionnelle UI (yRB49) a été co-transformée par les plasmides pRB34 et pRB35 (interaction Fe65/App). Une culture de la souche transformée a été diluée à DO = 0,1 et 0,01 et 10 ml de chaque dilution ont été étalés sur milieu supplémenté en histidine (+HIS). Les boîtes ont été incubées 48 heures à 30°C.
Figure 8B :
   Détermination de la concentration de 3-AT :
      La souche contenant le système d'interférence transcriptionnelle UI (yRB49) co-transformée par les plasmides pRB34 et pRB35 (interaction Fe65/App) ou avec les plasmides de contrôle pTAL20 et pDBT (AD/DBD) a été étalée sur milieu avec histidine et sans 3-AT, sans histidine et sans 3-AT, et avec histidine et avec 0,5 mM ou 1 mM de 3-AT. Sur chaque boîte ont ensuite été déposés 1 µl de DMSO 100 %, 1 µl d'une solution d'une molécule x à 10 mM en DMSO, et 1 µl d'une solution d'une molécule y à 10 mM en DMSO. Les boîtes ont été incubées 48 heures à 30°C.
Figure 8C :
   Restauration de croissance par l'histidine :
      La souche contenant le système d'interférence transcriptionnelle UI (yRB49) et co-transformée par les plasmides pRB34 et pRB35 (interaction Fe65/App) a été étalée sur des milieux sans histidine et contenant 1 mM de 3-AT. On a déposé 1 µl d'uracile (U), d'adénine (A), de lysine (K) ou d'histidine (H), à 0,125 %, 0,25 %, 0,5 % ou 1 %, au centre de chaque boîte. Les boîtes ont été incubées 48 heures à 30°C.
Figure 9 :
   Détection d'inhibiteurs de l'interaction Fe65/App par restauration de croissance.
      La souche contenant le système d'interférence transcriptionnelle UI (yRB49) et co-transformée par les plasmides pRB34 et pRB35 (interaction Fe65/App) a été étalée sur milieu sans histidine et contenant 1 mM de 3-AT. On a déposé sur cette boîte 1 µl d'une solution à 10 mM d'une molécule 1, inhibiteur putatif de l'interaction Fe65/App, 1 µl d'une solution à 10 mM d'une molécule 2, inhibiteur putatif de l'interaction Fe65/App, et 1 µl d'une solution à 10 mM d'une molécule 3, inactive sur l'interaction Fe65/App. Les boîtes ont été incubées 48 heures à 30°C.
Figure 10 :
   Comparaison des interactions Fe65/App et p53/MDM2 pour l'activation du système d'interférence.
      La souche contenant le système d'interférence de transcription UI (yRB49) a été co-transformée par les couples de plasmides pRB34 et pRB35 (interaction Fe65/App), pDBT-mdm2 et pVP16-p53Nt (interaction mdm2/p53) ou pTAL20 et pDBT (contrôle). Pour chaque transformation, deux clones indépendants ont été mis en culture. Après une nuit d'incubation à 30°C, toutes les cultures ont été amenées à la même DO. Des séries de dilution de 10 en 10 de chaque culture ont été réalisées et une goutte de chaque dilution déposée sur milieu sans histidine et supplémenté avec différentes concentrations en 3-AT (de 1 à 32 mM). Les boîtes ont été incubées 48 heures à 30°C.
Figure 11 :
   Confirmation de la spécificité des inhibiteurs de l'interaction Fe65/App.
      La souche contenant le système d'interférence transcriptionnelle UI (yRB49) a été co-transformée par les couples de plasmides pRB34 et pRB35 (interaction Fe65/App) ou pDBT-mdm2 et pVP16-p53Nt (interaction mdm2/p53). La souche contenant les plasmides pRB34 et pRB35 (interaction Fe65/App) a été étalée sur 5 boîtes de milieu sans histidine supplémenté avec 1 mM de 3-AT (rangée du haut) ; la souche contenant les plasmides pDBT-mdm2 et pVP16-p53Nt (interaction mdm2/p53) a de même été étalée sur 5 boîtes de milieu sans histidine supplémenté avec 20 mM de 3-AT (rangée du bas)
      Une boîte contrôle a été réalisée pour chaque interaction en déposant 1 µl d'histidine à 0,125 % w/v au centre de cette boîte. Sur chacune des 4 autres boîtes a été déposée une molécule différente, A, B, C ou D, suivant le schéma suivant : dans le coin supérieur gauche est déposé 1 µl du produit à une concentration de 0,1 mM ; dans le coin supérieur droit, 1 µl du même produit à une concentration de 1 mM ; au centre, 1 µl de ce produit à 10 mM.
      Les boîtes ont été incubées 48 heures à 30°C.

La présente invention est illustrée à l'aide des exemples qui suivent, qui doivent être considérés comme illustratifs et non limitatifs.

Les techniques de biologie moléculaire utilisées dans les exemples qui suivent correspondent à des protocoles standard de biologie moléculaire et sont décrites par (Sambrook, J. *et al.,* 1989). Les séquences d'ADN ont été amplifiées en utilisant un système de PCR de fidélité élevée (Expand High Fidelity PCR System, Roche Diagnostics, Penzberg, Allemagne).

### EXEMPLE 1 : Construction du plasmide pRBV :

Un fragment de 761 pb correspondant au promoteur constitutif complet du gène *ADH1* est généré par PCR en utilisant comme amorces les oligonucléotides suivants : RB43 5'-AAAATCTAGAGGCGCCATATCCTTTTGTTGTTTCCGG-3' (SEQ ID N° 1) et RB44 5'- AAAAACGCGTGGCGCCCATCTTTCAGGAGGCTTGC -3' (SEQ ID N° 2) (les sites de restriction *Xba*I et *Mlu*I sont soulignés) et comme matrice de l'ADN génomique de la souche FL100 de *S. cerevisiae* (accessible notamment auprès de l'American Type Culture Collection (ATCC) (Manassas, Virginie, USA), sous le numéro 28383).

Deux oligonucléotides simple brin phosphorylés : RB75 de séquence 5'-CGCGTGGGGGGTTAATTAAAAAAAAGC-3' (SEQ ID N° 3) et RB76 de séquence 5'-GGCCGCTTTTTTTTAATTAACCCCCCA-3' (SEQ ID N° 4) sont hybridés afin d'obtenir un bras de liaison (linker) double brin présentant aux extrémités des bouts cohésifs compatibles *Mlu*I et *Not*I, respectivement. Pour cela les deux oligonucléotides simples brin sont mélangés à des concentrations équimolaires dans l'eau. Le mélange est chauffé à 90°C pendant 5 min dans un bloc de chauffage (heat block) puis le bloc est laissé à refroidir jusqu'à la température ambiante (environ 2 heures).

Afin de construire le vecteur pRB3, le fragment de 761 pb obtenu par PCR (fragment Padh1) est tout d'abord digéré par les enzymes de restriction *Xba*I et *Mlu*I. Le fragment ainsi digéré est ensuite inséré conjointement avec le linker dans le vecteur pBluescript KS+ (commercialisés par la société Stratagene (LaJolla, Californie, USA)) préalablement digéré par *Xba*I-*Not*I. Le plasmide ainsi obtenu est nommé pRB3.

Un fragment de 660 pb correspondant à la phase ouverte de lecture du gène *HIS3* est ensuite amplifié par PCR en utilisant comme matrice de l'ADN génomique de la souche FL100 de *S. cerevisiae* et comme amorces les oligonucléotides suivants : RB45 5'-AAAAACGCGTACAGAGCAGAAAGCCCTAG-3' (SEQ ID N° 5) et RB46 5'-AAAAAAGCGGCCGCGGCGCGCCTTAATTAACTACATAAGAACACCTTTG GTG -3' (SEQ ID N° 6) (les sites *Mlu*I, *Not*I et *Asc*I sont soulignés). Ce fragment a été digéré par les enzymes de restriction *Mlu*I et *Not*I et cloné dans le vecteur pRB3 préalablement linéarisé par les enzymes de restriction *Mlu*I et *Not*I. Le vecteur ainsi obtenu a été nommé pRB4.

Un fragment de PCR de 203 pb codant pour le terminateur du gène *ADH1* (Tadh1) est ensuite amplifié par PCR en utilisant comme matrice de l'ADN génomique de la souche FL100 de *S. cerevisiae* et comme amorces les oligonucléotides suivants : RB51 5'- AAAAGGCGCGCCTAATTCCGGGCGAATTTCT -3' (SEQ ID N° 7) et RB52 5'-AAAAGAGCTCTGCATGCCGGTAGAGGTG-3' (SEQ ID N° 8) (les sites *Asc*I et *Sac*I sont soulignés). Le fragment obtenu est digéré par les enzymes de restriction *Asc*I et *Sac*I et il est inséré dans le vecteur pRB4 préalablement digéré par les enzymes *Asc*I et *Sac*I. Le plasmide ainsi obtenu est dénommé pRB5.

Un fragment de 88 pb codant pour le terminateur du gène *CYC1* (Tcyc1) est obtenu par hybridation de deux oligonucléotides simple brin phosphorylés RB98 de séquence : et RB99 de séquence : Pour cela les deux oligonucléotides simples brin sont mélangés à des concentrations équimolaires dans l'eau. Le mélange est chauffé à 90°C pendant 5 min dans un bloc de chauffage (heat block) puis le bloc est laissé à refroidir jusqu'à la température ambiante (environ 2 heures). Le fragment double brin ainsi obtenu présente à ses extrémités des bouts cohésifs compatibles *EcoR*I et *Cla*I respectivement. Ceci permet l'insertion de ce fragment double brin (correspondant au terminateur Tcycl) dans le vecteur pRB5 décrit ci-dessus préalablement digéré par les enzymes *EcoR*I et *Cla*I. Le vecteur ainsi obtenu est dénommé pRBV.

La séquence de cette construction est vérifiée par un séquençage grâce au kit « Big Dye Terminator » (Perkin-Elmer, Wellesley, Massachusetts, USA).

### EXEMPLE 2 : Construction de systèmes d'interférence transcriptionnelle

### 2.1 : Construction du vecteur d'interférence aval pRB17 et du vecteur d'interférence aval non sens pRB16 :

Le vecteur d'interférence aval pRB17 (dénommé également DI pour « Dowstream Interference ») et le vecteur d'interférence aval non-sens pRB16 (dénommé également nDI pour « nonsense Dowstream Interference ») sont obtenus en insérant un fragment de PCR de 549 pb correspondant au promoteur Pgal1 dans le vecteur pRBV (cf. exemple 1).

Ce fragment de PCR est obtenu en utilisant comme matrice de l'ADN génomique de la souche FL100 de *S. cerevisiae* et comme amorces les oligonucléotides suivants : RB49 5'-AAAAGGCGCGCCGTAAAGAGCCCCATTATCTTAG-3' (SEQ ID N° 11) et RB50 5'-AAAAGGCGCGCCTTTGAGATCCGGGTTTTTTCT-3' (SEQ ID N° 12) (les sites de restriction *Asc*I sont soulignés). Ce fragment de PCR est ensuite digéré par l'enzyme *Asc*I et inséré dans le vecteur pRBV rendu linéaire avec l'enzyme *Asc*I. Le fragment peut s'insérer dans les deux sens ce qui permet d'obtenir les deux constructions : le vecteur d'interférence aval pRB17 (dénommé également DI et dans lequel Padh1 et Pgall sont orientés dans des sens opposés) (cf. figure 2A) et le vecteur d'interférence aval non-sens pRB16 (dénommé également nDI et dans lequel Padh1 et Pgal1 sont dans la même orientation) (cf. figure 2D).

La séquence de ces constructions est vérifiée par un séquençage grâce au kit « Big Dye Terminator » (Perkin-Elmer, Wellesley, Massachusetts, USA).

Le promoteur unidirectionnel *Pgal1* utilisé provient du promoteur divergent bidirectionnel *GAL1-10* (décrit par Johnston, M., et R. W. Davis., 1984), et contient quatre sites consensus « GAL4p-responsive DNA enhancer elements » ainsi que le promoteur de base du gène *GAL1*, mais ne présente pas la boîte TATA du promoteur du gène *GAL10.*
Le promoteur *GAL1* est régulé par le facteur de transcription GAL4p pour lequel un couple double-hybride (Y2H) est disponible (domaines AD et DBD séparés). Ce promoteur est bien caractérisé et a été utilisé avec succès dans des applications requérant l'expression inductible d'une protéine, comme pour la purification de protéines, l'étude de la fonction d'un gène essentiel etc...

### 2.2 : Construction du vecteur d'interférence amont pRB18 et du vecteur d'interférence amont non sens pRB 19 :

Afin de construire le vecteur d'interférence amont pRB 18 (dénommé également UI pour « Upstream Interference ») et le vecteur d'interférence amont non-sens pRB19 (dénommé également nUI pour « nonsense Upstream Interference »), une courte phase ouverte de lecture codant pour les acides aminés de l'extrémité carboxy de la protéine GST (C-ter GST) est générée par hybridation de deux oligonucléotides simple brin phosphorylés :

Pour cela les deux oligonucléotides simples brin sont mélangés à des concentrations équimolaires dans l'eau. Le mélange est chauffé à 90°C pendant 5 min dans un bloc de chauffage (heat block) puis le bloc est laissé à refroidir jusqu'à la température ambiante (environ 2 heures). Le fragment double brin ainsi obtenu présente aux extrémités des bouts cohésifs compatibles *Xma*I et *Xba*I, respectivement.

En parallèle, un fragment de 552 pb correspondant au promoteur du gène *GAL1* (il s'agit du même promoteur que celui utilisé pour la construction du vecteur d'interference aval pRB17, cependant le fragment est 3pb plus long que celui utilisé pour la construction aval puisqu'il contient un codon start ATG pour initier la traduction de la courte phase ouverte de lecture) a ensuite été amplifié par PCR. Ce fragment est obtenu en utilisant comme matrice de l'ADN génomique de la souche FL100 de *S. cerevisiae* et comme amorces les oligonucléotides suivants : RB55 5'-AAAACCCGGGGTAAAGAGCCCCATTATCTTAG -3' (SEQ ID N° 15) et RB56 5'-AAAACCCGGGCATTTTGAGATCCGGGTTTTTTC-3' (SEQ ID N° 16) (les sites *Xma*I sont soulignés). Ce fragment est ensuite digéré avec l'enzyme *Xma*I.

Une ligation à trois fragments est ensuite réalisée (les deux fragments obtenus ci-dessus + le vecteur pRBV). Cette ligation consiste en l'insertion du fragment Pgal1 digéré avec l'enzyme *Xma*I et de la courte phase ouverte de lecture C-ter GST (bouts cohésifs compatibles *Xma*I et *Xba*I) dans le vecteur pRBV (digéré préalablement par *XMa*I-*Xba*I). Le fragment Pgal1 peut s'insérer dans les deux sens. En conséquence, deux constructions sont obtenues : le vecteur d'interférence amont pRB18 (dénommé également UI et dans lequel Padh1 et Pgal1 sont dans la même orientation) (cf. figure 2B) et le vecteur d'interférence amont non sens pRB19 (dénommé également nUI et dans lequel Padh1 et Pgal1 sont orientés dans des sens opposés) (cf. figure 2E).

La séquence de ces constructions est vérifiée par un séquençage grâce au kit « Big Dye Terminator » (Perkin-Elmer, Wellesley, Massachusetts, USA).

### 2.3 : Construction du vecteur d'interférence amont-aval pRB27 :

Le plasmide d'interférence amont-aval pRB27 (dénommé également UDI pour « Upstream-Downstream Interference ») est construit en insérant le même fragment de PCR de 549 pb correspondant au promoteur Pgal1 que celui utilisé pour la construction de pRB16 et pRB17 (cf. exemple 2 : ce fragment de PCR est obtenu en utilisant comme matrice de l'ADN génomique de la souche FL100 de *S. cerevisiae* et comme amorces les oligonucléotides RB49 et RB50) dans le vecteur pRB18. Pour cela, le fragment de PCR est digéré par l'enzyme *Asc*I et inséré dans le vecteur pRB18 rendu linéaire par digestion avec l'enzyme *Asc*I. Le fragment pouvant s'insérer dans les deux sens, un plasmide avec l'insert dans l'orientation souhaitée (dans lequel les deux promoteurs Pgall sont orientés dans des sens opposés) est plus particulièrement sélectionné (par profil de restriction) (cf. figure 2C).
Le vecteur ainsi obtenu est nommé pRB27. La séquence de cette construction est vérifiée par un séquençage grâce au kit « Big Dye Terminator » (Perkin-Elmer, Wellesley, Massachusetts, USA).

### 2.4 : Principe de la construction de systèmes d'interférence transcriptionnelle :

La Demanderesse a conçu deux types de systèmes rY2H (reverse double-hybride) qui sont tous deux basés sur une mise sous silence de gène par interférence de transcription. Dans la première configuration, Pgal1 est situé en aval du gène *HIS3*, et Padh1 et Pgall présentent une orientation convergente. Cette configuration est nommée interférence aval (DI) car l'activation en aval de Pgal1 interfère avec la transcription du gène *HIS3*. Dans la deuxième configuration, Pgal1 est situé en amont de Padh1 et les deux promoteurs présentent une orientation en tandem. Par conséquent, cette configuration est nommée interférence en amont (UI) car l'activation de Pgal1 en amont interfère avec l'activité du promoteur Padh1.

Sur la base des configurations UI et DI, cinq constructions d'interférence de transcription rY2H ont été développés (cf. figure 2A à 2E).
Une activation de Pgal1 dans le système UI conduira à une réduction de l'activité de Padh1 en raison d'une interférence de transcription. L'activité de Pgal1 pourrait cependant encore permettre de produire un transcrit sens du gène *HIS3*. Afin d'empêcher la traduction de ce transcrit sens en IGP déshydratase fonctionnelle, une courte phase ouverte de lecture de 22 acides aminés suivi par deux codons stop a été insèrée entre Pgal1 et Padh1. Par conséquent, une interférence de transcription dans un système UI produit un messager bicistronique à partir duquel seul la première courte phase ouverte de lecture est traduite.
La troisième construction est une combinaison des systèmes UI et DI. Dans ce système dénommé système d'interférence amont-aval (UDI), Pgal1 est inséré en amont et en tandem par rapport à Padh1-*HIS3*, et une copie supplémentaire de Pgal1 est placée en aval de la phase ouverte de lecture de *HIS3* et ce dans une orientation convergente par rapport à Padh1 (cf. figure 2C).
Afin de contrôler la spécificité de l'interférence de transcription induite par Pgal1, un système d'interférence non sens amont (nUI) et non sens aval (nDI) ont été construits. Dans ces deux constructions contrôle, l'orientation de Pgal1 est inversée par rapport aux constructions UI et DI, respectivement. Aucune interférence transcriptionnelle ne devrait être observée lors de l'utilisation de ces constructions contrôle, même en présence d'une interaction Y2H valide.

Les cinq constructions décrites ci-dessus (les cinq inserts du plasmide) sont bordées à leurs extrémités par des terminateurs de transcription bidirectionnels (cf. exemple 1). On trouve ainsi d'un côté un fragment codant pour le terminateur de transcription du gène *CYC1* (Tcyc1) (décrit par Osborne, B. I., et L. Guarente., 1989) et de l'autre côté un fragment codant pour le terminateur du gène *ADH1* (Tadh1) (décrit par Irniger, S. et *al*., 1991.). Ces deux éléments arrêtent la transcription d'une manière bidirectionnelle. Ceci empêche l'extension de la transcription dirigée par Pgal1 ou Padh1 au-delà des sites de restriction *Sal*I/*Sac*I et protège également les systèmes rY2H de l'éventuelle influence d'activités de transcription en dehors de la cassette du gène rapporteur. Les constructions d'interférence ont été initialement construites sur des vecteurs bactériens et les sites de restriction *Sal*I et *Sac*I ont été utilisés pour un sous-clonage dans des vecteurs de réplication et d'intégration de levure (cf. ci-dessous).

### EXEMPLE 3 : Construction d'un nouveau vecteur d'intégration pRB2 :

Un fragment de PCR de 871 pb correspondant à la région +29 à +900 située en aval du codon stop TAA du gène *URA3* est généré par PCR en utilisant comme amorces les oligonucléotides suivants :
RB83 5'-AAAAAAGAGCTCTACTAAACTCACAAATTAGAGC-3' (SEQ ID N° 17) et RB84 5'-AAAAAAGAATTCGCGGCCGCAAATATACTGGGGAACCAGTC-3' (SEQ ID N° 18) (les sites *Eco*RI et *SacI* sont soulignés) et comme matrice de l'ADN génomique de la souche FL100 de *S*. *cerevisiae*.
Le produit de PCR est digéré par *EcoRI-Sac*I et cloné au niveau des sites *EcoRI*-*Sac*I du vecteur pFA6a-kanMX-PGAL1-3HA (décrit par Longtine, M. S., *et al*., 1998 et Wach A, *et al*., 1997 et dont le contenu technique en ce qui concerne la construction de ce vecteur est incorporé par référence à la présente demande). Le vecteur ainsi obtenu a été dénommé pRB 12.

En parallèle, un fragment de 769 pb correspondant à la région -223 à -992 située en amont de l'ATG du gène *URA3* est généré par PCR en utilisant comme amorces les oligonucléotides suivants :
RB85 5'-AAAAAACGTACGGCGGCCGCGATAAGGAGAATCCATACAAG-3' (SEQ ID N° 19) et RB86 5'-AAAAAACGTACGTTTATGGACCCTGAAACCAC-3' (SEQ ID N° 20) (les sites *Bsi*WI sont soulignés) et comme matrice de l'ADN génomique de la souche FL100 de *S. cerevisiae*. Le produit de PCR est digéré par l'enzyme *Bsi*WI et cloné au niveau du site unique *Bsi*WI de pRB12. Le fragment pouvant s'insérer dans les deux sens, un plasmide avec l'insert dans l'orientation souhaitée (dans lequel les deux promoteurs Pgal1 sont orientés dans des sens opposés) est sélectionné par analyse de profil de restriction.

La séquence de cette construction est vérifiée par un séquençage grâce au kit « Big Dye Terminator » (Perkin-Elmer, Wellesley, Massachusetts, USA).

Le vecteur pRB2 contient deux fragments homologues aux régions en amont et en aval, respectivement, de la phase ouverte de lecture du gène *URA3* de *S. cerevisiae*. Une séquence insérée entre ces deux fragments cible une intégration au niveau du locus *URA3*, son intégration génomique produisant une délétion de 1104 pb, ce qui élimine la phase ouverte de lecture de *URA3* (cf. figure 3).
Cette délétion de *URA3* est proche de la délétion publiée dans le modèle *ura3D0* (3), la différence par rapport à *ura3D0* étant que l'on enlève 22 pb de moins de la région en aval de *URA3* lors de l'intégration de pRB2.
Le gène *GEA2* est situé en amont de *URA3* et il a été montré précédemment, que le niveau de transcription et la taille des transcrits du gène *GEA2* ne sont pas affectés par la délétion de *ura3D0* (Brachmann, C. B., *et al*., 1998).
Le gène *TIM9* est localisé en aval de *URA3* et est un gène essentiel à la levure *S*. *cerevisiae*. Il n'a pas été rapporté de taux de survie réduit pour les souches *ura3D0*, *TIM9* s'avère rester entièrement fonctionnel dans les souches *ura3D0*. Par conséquent, la délétion *ura3D0* s'avère ne pas affecter la transcription des gènes adjacents.
Par conséquent, les systèmes d'interférence intégrés avec pRB2 n'affecteront probablement pas ni ne seront affectés par la transcription de *GEA2* et *TIM9*. Un avantage des vecteurs pRB2 est que l'on peut également les utiliser avec des souches mutantes d'*URA3* comme *ura3-52* et *ura3D0.*

### EXEMPLE 4 : Sous-clonage des différents inserts des vecteurs d'interférence dans le vecteurs d'intégration et de réplication de levure pRB2 :

Les différents systèmes d'interférences et de contrôles (DI, nDI, UI, nUI et UDI) décrits dans les exemples 2 (cf. Figure 2A à 2E) sur des vecteurs pBluescript sont sous-clonés sous forme de fragments *Sal*I-*Sac*I dans le vecteur d'intégration pRB2. Pour cela les différents plasmides pRB16, pRB17, pRB 18, pRB19 et pRB27 sont digérés par les enzymes *Sal*I-*Sac*I et l'insert est sous-cloné dans le vecteur d'intégration pRB2 préalablement digéré par les enzymes *Sal*I-*Sac*I. Les différentes constructions obtenues ont été dénommées pRBV31, pRB23, pRB24, pRB25 et pRB29 respectivement (cf. table 2).

Après intégration des systèmes d'interférence dans le génome, le gène rapporteur *HIS3* est en tandem par rapport aux gènes *GEA2* et *TIM9.*

**Table 2 : Vecteurs et souches d'interférence.**

| *Constructions* | *Dans le vecteur pBluescript KS*+ | *Dans pLac33* | *Dans pRB2* | *Nom de la souche une fois la construction intégrée* |
|---|---|---|---|---|
| nDI | pRB16 | - | pRB31 | yRB82 |
| DI | pRB17 | pRB20 | pRB23 | yRB48 |
| UI | pRB18 | pRB21 | pRB24 | yRB49 |
| nUI | pRBV19 | - | pRB25 | yRB50 |
| UDI | pRB27 | pRBV28 | pRB29 | yRB83 |

| | | | | |
|---|---|---|---|---|
| Abréviations : nDI : interférence aval non sens; DI : interférence aval; UI : interférence amont; nUI : interférence amont non sens; UDI : interférence amont-aval. | | | | |

### EXEMPLE 5 : Construction de souches d'interférence transcriptionnelle avec intégration génomique des vecteurs d'interférence :

### 5.1 : Intégration génomique des vecteurs d'interférence :

Les milieux de culture utilisés sont tels que décrits par Gutherie, C. et G. R. Fink, 2002. Pour toutes les expériences, on fait croître les cellules dans un milieu minimal défini YNB (Difco Laboratories, Detroit, USA) + glucose à 2% (Sigma-Aldrich, Lyon, France) à 30°C. Pour compléter les marqueurs d'auxotrophie, on ajoute les acides aminés (Sigma-Aldrich, Lyon, France) requis.

La souche yCM15 (*MAT a* Δ*gal4* Δ*gal80 ura3-52 lys2-801 his3*Δ*200 trp1-*Δ*63 leu2 ade2-101*) est utilisée pour les constructions de souches avec intégration génomique. La souche yCM15 a été construite à partir de la souche PCY2 (décrite par Chevray PM et Nathans D., 1992), dans laquelle la cassette rapporteuse a été éliminée.

La souche PCY2 (*MAT* α Δ*gal4* Δ*gal80 URA3::GAL1-lacZ lys2-801 ade2-101 trp1-*Δ*63 his3-*Δ*200 leu2*) est issue d'un croisement entre la souche YPH499 (*MAT a ura3-52 lys2-801 ade2-101 trp1-*Δ*63 his3-*Δ*200 leu2-*Δ*1;* ATCC 76625, 204679; (Sikorski and Hieter, 1989)) (accessible notamment auprès de l'American Type Culture Collection (ATCC) (Manassas, Virginie, USA), sous le numéro 204679) avec la souche GGY1::171 (*MAT* α Δ*gal4* Δ*gal80 leu2 his3 URA3::GAL1-lacZ*) (Gill and Ptashne, 1987).

La souche GGY1:171 a été construite par l'intégration de la cassette *URA3::GAL1-ZacZ* (Yocum et al, 1984), qui porte le gène rapporteur *lacZ* sous le contrôle du promoteur du gène *GAL1* activé par UAS_{G}, dans la souche GGY1 (*MAT* α Δ*gal4* Δ*gal80 leu2 his3* (Gill and Ptashne, 1987)). La souche GGY1 est elle-même le résultat d'un croisement entre la souche DBY745 (*MAT* α Δ*gal4* Δ*gal80 leu2-3,112 ade1-100 ura3-52*; (Graham and Chambers, 1996 ; Ferreiro et al., 2004; Nagarajan and Storms, 1997 ; Dormer et al., 2000) et la souche YM709 (Pearlberg, J., 1995).

A la place de la souche PCY2, toute souche de levure *S. cerevisiae* ayant le génotype (*gal4- gal80- trp1- his3- leu2- ura3-)* peut être utilisée. Par exemple, il peut être utilisé à la place de la souche PCY2, la souche Y187 (décrite par Harper, J. W. *et al*., 1993 et accessible notamment auprès de l'American Type Culture Collection (ATCC) (Manassas, Virginie, USA), sous le numéro de référence 96399). La table 3 présente d'autres exemples de souches pouvant se substituer à la souche PCY2. Il est présenté leurs désignations, une courte description de ces souches et leurs numéros de référence auprès de l'American Type Culture Collection (ATCC) (Manassas, Virginie, USA), organisme auprès duquel ces souches sont accessibles.

Afin de construire la souche yCM15, 10⁶ à 10⁷ cellules d'un clone de la souche PCY2 sont suspendues dans de l'eau stérile et étalées sur une boîte de gélose de milieu YNB + glucose 2 % complété par les acides amines lys + ade + his + ura + trp + leu. La boîte de gélose contient également du 5-fluoroorotic acide (5-FOA) a une concentration de 1mg/ml, le 5-FOA est toxique pour les cellules exprimant le gène *URA3*. En conséquence, on sélectionne avec ces boîtes contenant du 5-FOA, des mutants de la souche PCY2 qui ont perdu la cassette rapporteuse *URA3::GAL1-lacZ* (Boeke et al., 1987). L'absence de la cassette rapporteuse dans les mutants obtenus est vérifiée en testant l'activité du rapporteur *lacZ.* Les mutants sont ensuite transformés avec le plasmide pCL1 (Fields, S., et O. Song. 1989) codant pour le facteur de transcription GAL4p qui est un très fort activateur du promoteur *GAL1* et l'activité *lacZ* est mesurée (Breeden, L. et K. Nasmyth, 1985). Un mutant résistant au 5-FOA et sans activité *lacZ* est sélectionné et nommé yCM15.

**Table 3 : Souche pouvant se substituer à la souche PCY2**

| **Designation** | **Description** | **Référence ATCC** |
|---|---|---|
| CTY10-5D | Saccharomyces cerevisiae Hansen, teleomorph (budding yeast) | 201449 |
| PJ69-4A | Saccharomyces cerevisiae Hansen, teleomorph (budding yeast) | 201450 |
| JC981 | Saccharomyces cerevisiae Hansen, teleomorph (budding yeast) | 204096 |
| JC993 | Saccharomyces cerevisiae Hansen, teleomorph (budding yeast) | 204097 |
| Y166 | Saccharomyces cerevisiae Hansen, teleomorph (budding yeast) | 96397 |
| YAS1760 | Saccharomyces cerevisiae Hansen, teleomorph (budding yeast) | MYA-2200 |
| YAS1924 | Saccharomyces cerevisiae Hansen, teleomorph (budding yeast) | MYA-2210 |
| Y190 | Saccharomyces cerevisiae Hansen, teleomorph (budding yeast) | MYA-320 |

Les vecteurs d'intégration pRB31, pRB23, pRB24, pRB25 et pRB29 sont linéarisés par digestion avec l'enzyme *Drd*I. La souche yCM15 est transformée indépendamment par 1 µg d'ADN linéarisé de chacun des vecteurs d'intégration en utilisant la méthode de transformation à l'acétate de lithium (telle que décrite par Gietz, R. D. et A. Sugino., 1988) et les cellules sont mises à pousser sur un milieu dépourvu histidine (YNB (Difco Laboratories, Detroit, USA) + glucose à 2% (Sigma-Aldrich, Lyon, France) complété par les acides aminés lys + ade + trp + leu et de l'uracile (Sigma-Aldrich, Lyon, France). Les clones auxotrophes pour l'histidine sont isolés et l'intégration des cassettes rapporteuses au niveau du locus *URA3* est vérifiée par PCR de colonie (décrites par Gutherie, C *et al*, 2002) en utilisant des amorces internes et externes spécifiques pour chaque jonction (voir Table 4).

**Table 4 :**

| | jonction *URA3* upstream | | | jonction *URA3* downstream | | |
|---|---|---|---|---|---|---|
| vecteur | Amorce 1 | amorce 2 | taille produit PCR (bp) | amorce 1 | amorce 2 | taille produit PCR (bp) |
| pRB23 | RB103 | RB61 | 1183 | RB68 | RB109 | 1252 |
| pRB24 | RB103 | RB68 | 1155 | RB66 | RB109 | 1395 |
| pRB25 | RB103 | RB38 | 1294 | RB66 | RB109 | 1395 |
| pRB29 | RB103 | RB68 | 1155 | RB68 | RB109 | 1253 |
| pRB31 | RB103 | RB61 | 1183 | RB38 | RB109 | 1389 |

| | | | | | | |
|---|---|---|---|---|---|---|
| Les séquences des amorces utilisées sont les suivantes : RB38 : 5'-GGGGTAATTAATCAGCGAAGCGATG-'3 (SEQ ID N° 21) RB61 : 5'-CGATGTATGGGTTTGGTTG-'3 (SEQ ID N° 22) RB66 : 5'-TCTTGCGAGATGATCCCGC-'3 (SEQ ID N° 23) RB68 : 5'-TAAGCGTATTACTGAAAGTTCC-'3 (SEQ ID N° 24) RB103 : 5'-GCAATGAAAGAGCAGAGCGAGAG-'3 (SEQ ID N° 25) RB109 : 5'-GTTATCAGATATTATCAGGTGGGAA-'3 (SEQ m N° 26) | | | | | | |

Les souches obtenues par la transformation indépendante de la souche yCM15 par chacun des vecteurs d'intégration pRBV31, pRB23, pRB24, pRB25, pRBV29, et dont l'intégration de chacune des cassettes rapporteuses au niveau du locus *URA3* a été démontrée, ont été dénommées yRB82, yRB48, yRB49, yRB50, yRB83 respectivement (cf table 2).

### 5.2 : Activation des constructions d'interférence intégrées :

Afin d'évaluer l'activité des systèmes d'interférence, les souches rapporteuses obtenues à l'exemple 5.1 sont transformées avec deux paires de plasmides. On utilise la première paire pour étudier la réponse de systèmes d'interférence vis-à-vis d'une activation puissante de Pgal1 en raison de l'expression du facteur de transcription de GAL4p de longueur complète (Fields, S. et O. Song, 1989). La deuxième paire de plasmides permet l'expression du domaine d'activation (AD) et du domaine de liaison à l'ADN (DBD) de GAL4p séparément et sert de contrôle négatif car les domaines distincts seuls ne peuvent pas activer Pgal1.

Ainsi les souches obtenus dans l'exemple 5.1 (souches yRB82, yRB48, yRB49, yRB50, yRB83) sont co-transformées par le plasmide centromérique pCL1 codant pour le facteur de transcription de GAL4p (décrit par Fields, S., et O. Song. 1989) et le plasmide sans insert centromérique pFL39 (décrit par Bonneaud N, *et al*., 1991). Ces plasmides se répliquant chez la levure, portent le gène *LEU2* ou *TRP1* comme marqueur de sélection.

Les transformants sont sélectionnés sur un milieu YNB (Difco Laboratories, Detroit, USA) + glucose à 2% (Sigma-Aldrich, Lyon, France) complété par les acides aminés lys + ade+ his et de l'uracile (Sigma-Aldrich, Lyon, France).

Les transformants obtenus sont dénommés respectivement nDI+, DI+, UI+, nUI+ et UDI+.

Les contrôles négatifs sont obtenus en co-transformant les souches décrites dans l'exemple 5.1 (souches yRB82, yRB48, yRB49, yRB50, yRB83) par les vecteurs pTAL20 et pDBT. Les vecteurs pTAL20 et pDBT permettent l'expression du domaine d'activation (AD) de GAL4p et du domaine de liaison à l'ADN (DBD) de GAL4p (ces vecteurs sont décrits par Navarro, P., *et al*., 1997).
Les transformants ainsi obtenues sont dénommées respectivement nDI-, DI-, UI-, nUI- et UDI-.

### EXEMPLE 6 : Etude de la croissance de levure_ayant un système d'interférence transcriptionnelle intégré dans leur génome:

La croissance cellulaire des transformants est étudiée par une méthode d'évaluations en goutte. Pour cela, on dépose les cellules sur des boîtes de gélose contenant de l'histidine et on les incube pendant une nuit à 30°C. Puis, on met en suspension les cellules dans de l'eau à approximativement 10⁶ cellules/ml et on prépare des séries de dilutions d'un facteur 10. On prélève à la pipette des gouttes de 5 µl de chaque dilution et les dépose sur des boîtes de gélose pré-séchées comprenant le milieu souhaité. On incube les boîtes à 30°C.

La croissance cellulaire des transformants d'interférence est ainsi évaluée sur milieu dépourvu d'histidine. Un inhibiteur compétitif de la protéine HIS3p (le 3-amino-1,2,4-triazole (3-AT)) est éventuellement ajouté au milieu de culture à des concentrations croissantes afin d'observer des différences au niveau du niveau d'activité du gène rapporteur *HIS3* entre les différentes souches d'interférence (Fig. 4). Les milieux contenant l'inhibiteur sont préparés à partir d'une solution stock à la concentration de 1M de l'inhibiteur compétitif 3-amino-1,2,4-triazole (3-AT; Sigma) dans de l'eau de qualité Millipore. La gélose est refroidie jusqu'à 60°C avant l'addition du 3-AT aux concentrations voulues. Les différents milieux de culture testés sont un milieu minimum (YNBG (Difco Laboratories, Detroit, USA) + glucose à 2% (Sigma-Aldrich, Lyon, France) complété par les acides aminés lys + ade et de l'uracile (Sigma-Aldrich, Lyon, France) sans 3-AT ou avec 3-AT à des concentrations de 8, 16, 22, 32 mM. Un contrôle positif est également réalisé (milieu de culture avec histidine et sans 3-AT). Les boîtes sont incubées à 30°C pendant 48 à 72 heures. Une partie des résultats est présentée en figure 4A à 4E.

En présence de GAL4p (souches +), on observe la réduction la plus forte de la croissance cellulaire pour les transformants UI+. Les cellules UI exprimant GAL4p ne peuvent pas croître en absence d'histidine alors que les cellules portant le contrôle AD/DBD (transformants UI-) croissent normalement à des concentrations en 3-AT supérieures à 32 mM. Ni GAL4p ni le contrôle AD/DBD n'inhibent la croissance cellulaire des transformants contrôle nUI+ et nUI-, ce qui montre que inhibition de croissance des cellules UI+ exprimant GAL4p est due à l'activation de la transcription de Pgal1 dirigée vers Padh1.

Dans les cellules DI+, l'expression de GAL4p n'affecte pas la croissance cellulaire lorsque l'inhibiteur 3-AT est absent (cf. figure 4B), mais la croissance est totalement inhibée lorsque du 3-AT est présent à 8 mM ou à des concentrations supérieures (cf. figure 4C à 4E). Au contraire, la croissance cellulaire des transformants contrôle DBD/AD (transformants DI-) n'est pas affectée même à des concentration de 3-AT de 32 mM (cf. figure 4E). Par conséquent, la transcription dirigée par Pgal1 du brin anti-sens du gène *HIS3* augmente significativement le niveau d'auxotrophie pour l'histidine des cellules DI+.

De manière surprenante, GAL4p inhibe également la croissance cellulaire dans les transformants nDI+ (la croissance est inhibée lorsque du 3-AT est présent à 8 mM ou à des concentrations supérieures (cf. figure 4C à 4E). Dans ces cellules, la transcription dirigée par Pgal1 est dirigée vers Tadh1 (cf. figure 2D), on ne s'attend pas à ce qu'elle interfère avec l'expression de *HIS3*. L'inhibition de croissance est due à l'activation spécifique de Pgal1 car on n'observe pas d'inhibition de croissance lorsque le contrôle AD/DBD est présent dans les cellules nDI- (cf. figure 4B à 4E).

En présence de GAL4p, les transformants UDI+ montre un niveau d'inhibition de croissance similaire à celui de cellules DI+. Le contrôle DBD/AD n'affecte pas la croissance des cellules UDI- (cf. figure 4B à 4E).

Nous pouvons conclure que dans les souches UI+, DI+ et UDI+, l'activation dûe à GAL4p du promoteur Pgal1 conduit à une inhibition significative de la croissance cellulaire et que cette inhibition est due à une activité réduite du rapporteur *HIS3*. L'inhibition de croissance est légèrement plus forte dans les cellules UI que dans les cellules DI et UDI.

La réduction induite par GAL4p de l'activité de *HIS3* ralentit donc significativement la croissance cellulaire ; toutefois, elle ne l'inhibe pas complètement. Alors que dans des conditions d'interférence on ne détecte pas de colonies sur les boîtes pendant les 6-7 premiers jours d'incubation, de minuscules colonies deviennent visibles après des temps d'incubation plus longs.

Nous avons observé que l'interférence est plus puissante avec le système UI qu'avec les systèmes DI, UDI ou nDI, ce qui peut provenir du mécanisme moléculaire générant l'interférence de transcription. Des études détaillées d'interférence en amont ont montré qu'une activation de promoteur en amont réduit l'activité du promoteur en aval en enlevant les facteurs d'activation de leurs sites de liaison au niveau du promoteur en aval (Greger, I. H. *et al*., 2000, Greger, I. H. *et al*., 1998, Valerius, O.C. *et al*., 2002, Callen BP *et al*., 2004). Le promoteur générant une interférence étant plus proche du promoteur du gène rapporteur dans la construction UI, l'interférence peut être plus efficace dans la construction UI que dans 1a DI.

Il est également possible qu'une interférence en aval provienne d'un mécanisme moléculaire différent. Dans le système DI, des ARN polymérases transcrivant de manière convergente peuvent entrer en collision et ceci peut mener à un arrêt de la transcription et à une mise sous silence du gène *HIS3*. Une collision frontale d'ARN polymérases pourrait être un moyen moins efficace de mise sous silence de *HIS3* que l'enlèvement de facteurs d'activation de transcription de Padh1. En outre, il est possible que dans le système DI, l'activation de Padh1 inactive Pgal1 par une interférence de transcription, ce qui peut également expliquer pourquoi le système UDI n'est supérieur ni au système UI ni au système DI.

De manière surprenante, la demanderesse a observé qu'une interférence de transcription en aval existe également lorsque Pgall est inséré en tandem et en aval par rapport à la cassette rapporteuse *HIS3* (construction nDI). Le niveau de transcription d'un gène dépend de la puissance du promoteur ainsi que de la qualité du signal d'arrêt de transcription (Wahle, E., and U. Ruegsegger, 1999). Pgal1 étant inséré entre l'ORF de *HIS3* et son terminateur de transcription Tadh1, une activation de Pgal1 pourrait interférer avec un arrêt approprié du transcrit de *HIS3* dans la construction nDI, ce qui réduit ainsi l'activité de HIS3p dans les cellules.

### EXEMPLE 7 : Sous-clonage des différents inserts des vecteurs d'interférence dans le vecteur pLac33 :

Afin de tester si les systèmes d'interférence transcriptionnelle sont fonctionnelles lorsqu'elles sont présents sur un plasmide et non intégrées au niveau génomique, les différents systèmes d'interférences (DI, UI, et UDI) décrits dans l'exemple 2 (cf. Figure 2A à 2E) sur des vecteurs pBluescript sont sous-clonés sous forme de fragments *Sal*I-*Sac*I dans le vecteur d'intégration pLac33. Ce vecteur contient le gène *URA3* de la levure comme marqueur de sélection et a été décrit par Gietz, R. D., et A. Sugino. 1988. Pour cela les différents plasmides pRB27, pRB17, pRB18 sont digérés par les enzymes *Sal*I-*Sac*I et l'insert est sous-cloné dans le vecteur replicatif pLac33 préalablement digéré par les enzymes *SalI-Sac*I. Les différentes constructions obtenues ont été dénommées pRB28, pRB20, pRB21 respectivement.

### EXEMPLE 8 : Etude de la croissance de levure ayant un système d'interférence transcriptionnelle non intégré dans leur génome:

Il a également été étudié si les constructions d'interférence de transcription UI, DI et UDI sont également fonctionnelles lorsqu'elles sont présentes sur un plasmide et non intégrées au niveau génomique. Pour cela, les vecteurs dérivés du plasmide pLac33 portant les différentes constructions d'interférence transcriptionnelle et décrit à l'exemple 7, ont été utilisés pour transformer indépendamment la souche yCM15.

La souche yCM15 est co-transformée indépendamment par 0.3µg d'ADN de chacun des vecteurs pRB28, pRBV20 ou pRB21 (cf. exemple 7) associés aux vecteurs pCL1 et pFL39. La souche yCM15 est donc transformée indépendamment par trois groupes de trois plasmides (pRB28 + pCL1 + pFL39 ou pRB20 + pCL1 + pFL39 ou pRB21 + pCL1 + pFL39). Le plasmide centromérique pCL1 code pour le facteur de transcription de GAL4p (décrit par Fields, S., et O. Song. 1989) et le plasmide pFL39 est un plasmide sans insert centromérique (décrit par : Bonneaud N, et al., 1991). Ces plasmides de réplication de levure portent le gène *LEU2* ou *TRP1* comme marqueur de sélection.

La souche yCM15 est également co-transformée indépendamment par 0.3µg d'ADN de chacun des vecteurs pRB28, pRB20 ou pRB21 (cf. exemple 7) associés aux vecteurs pTAL20 et pDBT (permettant l'expression du domaine d'activation (AD) de GAL4p et du domaine de liaison à l'ADN (DBD) de GAL4p (ces vecteurs sont décrits par Navarro, P., *et al*., 1997)). La souche yCM15 est donc transformée indépendamment par trois groupes de trois plasmides (pRB28 + pTAL20 + pDBT, ou pRB20 + pTAL20 + pDBT ou pRB21 + pTAL20 + pDBT).

La croissance cellulaire de chacun de ces triples transformants est étudiée sur boîtes de gélose avec et sans histidine et pour les boîtes sans histidine, sans ou avec des concentrations croissantes en 3-AT, comme décrit dans l'exemple 6.

Les résultats obtenus avec les constructions d'interférence basées sur des plasmides non intégrés sont similaires à ceux obtenus pour les constructions d'interférence intégrées (cf. figure 5A à 5D). Ainsi lorsque le milieu est dépourvu d'histidine et de 3-AT, les cellules UI exprimant GAL4p (triple transformants pRB21 + pCL1 + pFL39) montrent une croissance très fortement réduite comparée aux cellules UI n'exprimant pas de GAL4p active (triple transformants pRB21 + pTAL20 + pDBT). Le niveau d'inhibition de croissance de UI n'est toutefois pas aussi puissant que celui observé avec la version intégrée de la construction UI (cf. exemple 6).

Les cellules DI (triple transformants pRB20 + pCL1 + pFL39 (exprimant une GAL4p active) ou pRB20 + pTAL20 + pDBT (n'exprimant pas une GAL4p active)) et UDI (triple transformants pRB28 + pCL1 + pFL39 ((exprimant une GAL4p active) ou pRB28 + pTAL20 + pDBT (n'exprimant pas une GAL4p active) croissent quasi normalement qu'elles expriment ou pas de GAL4p active.

Lorsque le milieu contient du 3-AT à 8 mM ou à une concentration supérieure, les cellules exprimant GAL4p et transformées par la construction UI, DI ou UDI ne croissent plus du tout (cf. figure 5C et 5D). Ce résultat contraste avec ceux obtenus pour les cellules contrôle négatif (n'exprimant pas de GAL4p active (AD/DBD)) et transformées par les plasmides d'interférence dont la croissance cellulaire n'est pas affectées (cf. figure 5C et 5D).

La Demanderesse conclut que des versions basées sur des plasmides de UI, DI et UDI sont fonctionnelles mais légèrement moins sensibles à une activation de Pgal1 que les versions intégrées correspondantes des constructions d'interférence.

### EXEMPLE 9 : Indépendance des systèmes d'interférence de transcription vis à vis de l'arrière-plan génétique de la souche hôte :

Afin d'étudier si l'activité des systèmes d'interférence de transcription dépend de l'arrière-plan génétique de la souche hôte, il a également été évalué des constructions basées sur des plasmides (telles que décrit à l'exemple 7) dans une deuxième souche de levure: la souche yRB31. La souche yRB31 (*MAT* α *ura3-52 his3-*Δ*200 ade2-101 trp1-901 leu2-3,112 met-* Δ*gal4* Δ*gal80*) est construite en éliminant la cassette *URA3::GAL1(UAS)-GAL1(TATA)-lacZ* de la souche Y187 (la souche Y187 a été décrite Harper, J. W. *et al*., 1993 et est accessible notamment auprès de l'American Type Culture Collection (ATCC) (Manassas, Virginie, USA), sous le numéro de référence 96399).

Afin de construire la souche yRB31, 10⁶ a 10⁷ cellules d'un clone de la souche Y187 sont suspendues dans l'eau stérile et étalées sur une boîte de gélose de milieu YNB + glucose 2 % complété par les acides amines met + ade + his + ura + trp + leu. La boîte de gélose contient également du 5-fluoroorotic acide (5-FOA) à une concentration de 1 mg/ml, le 5-FOA est toxique pour les cellules exprimant le gène URA3. En conséquence on sélectionne avec ces boîtes contenant du 5-FOA, des mutants de la souche Y187 qui ont perdu la cassette rapporteuse *URA3::GAL1(UAS)-GAL1(TATA)-lacZ* (Boeke et al., 1987). L'absence de la cassette rapporteuse dans les mutants obtenus est vérifiée en testant l'activité du rapporteur *lacZ.* Les mutants sont transformés avec le plasmide pCL1 (Fields S. et O. Song, 1989) codant pour le facteur de transcription GAL4p qui est un très fort activateur du promoteur *GAL1* et l'activité *lacZ* est mesurée (Breeden L. et K. Nasmyth, 1985). Un mutant résistant au 5-FOA et sans activité *lacZ* est sélectionné et nommé yRB31.

La souche yRB31 a été transformée de la même manière que décrit à l'exemple 8 pour la souche yCM15. Les transformants obtenus ont été étudiés en ce qui concerne leur croissance cellulaire de la même façon que décrit à l'exemple 8. Il n'a pas été observé de différence entre les souches yCM15 et yRB31 en ce qui concerne l'activité des plasmides d'interférence.

### EXEMPLE 10 : Induction de l'expression des systèmes d'interférence UI et DI par l'interaction protéines-protéine et effet sur l'inhibition de la croissance cellulaire :

### 10.1 : Induction de l'expression des systèmes d'interférence par l'interaction de la protéine Fe65 avec la protéine APP précédemment identifiée:

Il a été étudié si une interaction protéine-protéine précédemment identifiée entre les protéines Fe65 et APP (McLoughlin DM et Miller CC, 1996 ; Fiore F *et al*., 1995 ; Zambrano N *et al*., 1997) est capable d'induire suffisamment une construction d'interférence de transcription pour inhiber la croissance cellulaire de levure.

Les versions intégrées des constructions UI et DI sont utilisées. Les protéines de fusion AD-Fe65 et DBD-AppCt sont exprimées sous le contrôle du promoteur *ADH1* tronqué fortement actif (promoteur décrit par Ruohonen L, *et al*., 1995).

Le plasmide pRB34 permet l'expression de la protéine de fusion AD-Fe65 sous le contrôle d'un promoteur de *ADH1* tronqué fortement actif.

La construction du plasmide pRB34 a été effectuée en plusieurs étapes. Dans une première étape une partie du cadre de lecture du gène Fe65 est cloné dans le vecteur pGAD424 (BD Biosciences Clontech, (Palo Alto, Californie, USA)). Un fragment de PCR de 1694 pb correspondant aux acide amines 152 à 708 du gène Fe65 (numéro d'accès Genebank : BC010854) est généré par PCR en utilisant comme amorces les oligonucléotides suivants :
RB250 5'- AGATCGAATTCAAGGCGGCCGGGGAGGCCGAGG-3' (SEQ ID N° 27) et RB251 5'- GCAGGTCGACTCATGGGGTATGGGCCCCCAGC-3' (SEQ ID N° 28) (les sites *EcoRI* et *Sal*I sont soulignés) et comme matrice une banque l'ADN plasmidique d'ADNc de cerveau humain commercial (BD Biosciences Clontech, (Palo Alto, Californie, USA)). Le produit de PCR est digéré par les enzymes *EcoRI-Sal*I et cloné au niveau des sites *Eco*RI-*Sal*I du vecteur pGAD424. Le vecteur ainsi obtenu est dénommé pRBSG11. La séquence de cette construction est vérifiée par un séquençage grâce au kit « Big Dye Terminator » (Perkin-Elmer, Wellesley, Massachusetts, USA).

Dans une deuxième étape, une digestions partielle du plasmide pRBSG11 est effectuée avec l'enzyme *Sph*I et le fragment codant pour le promoteur *ADH1* tronqué faiblement actif, la protéine de fusion AD-Fe65 et le terminateur *ADH1* est inséré dans le site *Sph*I du plasmide centromerique pFL36 (décrit par Bonneaud N, *et al*., 1991), ceci permet d'obtenir le plasmide pRBIM54. L'obtention de la bonne construction est vérifiée par analyse de restriction. Le fragment peut s'insérer dans le deux sens et un clone présentant l'orientation souhaitée, dans lequel le promoteur *ADH1* est proche du site unique *Sac*I du plasmide pFL36, est sélectionné. Dans une troisième étape, un fragment codant pour le promoteur *ADH1* tronqué fortement actif est amplifié par PCR à partir de l'ADN génomique de la souche FL100 avec les amorces RB136 (5'-TTGTAAAACGACGGCCAGTGAATTCCGTACGATATCCTTTTGTTGTTTCCGG GTG-3') (SEQ ID N° 29) et RB137 (5'-AGTTGATTGTATGCTTGGTATAGC-3') (SEQ ID N° 30). En utilisant la méthode de clonage par recombinaison homologue (GAP repair) (DeMarini *et al*., 2001; Orr-Weaver *et al*., 1983), le fragment de PCR est utilisé pour remplacer le promoteur *ADH1* tronqué faiblement actif par le promoteur *ADH1* tronqué fortement actif dans le plasmide pRBIM54 préalablement digéré par l'enzyme *Sac*I. La recombinaison homologue est effectuée dans la souche yCM15. Le nouveau plasmide construit est vérifié par analyse de restriction et est nommé pRB34. La séquence de ce plasmide est présentée en SEQ ID N° 31.

Le plasmide pRB35 permet l'expression de la protéine de fusion DBD-AppCt sous le contrôle d'un promoteur de *ADH1* tronqué fortement actif. La construction du plasmide pRB35 a été effectuée en plusieurs étapes.

Dans une première étape la partie carboxy-terminal du cadre de lecture du gene HSAFPA4 (App, numéro d'accès EMBL Y00264) est cloné dans le vecteur pGBT9 (BD Biosciences Clontech, (Palo Alto, Californie, USA)). Un fragment de 143pb codant pour les acides amines 650 à 695 du gène HSAFPA4 est obtenu par hybridation de deux oligonucléotides simple brin phosphorylés de séquence : RB252 5'-AATTCAAGAAACAGTACACATCCATTCATCATGGTGTGGTGGAGGTTGACG CCGCTGTCACCCCAGAGGAGCGCCACCTGTCCAAGATGCAGCAGAACGGCT ACGAAAATCCAACCTACAAGTTCTTTGAGCAGATGCAGAACTAGG-3' (SEQ ID N° 32) et RB253 de séquence :
5'-TCGACCTAGTTCTGCATCTGCTCAAAGAACTTGTAGGTTGGATTTTCGTA GCCGTTCTGCTGCATCTTGGACAGGTGGCGCTCCTCTGGGGTGACAGCGGC GTCAACCTCCACCACACCATGATGAATGGATGTGTACTGTTTCTTG -3' (SEQ ID N° 33). Pour cela les deux oligonucléotides simples brin sont mélangés à des concentrations équimolaires dans l'eau. Le mélange est chauffé à 90°C pendant 5 min dans un bloc de chauffage (heat block) puis le bloc est laissé à refroidir jusqu'à la température ambiante (environ 2 heures). Le fragment double brin ainsi obtenu présente à ses extrémités des bouts cohésifs compatibles *EcoR*I et *Sal*I, respectivement. Ceci permet l'insertion de ce fragment double brin (correspondant à la partie carboxy-terminal de HSAFPA4) dans le vecteur pGBT9 (BD Biosciences Clontech). Le vecteur ainsi obtenu permet l'expression de la protéine de fusion entre GAL4DBD et les acides amines 650 à 695 de App (DBD-AppCt) et est dénommé pSG14. La séquence de cette construction est vérifiée par un séquençage grâce au kit « Big Dye Terminator » (Perkin-Elmer, Wellesley, Massachusetts, USA).

Dans une deuxième étape, le plasmide pSG14 est partiellement digéré avec l'enzyme *Sph*I. Puis le fragment codant pour le promoteur *ADH1* tronqué faiblement actif, la fusion DBD-AppCt et le terminateur *ADH1* est inséré dans le vecteur pFL39 (décrit par Bonneaud N, *et al*., 1991) préalablement digéré par l'enzyme *Sph*I. Le plasmide résultant est nommé pIM55. L'obtention de la bonne construction est vérifiée par analyse de restriction. Le fragment peut s'insérer dans les deux sens et un clone présentant l'orientation souhaitée, dans lequel le promoteur *ADH1* est proche du site unique *Sac*I du plasmide pFL36, a été sélectionné.

Le plasmide pRB35 est alors obtenu en échangeant le promoteur *ADH1* tronqué faiblement actif de pIM55 par un promoteur *ADH1* tronqué fortement actif. Le même fragment de PCR que celui utilisé pour la construction de pRB34 est introduit dans pIM55 préalablement digéré par l'enzyme *Sph*I en utilisant la même méthode de GAP repair. Le plasmide construit est vérifié par analyse de restriction et est dénommé pRB35. La séquence de ce plasmide est présentée en SEQ ID N° 34.

Dans un premier temps l'activité des constructions a été vérifiée. Pour cela, la souche reportrice Y187 (la souche Y187 possède le gène *URA3* placé sous le contrôle du promoteur Pgall et a été décrite par Harper, J. W. *et al*., 1993 et est accessible notamment auprès de l'American Type Culture Collection (ATCC) (Manassas, Virginie, USA), sous le numéro de référence 96399) est transformée par trois couples de plasmides. Ces couples sont les suivants : pRB34 + pRB35, pRB34 + pDBT et pTAL20 + pRB35. Deux clones co-transformés par chacun de ces couples de plasmides sont sélectionnés. Leur capacité à pousser sur milieu minimum avec et sans uracile est étudié. Il est montré que les six clones correspondant sont capables de pousser sur milieu minimum en présence d'uracile (cf. figure 6A). Toutefois, en absence d'uracile, seul les deux clones co-transformés par les plasmides pRB34 + pRB35 sont capables de pousser (cf. figure 6A). Ceci montre que le gène *URA3* est activé dans la souche Y187 et ce grâce à l'interaction entre AD-Fe65 et DBD-AppCt reconstituant un facteur de transcription GAL4p actif capable d'activer le promoteur Pgal1. L'activité des constructions utilisées est donc démontrée.

Les souches yRB48 (système DI) et yRB49 (système UI) sont ensuite co-transformées chacune par le couple de plasmides pRB34 et pRB35 permettant l'expression des deux protéines hybrides AD-Fe65 et DBD-AppCt. Les souches yRB48 (système DI) et yRB49 (système UI) sont également co-transformées chacune par les couples de plasmides pRB34 + pDBT et pTAL20 + pRB35 comme contrôles. Deux clones co-transformés par chacun de ces couples de plasmides sont sélectionnés. La croissance cellulaire de chacun des deux clones de ces doubles transformants est étudiée sur boîtes de gélose avec et sans histidine et sur boîtes sans histidine et avec ou sans concentrations croissantes en 3-AT, comme décrit dans l'exemple 6.

Comme montré à la Figure 6b à 6E, l'interaction entre les protéines de fusion AD-Fe65 et DBD-AppCt inhibe la croissance cellulaire des souches portant les constructions d'interférence UI et DI alors que les cellules exprimant la protéine de fusion AD-Fe65 et le domaine DBD ou la protéine de fusion DBD-AppCt et le domaine AD croissent normalement. Dans cette expérience, les différences au niveau de la croissance cellulaire sont détectées lorsqu'on ajoute du 3-AT à des concentrations supérieures à 15mM.

Il peut être conclu qu'une interférence de transcription induite par une interaction double-hybride peut être aisément détectée avec les systèmes rY2H UI et DI.

### 10.2 : Induction de l'expression des systèmes d'interférence par l'interaction de la protéine p53 avec la protéine mdm2 précédemment identifiée:

Il a également été étudié si une autre interaction protéine-protéine précédemment identifiée entre les protéines p53 et mdm2 (Oliner JD *et al*., 1993; Chen J *et al*., 1993; Momand J *et al*., 1992) est capable d'induire suffisamment une construction d'interférence de transcription pour inhiber la croissance cellulaire de levure. Pour cela, la version intégrée de la construction UI est utilisées. Les protéines de fusion DBD-mdm2 et VP16-p53(1-55) sont exprimées sous le contrôle du promoteur complet du gène *ADH1* à partir des plasmides 2µ.

Afin de construire le vecteur pVP16-p53Nt la phase ouverte de lecture codant pour les 55 acides aminés de l'extrémité amino de la protéine p53 humaine (reference genebank : BC003596) est générée par hybridation de deux oligonucléotides simple brin phosphorylés : (les sites *Nol*I et *Bam*HI sont soulignées). Pour cela les deux oligonucléotides simples brins complémentaires sont mélangés à des concentrations équimolaires dans l'eau. Le mélange est chauffé à 90°C pendant 5 min dans un bloc de chauffage (heat block) puis le bloc est laissé à refroidir jusqu'à la température ambiante (environ 2 heures). Le fragment double brin ainsi obtenu présente aux extrémités des bouts cohésifs compatibles *Not*I et *Bam*H1, respectivement.

Puis une ligation de fragment est réalisée dans le vector pVP16 (Vojtek and Hollenberg, 1995; Vojtek et al., 1993) digéré préalablement par les enzymes *Not*I-*Bam*HI. La séquence de cette construction est vérifiée par un séquençage grâce au kit « Big Dye Terminator » (Perkin-Elmer, Wellesley, Massachusetts, USA).
Afin de construire le vecteur pDTB-mdm2 le gène codant pour la protéine humaine Mdm2 entière (référence genbank : NM_002392) est amplifié par PCR en utilisant le vecteur pCR3-mdm2fl (Sigalas *et al*., 1996) comme matrice et les oligonucléotides suivant:
RB202 (5'-CCCGGGAATTCAGATCCATATGTGCAATACCAACATGTCTGTAC-3') (SEQ ID N° 37) et
RB203 (5'-ACTTAGAGCTCTAGGGGAAATAAGTTAGCACAATC-3') (SEQ ID N° 38) (les sites *EcoRI* et *Sac*I sont soulignés). Le produit de PCR est digéré par les enzymes *Eco*RI et *Sac*I et cloné au niveau des sites *Eco*RI et *Sac*I dans le vecteur pDBT (Navarro *et al*., 1997). La séquence de cette construction est vérifiée par un séquençage grâce au kit «Big Dye Terminator » (Perldn-Elmer, Wellesley, Massachusetts, USA).

L'activité des constructions obtenues a été vérifiée. Pour cela, la souche reportrice Y187 (la souche Y187 possède le gène URA3 placé sous le contrôle du promoteur Pgal1 et a été décrite par Harper, J. W. *et al*., 1993 et est accessible notamment auprès de l'American Type Culture Collection (ATCC) (Manassas, Virginie, USA), sous le numéro de référence 96399) est transformée par trois couples de plasmides. Ces couples sont les suivants : pDBT-mdm2 + pVP16-p53Nt, pVP16-p53Nt + pDBT et pTAL20 + pDBT-mdm2. Deux clones co-transformés par les deux plasmides sont sélectionnés pour chacun de ces couples de plasmides. Leur capacité à pousser sur milieu minimum avec et sans uracile est étudiée. Il est montré que les six clones correspondant sont capables de pousser sur milieu minimum en présence d'uracile (cf. figure 6F). Toutefois, en absence d'uracile, seul les deux clones co-transformés par les plasmides pDBT-mdm2 + pVP16-p53Nt sont capables de pousser. Ceci montre que le gène *URA3* est activé dans la souche Y187 et ce grâce à l'interaction entre DBD-mdm2 et VP16-p53(1-55) reconstituant un facteur de transcription actif capable d'activer le promoteur Pgall. L'activité des constructions utilisées est donc démontrée.

La souche yRB49 (système UI) est ensuite co-transformées par le couple de plasmides pDBT-mdm2 + pVP16-p53Nt permettant l'expression des deux protéines hybrides DBD-mdm2 et VP16-p53(1-55). La souche yRB49 (système UI) est également co-transformées par le couple de plasmides pDBT-mdm2 + pTAL20 comme contrôles. Deux clones co-transformés par les deux plasmides sont sélectionnés pour chacun de ces deux couples de plasmides. La croissance cellulaire de chacun des deux clones de ces doubles transformants est étudiée sur boîtes de gélose avec et sans histidine et sur boîtes sans histidine et avec ou sans des concentrations croissantes en 3-AT, comme décrit dans l'exemple 6. Comme montré à la Figure 6G, l'interaction entre les protéines de fusion DBD-mdm2 et VP16-p53(1-55) inhibe la croissance cellulaire des souches portant les constructions d'interférence UI alors que les cellules exprimant la protéine de fusion la protéine de fusion DBD-mdm2 et le domaine AD croissent normalement.

Dans cette expérience, les différences au niveau de la croissance cellulaire sont détectées en présence d'une concentration de 3-AT égal ou supérieure à 5 mM..

Il peut être conclu qu'une interférence de transcription induite par une interaction double-hybride peut être aisément détectée avec les systèmes rY2H UI.

### EXEMPLE 11 : Application du système d'interférence transcriptionnelle rY2H UI au criblage d'inhibiteurs d'interactions protéine-protéine :

### 11.1 : Calibration du test de restauration de la croissance de levures sur milieux solides.

Une souche de levure contenant un système d'interférence transcriptionnelle rY2H UI intégré dans son génome (yRB49) a été utilisée pour isoler des inhibiteurs d'interactions protéine-protéine par un test de restauration de croissance.

Pour réaliser ce test, une souche de levure contenant le système d'interférence transcriptionnelle rY2H UI, couplé à une interaction protéine-protéine spécifique, est étalée sur milieu solide contenant du 3-AT. Des gouttes de solutions de composés chimiques sont déposées sur la boîte ensemencée. La diffusion des composés chimiques dans la gélose créée un gradient de concentration autour de la zone de dépôt. Les produits capables d'inhiber l'interaction protéine-protéine permettent la croissance des cellules, ce qui se traduit par un halo de restauration de croissance entourant la zone de dépôt.

Afin de déterminer les conditions pour le développement d'un tel test, une série d'expériences préliminaires a été réalisée. On a ainsi cherché à évaluer, premièrement, la quantité de cellules à déposer sur les boîtes de Petri et, deuxièmement, la concentration minimale de 3-AT dans le milieu de culture permettant d'inhiber la croissance des cellules.

Tout d'abord, des levures contenant une version intégrée du système UI (yRB49) ont été co-transformées par les plasmides pRB34 et pRB35 afin de mettre le système d'interférence transcriptionnelle sous la dépendance d'un couple d'interaction protéine-protéine Fe65/App. Ces levures sont ensuite cultivées en milieu liquide contenant de l'histidine. Dès que la densité optique (DO) de la culture atteint la valeur de 3 (3. 10⁷ cellules par ml), les cellules sont centrifugées 10 minutes à 3000 rpm, lavées deux fois avec de l'eau stérile et remise en solution à une DO = 0,1 ou 0,01. On étale 10 ml de cette suspension uniformément sur toute la surface du milieu de culture solide préalablement coulé en boîtes de Petri carrées de 120 mm de côté. Après une minute, le liquide est éliminé par aspiration. Les boîtes sont séchées pendant 15 minutes sous une hotte à flux laminaire puis incubées à 30°C pendant 48 heures.

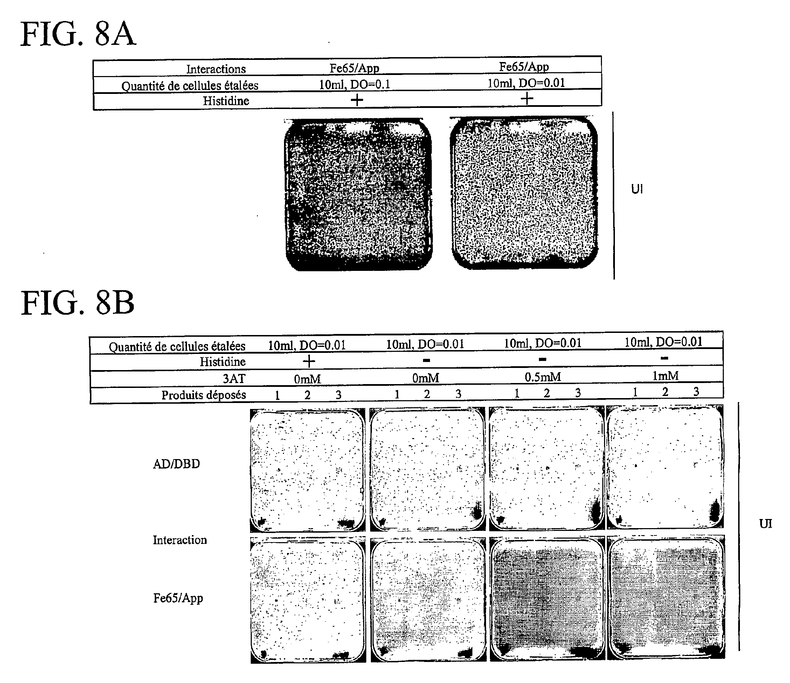

La figure 8A montre que l'ensemencement optimal des boîtes a été atteint avec la dilution la plus basse, soit DO = 0,01 (boîte de droite).

Dans le but de déterminer la concentration minimale de 3-AT capable d'inhiber la croissance de la levure, la souche yRB49 a été transformée comme précédemment par les plasmides pRB34 et pRB35 (Fe65/App) ou par les plasmides de contrôle pTAL20/pDBT (n'exprimant pas d'interactions protéine-protéine). Les deux souches transformées ont été étalées sur milieu, soit contenant de l'histidine, soit dépourvu d'histidine et supplémenté avec 0, 0,5 ou 1 mM de 3-AT. Comme le montre la figure 8B, la souche portant l'interaction Fe65/App présente une croissance très ralentie sur milieu contenant 1 mM de 3-AT, comparée à celle contenant les plasmides de contrôle, dont la croissance n'est pas affectée dans les mêmes conditions.

Dans la même expérience, afin de tester rapidement la toxicité des molécules en solution dans ce test de restauration de croissance, deux molécules ont été sélectionnées au hasard dans une librairie de 10 000 composés. Après mise en solution à 10 mM dans le DMSO, 1 µl de chacune a été déposé sur chaque boîte, ainsi que 1 µl de DMSO. Aucune de ces deux molécules n'est capable de restaurer la croissance des levures. Cependant, elles produisent un disque d'inhibition de croissance autour de la zone de dépôt des molécules, lié aux effets toxiques de ces molécules à forte concentration (Figure 8B).

Un ajout d'histidine dans le milieu corrige la carence de la souche pour la synthèse d'histidine en l'affranchissant du système d'interférence transcriptionnelle. Afin de confirmer que les conditions expérimentales décrites ci-dessus permettent de détecter une restauration de croissance de la levure, la souche yRB49 co-transformée par le couple de plasmides pRB34 et pRB35 (interaction Fe65/App) a été étalée sur le milieu précédent dépourvu d'histidine et supplémenté avec 1 mM 3-AT, puis des quantités croissantes d'uracile, d'adénine, de leucine ou d'histidine, ont été déposés au centre de chaque boîte.

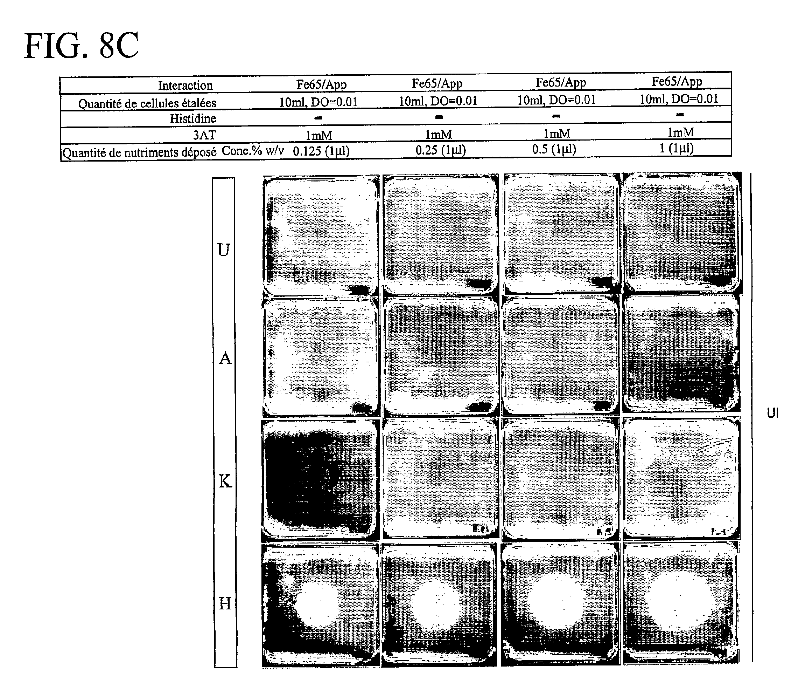

Comme le montre la figure 8C, seule l'histidine est capable de restaurer la croissance de la levure autour du dépôt et de façon dépendante de sa concentration.

### 11.2 : Détection d'une restauration de la croissance de la levure induite par un inhibiteur d'interactions protéine-protéine.

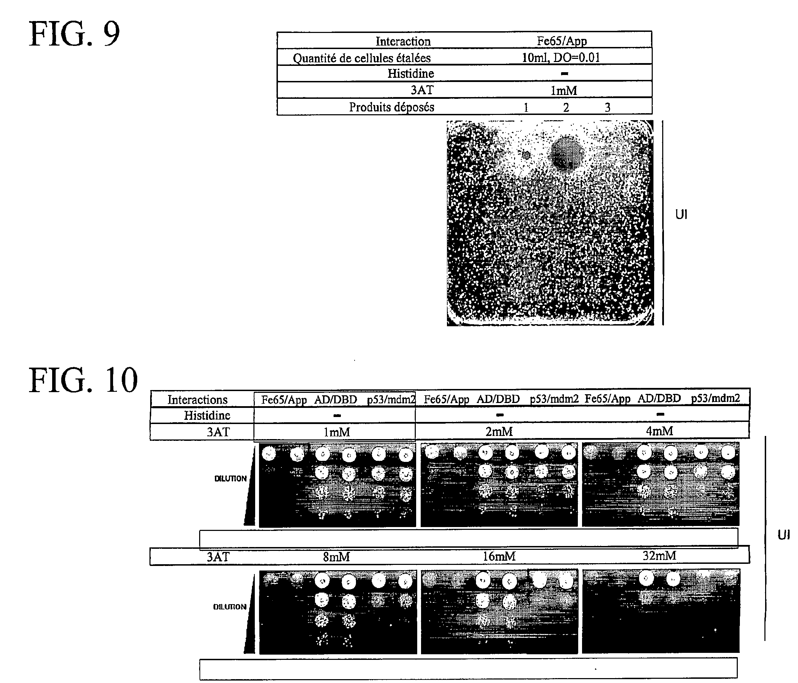

Les conditions expérimentales reportées ci-dessus ont été utilisées à grande échelle pour cribler une collection de 10 000 molécules. Dans ces conditions, chaque molécule de la collection a été déposée, à raison d'environ 0,2 µl d'une solution à 10 mM DMSO, grâce à un réplicateur à 96 aiguilles, sur milieu solide dépourvu d'histidine et contenant 1 mM de 3-AT. La plate-forme robotique utilisée pour ce criblage a été décrite précédemment par Beydon et collaborateurs (2000). Deux molécules ont ainsi été isolées qui sont capables de restaurer la croissance des levures : pour chacun de ces deux produits, un halo blanc intense de restauration de croissance est facilement distinguable autour d'une zone d'inhibition de croissance liée un effet toxique de ces molécules à fortes concentrations, alors que pour le produit de contrôle 3 aucun effet n'est détecté, comme attendu (figure 9).

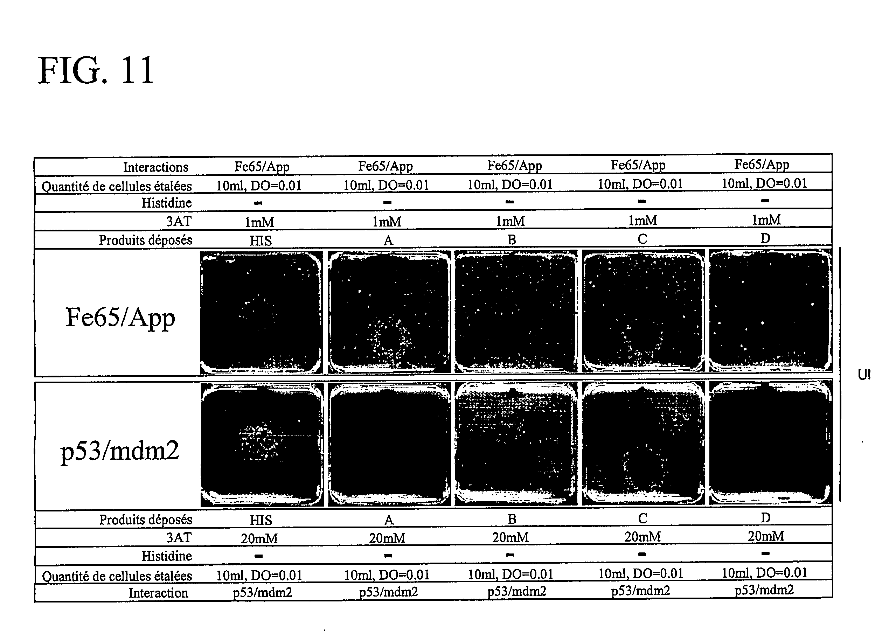

Pour confirmer la spécificité d'action de molécules isolées avec ce test, l'effet de trois autres molécules potentiellement inhibitrices de l'interaction Fe65/App (appelées A, B et C sur la Figure 11) a été testé sur un autre type d'interaction protéine-protéine utilisant ce système d'interférence, l'interaction p53/mdm2 (cf exemple 10.2).

Tout d'abord, la réponse relative du système d'interférence UI en fonction des interactions Fe65/App et p53/mdm2 a été testée par un test en goutte. On a ainsi observé que le complexe p53/mdm2 inhibe la croissance des levures sur milieu sans histidine mais à une concentration plus forte en 3-AT que celle utilisée avec la souche contenant l'interaction Fe65/App (Figure 10). Ceci suggère que l'interaction p53/mdm2 pourrait être plus faible que l'interaction App/Fe65 ou bien que les interactants sont moins bien exprimés dans la levure.

On a ensuite transformé la souche yRB49 par le couple de plasmides pRB34 et pRB35 conduisant à l'expression de l'interaction Fe65/App ou le couple pDBT-mdm2 et pVP16-p53Nt conduisant à l'expression de l'interaction p53/mdm2.

Les deux souches Fe65/App et p53/mdm2 ont été chacune étalées sur des boîtes de milieu dépourvu d'histidine et supplémenté respectivement avec 1 mM et 20 mM de 3-AT. Pour chacune des trois molécules potentiellement inhibitrices, on a déposé trois gouttes de 1 µl de solutions à 0,1, 1 et 10 mM en DMSO par boîte. De plus, 1 µl d'histidine (0,125 % w/v) a été déposé au centre d'une autre boîte, comme témoin positif de restauration de croissance. On a utilisé comme témoin négatif un produit pris au hasard dans la collection et utilisé aux même concentrations que les trois molécules testées.

Les résultats présentés Figure 11 montrent que les produits A' et B sont actifs, puisqu'ils induisent un halo de restauration de croissance de la souche Fe65/App, dont la taille est dépendante de la concentration utilisée. On n'observe pas un tel halo fonction de la concentration pour la souche p53/mdm2, ce qui démontre la spécificité de l'effet inhibiteur de ces deux molécules. Par contre, le produit C induit la croissance des deux souches, Fe65/App et p53/mdm2, et peut donc être classé comme actif mais non spécifique. Comme attendu, le produit D n'a pas d'effet sur les deux souches testées ; il est considéré comme inactif.
Le test de restauration de croissance permet donc d'isoler à partir d'une librairie de plusieurs milliers de molécules, des molécules qui sont des inhibiteurs spécifiques d'une interaction protéine/protéine.
Toutes les publications et brevets cités sont incorporés à la présente demande par référence.

### Bibliographie :

- Alber, T., and G. Kawasaki. (1982). J Mol Appl Genet 1: 419-434.
- Bartel, P. L., C.-T. Chien, R. Sternglanz, and S. Fields. 1993. Using the two-hybrid system to detect protein-protein interactions, p. 153-179. In D. A. Hartley (ed.), Cellular interactions in development: a practical approach. Oxford University Press, Oxford.
- Beydon, M-H., A. Fournier, L. Drugeault and J. Beyquard. (2000). J.Mol.Screen 5: 12-21
- Boeke, J. D., J. Trueheart, et al. 1987. Methods Enzymol 154: 164-75.
- Bonneaud N, Ozier-Kalogeropoulos O, Li GY, Labouesse M, Minvielle-Sebastia L, Lacroute F. 1991. Yeast. 7(6): 609-15.
- Brachmann, C. B., A. Davies, G. J. Cost, et al. 1998. Yeast 14:115-32.
- Breeden, L. and K. Nasmyth. 1985. Regulation of the yeast HO gene. Cold Spring Harb Symp Quant Biol 50: 643-50.
- Burke, T. W., J. G. Cook, M. Asano, and J. R. Nevins. 2001. J Biol Chem 276: 15397-408.
- Cagney, G., P. Uetz, and S. Fields. 2000. Methods Enzymol 328: 3-14.
- Callen BP, Shearwin KE, Egan JB. 2004. Mol Cell. 14(5): 647-56.
- Causier, B., and B. Davies. 2002. Plant Mol Biol 50: 855-70.
- Chen J, Marechal V, Levine AJ. 1993. Mol Cell Biol. 13(7): 4107-14.
- Chevray PM and Nathans D. Proc Natl Acad Sci U S A. 1992. 89(13): 5789-93.
- Chien, C. T., P. L. Bartel, R. Sternglanz, and S. Fields. 1991. Proc Natl Acad Sci U S A 8b8: 9578-82.
- Coates, P. J., and P. A. Hall. 2003. J Pathol 199: 4-7.
- Crispino, J. D., M. B. Lodish, J. P. MacKay, and S. H. Orkin. 1999. Mol Cell 3: 219-28.
- Daros, J. A., M. C. Schaad, and J. C. Carrington. 1999. J Virol 73: 8732-40.
- Das, G. C., Niyogi, S. K., and Salzman, N. P. 1985. Prog Nucleic Acid Res Mol Biol 32: 217-236.
- DeMarini, D. J., C. L. Creasy, et al. 2041. Biotechniques 30(3): 520-3.
- Dormer, U. H., Westwater, J., McLaren, N. F., Kent, N. A., Mellor, J., and Jamieson, D. J. 2000. J Biol Chem 275: 32611-32616.
- Elledge, S. J., and Spottswood, M. R. 1991. Embo J 10: 2653-2659.
- Ernst, J. F. (1986). DNA 5, 483-491.
- Ferreiro, J. A., Powell, N. G., Karabetsou, N., Kent, N. A., Mellor, J., and Waters, R. 2004. Nucleic Acids Res 32: 1617-1626.
- Fields, S., and O. Song. 1989. Nature 340: 245-6.
- Fiore F, Zambrano N, Minopoli G, Donini V, Duilio A, Russo T. 1995. J Biol Chem. 270(52): 30853-6.
- Foecking, M. K., and Hofstetter, H. 1986. Gene 45: 101-105.
- Gietz, R. D., and A. Sugino. 1988. Gene 74: 527-34.
- Gill, G. and M. Ptashne. 1987. Cell 51(1): 121-6.
- Graham, I. R., and Chambers, A. 1996. Curr Genet 30: 93-100
- Greger, I. H., A. Aranda, and N. Proudfoot. 2000. Proc Natl Acad Sci U S A 97: 8415-20.
- Greger, I. H., F. Demarchi, M. Giacca, and N. J. Proudfoot. 1998. Nucleic Acids Res 26: 1294-301.
- Greger, I. H., and N. J. Proudfoot. 1998. Embo J 17: 4771-9.
- Gutherie, C., and G. R. Fink (ed.). 2002. Guide to yeast genetics and molecular cell biology, vol. 350. Academic press, San Diego, CA.
- Gyuris, J., Golemis, E., Chertkov, H., and Brent, R. 1993. Cell 75, 791-803.
- Harper, J. W., G. R. Adami, N. Wei, K. Keyomarsi, and S. J. Elledge. 1993. Cell 75: 805-16.
- Huang, J., and S. L. Schreiber. 1997. Proc Natl Acad Sci U S A 94: 13396-401.
- Irniger, S., C. M. Egli, and G. H. Braus. 1991. Mol Cell Biol 11: 3060-9.
- Johnston, M., and R. W. Davis. 1984. Mol Cell Biol 4: 1440-8.
- Kaufer, N. F., H. M. Fried, W. F. Schwindinger, M. Jasin, and J. R. Warner. 1983. Nucleic Acids Res 11: 3123-35.
- Kronman, C., Velan, B., Gozes, Y., Leitner, M., Flashner, Y., Lazar, A., Marcus, D., Sery, T., Papier, Y., Grosfeld, H., and et al. 1992. Gene 121: 295-304.
- Leanna, C. A., and M. Hannink. 1996. Nucleic Acids Res 24: 3341-7.
- Longtine, M. S., A. McKenzie, 3rd, D. J. Demarini, N. G. Shah, A. Wach, A. Brachat, P. Philippsen, and J. R. Pringle. 1998. Yeast 14: 953-61.
- McLoughlin DM and Miller CC. 1996. FEBS Lett. 397(2-3): 197-200.
- Martens, J. A., Laprade, L., and Winston, F. 2004. Nature 429, 571-574.
- Mizushima, S., and Nagata, S. 1990. Nucleic Acids Res 18: 5322.
- Momand J, Zambetti GP, Olson DC, George D, Levine AJ. 1992. Cell. 69(7): 1237-45.
- Nacken, V., Achstetter, T., and Degryse, E. (1996). Gene 175, 253-260.
- Nagarajan, L., and Storms, R. K. 1997. J Biol Chem 272: 4444-4450.
- Navarro, P., P. Durrens, and M. Aigle. 1997. Biochim Biophys Acta 1343: 187-92.
- Nenoi, M., Mita, K., Ichimura, S., Cartwright, I. L., Takahashi, E., Yamauchi, M., and Tsuji, H. 1996. Gene 175: 179-185.
- Oliner JD, Pietenpol JA, Thiagalingam S, Gyuris J, Kinzler KW, Vogelstein B. 1993. Nature. 362(6423): 857-60.
- Orr-Weaver, T. L. and J. W. Szostak. 1983. Proc Natl Acad Sci U S A 80(14): 4417-21.
- Osborne, B. I., and L. Guarente. 1989. Proc Natl Acad Sci U S A 86: 4097-101.
- Pearlberg, J. 1995. Studies of the yeast transcription factor GAL11. Ph.D. thesis. Harvard University, Cambridge, Mass.
- Peterson, J. A., and A. M. Myers. 1993. Nucleic Acids Res 21: 5500-8.
- Picard, D., Schena, M., and Yamamoto, K. R. 1990. Gene 86, 257-261.
- Puig, S., J. E. Perez-Ortin, and E. Matallana. 1999. Curr Microbiol 39: 369-0373.
- Puthalakath, H., D. C. Huang, L. A. O'Reilly, S. M. King, and A. Strasser. 1999. Mol Cell 3: 287-96.
- Ren, B., Robert, F., Wyrick, J. J., Aparicio, O., Jennings, E. G., Simon, I., Zeitlinger, J., Schreiber, J., Hannett, N., Kanin, E., et al. 2000. Science 29: 2306-2309.
- Ruohonen, L., M. K. Aalto, and S. Keranen. 1995. J Biotechnol 39: 193-203.
- Sambrook, J., E. F. Fritsch, and T. Maniatis. 1989. Molecular cloning: A laborator manual. Cold Spring Habor Lab. Press, Plainview, NY.
- Schorpp, M., Jager, R., Schellander, K., Schenkel, J., Wagner, E. F., Weiher, H., and Angel, P. 1996. Nucleic Acids Res 24: 1787-1788.
- Serebriiskii, I., Khazak V. and Golemis, E.A. 1999. J Biol Chem 274, 17080-17087.
- Serebriiskii, I. G., V. Khazak, and E. A. Golemis. 2001. Biotechniques 30: 634-6, 638, 640 passim.
- Sigalas, I., A. H. Calvert, et al. 1996. Nat Med 2(8): 912-7.
- Sikorski, R. S., and Hieter, P. 1989. Genetics 122: 19-27.
- Springer, C., O. Valerius, A. Strittmatter, and G. H. Braus. 1997. J Biol Chem 272: 26318-24.
- Steiner, S., and Philippsen, P. 1994. Mol Gen Genet 242, 263-271.
- Svetlov, V. V., and Cooper, T. G. (1995). Yeast 11, 1439-1484.
- Valerius, O., C. Brendel, K. Duvel, and G. H. Braus. 2002. J Biol Chem 277: 21440-5.
- Vidal, M., R. K. Brachmann, A. Fattaey, E. Harlow, and J. D. Boeke. 1996. Proc Natl Acad Sci U S A 93: 10315-20.
- Vidal, M., and H. Endoh. 1999. Trends Biotechnol 17: 374-81.
- Vojtek, A. B., S. M. Hollenberg, et al. 1993. Cell 74(1): 205-14.
- Vojtek, A. B. and S. M. Hollenberg 1995. Methods Enzymol 255: 331-42.
- Wach A, Brachat A, Alberti-Segui C, Rebischung C, Philippsen P. 1997. Yeast. 13(11): 1065-75.
- Wahle, E., and U. Ruegsegger. 1999. FEMS Microbiol Rev 23: 277-95.
- Xu, C. W., Mendelsohn, A. R., and Brent, R. 1997. Proc Natl Acad Sci U S A 94, 12473-12478.
- Weinstock, K. G., and Strathern, J. N. 1993. Yeast 9, 351-361.
- Winzeler EA, Shoemaker DD, Astromoff A et al. 1999. Science 285(5429), 901-6.
- Yamamoto, T., de Crombrugghe, B., and Pastan, I. 1980. Cell 22: 787-797.
- Yocum, R. R., Hanley, S., West, R., Jr., and Ptashne, M. 1984. Mol Cell Biol 4: 1985-1998.
- Young, K., S. Lin, L. Sun, E. Lee, M. Modi, S. Hellings, M. Husbands, B. Ozenberger, and R. Franco. 1998. Nat Biotechnol 16: 946-50.
- Zambrano N, Buxbaum JD, Minopoli G et al. 1997. J Biol Chem. 272(10): 6399-405.
- Zutshi, R., M. Brickner, and J. Chmielewski. 1998. Curr Opin Chem Biol 2: 62-6.

## Revendications

1. Cellule comportant une construction d'ADN d'interférence, ladite construction comprenant :
- Un gène rapporteur placé sous le contrôle d'un premier promoteur,
- Un ou plusieurs promoteur(s) inductible(s), dit(s) promoteur(s) d'interférence, choisi(s) et positionné(s) de manière à ce que son (leur) activation entraîne une interférence transcriptionnelle du premier promoteur, conduisant à une diminution détectable de l'expression du gène rapporteur,
ladite cellule exprimant en outre :
- Une première protéine chimère (Y-AD) constituée d'un domaine d'activation de la transcription (AD) fusionné à une protéine Y susceptible d'interagir avec une protéine partenaire X,
- Une deuxième protéine chimère (X-DBD) constituée d'un premier domaine liant l'ADN (DBD) fusionné à un deuxième domaine constitué par une protéine X susceptible d'interagir avec la protéine Y, l'interaction des deux protéines chimères X-DBD et Y-AD aboutissant à la constitution d'un facteur de transcription fonctionnel activant le ou les promoteur(s) d'interférence.

2. Cellule selon la revendication 1 **caractérisée en ce que** le promoteur régulant l'expression du gène rapporteur est un promoteur inductible, la protéine Y est susceptible d'interagir avec deux protéines partenaires X et Z, et **en ce que** la cellule exprime une troisième protéine chimère (Z-DBD) constituée d'un domaine liant l'ADN (DBD), fusionné à un deuxième domaine constitué par une protéine Z susceptible d'interagir avec la protéine Y, l'interaction des deux protéines chimères Z-DBD et Y-AD aboutissant à la constitution d'un facteur de transcription fonctionnel activant l'expression du gène rapporteur.

3. Cellule selon la revendication 2 **caractérisée en ce que** le promoteur inductible régulant l'expression du gène rapporteur comprend une séquence susceptible d'interagir avec le domaine liant l'ADN (DBD) de la protéine chimère (Z-DBD).

4. Cellule selon la revendication 1 **caractérisée en ce que** le promoteur régulant l'expression du gène rapporteur est un promoteur constitutif

5. Cellule selon l'une des revendications 1 à 4 **caractérisée en ce qu'**il s'agit d'une cellule hôte transformée ou transfectée par la construction d'ADN d'interférence et par des constructions d'ADN codant pour les protéines chimères, l'ensemble de ces constructions étant portées par un ou plusieurs vecteurs non intégratifs.

6. Cellule selon l'une des revendications 1 à 4 **caractérisée en ce qu'**il s'agit d'une cellule hôte transformée ou transfectée par des constructions d'ADN codant pour les protéines chimères, ces constructions étant portées par un ou plusieurs vecteurs non intégràtifs, et **en ce que** la construction d'ADN d'interférence est intégrée au génome de la cellule.

7. Cellule selon l'une des revendications 1 à 4 **caractérisée en ce que** la construction d'ADN d'interférence et les constructions d'ADN codant pour les protéines chimères sont intégrées au génome de la cellule.

8. Cellule selon la revendication 6 ou 7 **caractérisée en ce que** la construction d'ADN d'interférence est intégrée à un locus exempt d'activités de transcription génomiques perturbatrices.

9. Cellule selon l'une des revendications 1 à 8 **caractérisée en ce qu'**elle est choisie dans le groupe constitué par les cellules de mammifères, d'insectes, de plantes et de levures.

10. Cellule selon l'une des revendications 1 à 9 **caractérisée en ce qu'**il s'agit de cellules de levures.

11. Cellule selon l'une des revendications 1 à 10 **caractérisée en ce qu'**il s'agit de levures de l'espèce *Saccharomyces cerevisiae, Schizosaccharomyces pombe, Kluyveromyces lactis, Pichia pastoris, Saccharomyces carlsbergensis* ou *Candida albicans*.

12. Cellule selon l'une des revendications 1 à 11 **caractérisée en ce qu'**au moins un promoteur d'interférence est positionné en aval du gène rapporteur et du premier promoteur et dans une orientation opposée à ce dernier (DI).

13. Cellule selon l'une des revendications 1 à 11 **caractérisée en ce qu'**au moins un promoteur d'interférence est positionné en aval du gène rapporteur et du premier promoteur et dans la même orientation que ce dernier (nDI).

14. Cellule selon l'une des revendications 1 à 11 **caractérisée en ce qu'**au moins un promoteur d'interférence est positionné en amont du gène rapporteur et du premier promoteur et dans la même orientation que ce dernier (UI).

15. Cellule selon l'une des revendications 1 à 11 **caractérisée en ce que** au moins un promoteur d'interférence est positionné de part et d'autre du premier promoteur et du gène rapporteur, le ou les promoteurs d'interférences situés en aval du premier promoteur et du gène rapporteur présentant une orientation convergente par rapport au premier promoteur et le ou les promoteurs d'interférence positionnés en amont du premier promoteur et du gène rapporteur présentant une orientation identique à celle du premier promoteur (UDI).

16. Cellule selon l'une des revendications 1 à 11 **caractérisée en ce que** au moins un promoteur d'interférence est positionné de part et d'autre du premier promoteur et du gène rapporteur, le ou les promoteurs d'interférences situés en aval du premier promoteur et du gène rapporteur présentant une orientation en tandem par rapport au premier promoteur et le ou les promoteurs d'interférence positionnés en amont du premier promoteur et du gène rapporteur présentant une orientation identique à celle du premier promoteur (nUDI).

17. Cellule selon l'une des revendications 1 à 16 **caractérisée en ce que** le gène rapporteur est un gène essentiel à la survie de la cellule.

18. Cellule selon la revendication 17 **caractérisée en ce que** le gène rapporteur est un gène indispensable au métabolisme primaire, à la division cellulaire, à la synthèse protéique, à la synthèse d'ADN ou la synthèse d'ARN.

19. Cellule selon l'une des revendications 1 à 16 **caractérisée en ce que** le gène rapporteur n'est pas à lui seul essentiel à la survie de la cellule, mais est essentiel à la survie de la cellule lorsque sa transcription est inhibée en association avec un ou plusieurs gènes rapporteurs du même type, dont l'expression est contrôlée ou non par le système d'interférence transcriptionnelle.

20. Cellule selon l'une des revendications 1 à 19 **caractérisée en ce que** le ou les promoteurs inductibles d'interférence comprennent une séquence susceptible d'interagir avec le domaine liant l'ADN (DBD) de la protéine chimère (X-DBD).

21. Cellule selon l'une des revendications 1 à 20 **caractérisée en ce que** le ou les promoteurs inductibles d'interférence comprennent une séquence susceptible d'interagir avec une protéine possédant un domaine de liaison à l'ADN (DBD) choisie dans le groupe constitué par : GAL4 UAS, LexAop, cIop et TetRop et que le domaine de liaison à l'ADN de la protéine chimère X-DBD est le DBD correspondant (respectivement GAL4, LexA, cI ou TetR).

22. Cellule selon l'une des revendications 1 à 21 **caractérisée en ce que** le domaine d'activation de la transcription (AD) de la protéine chimère Y-AD est choisi dans le groupe constitué par les domaines d'activation de la transcription des protéines suivant : B42, VP 16 et GAL4p.

23. Cellule selon l'une des revendications 1 à 22 **caractérisée en ce que** la construction d'ADN d'interférence est bordée à ses extrémités par un ou plusieurs terminateurs de transcription unidirectionnels ou bidirectionnels.

24. Procédé d'identification d'un composé inhibant l'interaction d'une première protéine X, avec une deuxième protéine Y comprenant les étapes suivantes :
a) cultiver des cellules selon l'une des revendications 1 à 23,
b) incuber lesdites cellules en présence du composé à tester,
c) comparer l'expression du gène rapporteur en présence et en l'absence dudit composé, une augmentation de l'expression du gène rapporteur étant l'indication que le composé à tester est un inhibiteur de l'interaction de la protéine X avec la protéine partenaire Y, exprimées par les cellules cultivées.

25. Utilisation de cellules selon l'une des revendications 1 à 23 pour l'identification de composés inhibant l'interaction protéine-protéine.

26. Utilisation de cellules selon l'une des revendications 1 à 23 pour le criblage de banques d'ADNc ou de banques de peptides afin d'identifier des peptides ou des facteurs protéiques abrogeant spécifiquement une interaction protéine-protéine.

27. Kit pour la mise en place d'un système double-hybride comprenant:
- Une première construction d'ADN comprenant :
- Un gène rapporteur placé sous le contrôle d'un premier promoteur,
- Un ou plusieurs promoteur(s) inductible(s), choisi(s) et positionné(s) de manière à ce que son (leur) activation entraîne une interférence transcriptionnelle du premier promoteur, conduisant à une diminution détectable de l'expression du gène rapporteur,
- Une deuxième construction d'ADN codant pour :
- Une première protéine chimère (Y-AD) constituée d'un domaine d'activation de la transcription (AD) fusionné à une protéine partenaire Y susceptible d'interagir avec une protéine partenaire X,
- Une troisième construction d'ADN codant pour :
- Une deuxième protéine chimère (X-DBD) constituée d'un premier domaine liant l'ADN (DBD) fusionné à un deuxième domaine constitué par une protéine X susceptible d'interagir avec la protéine Y, l'interaction des deux protéines chimères X-DBD et Y-AD aboutissant à la constitution d'un facteur de transcription fonctionnel activant le ou les promoteur d'interférence lorsque les deux protéines chimères sont exprimées dans une cellule hôte.

28. Kit selon la revendication 27 **caractérisé en ce que** le promoteur régulant l'expression du gène rapporteur est un promoteur inductible, **en ce que** la protéine Y est susceptible d'interagir avec deux protéines partenaires X et Z, et **en ce que** le dit kit comprend une quatrième construction d'ADN codant pour une troisième protéine chimère (Z-DBD) constituée d'un domaine liant l'ADN (DBD), fusionné à un deuxième domaine constitué par une protéine Z susceptible d'interagir avec la protéine Y, l'interaction des deux protéines chimères Z-DBD et Y-AD aboutissant à la constitution d'un facteur de transcription fonctionnel activant l'expression du gène rapporteur.

29. Procédé d'identification d'un composé inhibant l'interaction d'une première protéine X, avec une deuxième protéine Y, mais n'inhibant pas ou moins l'interaction entre la protéine Y et une troisième protéine Z comprenant les étapes suivantes :
a) cultiver des cellules selon la revendication 2 ou 3,
b) incuber lesdites cellules en présence du composé à tester,
c) comparer l'expression du gène rapporteur en présence et en l'absence dudit composé, une augmentation de l'expression du gène rapporteur étant l'indication que le composé à tester est un inhibiteur de l'interaction de la protéine X, avec la protéine partenaire Y, mais que ce produit n'inhibe pas ou moins l'interaction entre la protéine Y et la protéine Z.

## Claims

1. Cell containing an interference DNA construct, said construct comprising:
- A reporter gene placed under the control of a first promoter,
- One or more inducible promoter(s), so-called interference promoter(s), chosen and positioned so that its (their) activation involves a transcriptional interference of the first promoter, leading to a detectable decrease in the expression of the reporter gene,
said cell additionally expressing:
- A first chimeric protein (Y-AD) formed of a transcription activation domain (AD) fused to a protein Y capable of interacting with a partner protein X,
- A second chimeric protein (X-DBD) formed of a first DNA-binding domain (DBD) fused to a second domain formed by a protein X capable of interacting with the protein Y, the interaction of the two chimeric proteins X-DBD and Y-AD leading to the formation of a functional transcription factor activating the interference promoter(s).

2. Cell according to Claim 1, **characterized in that** the promoter regulating the expression of the reporter gene is an inducible promoter, the protein Y is capable of interacting with two partner proteins X and Z, and **in that** the cell expresses a third chimeric protein (Z-DBD) formed of a DNA-binding domain (DBD), fused to a second domain formed by a protein Z capable of interacting with the protein Y, the interaction of the two chimeric proteins Z-DBD and Y-AD leading to the formation of a functional transcription factor activating the expression of the reporter gene.

3. Cell according to Claim 2, **characterized in that** the inducible promoter regulating the expression of the reporter gene comprises a sequence capable of interacting with the DNA-binding domain (DBD) of the chimeric protein (Z-DBD).

4. Cell according to Claim 1, **characterized in that** the promoter regulating the expression of the reporter gene is a constitutive promoter.

5. Cell according to one of Claims 1 to 4, **characterized in that** it is a host cell transformed or transfected by the interference DNA construct and by DNA constructs coding for the chimeric proteins, the whole of these constructs being carried by one or more nonintegrative vectors.

6. Cell according to one of Claims 1 to 4, **characterized in that** it is a host cell transformed or transfected by DNA constructs coding for the chimeric proteins, these constructs being carried by one or more nonintegrative vectors, and **in that** the interference DNA construct is integrated into the genome of the cell.

7. Cell according to one of Claims 1 to 4, **characterized in that** the interference DNA construct and the DNA constructs coding for the chimeric proteins are integrated into the genome of the cell.

8. Cell according to Claim 6 or 7, **characterized in that** the interference DNA construct is integrated into a locus free of perturbatory genomic transcription activities.

9. Cell according to one of Claims 1 to 8, **characterized in that** it is chosen from the group formed by the cells of mammals, of insects, of plants and of yeasts.

10. Cell according to one of Claims 1 to 9, **characterized in that** yeast cells are concerned.

11. Cell according to one of Claims 1 to 10, **characterized in that** yeasts of the species *Saccharomyces cerevisiae, Schizosaccharomyces pombe, Kluyveromyces lactis, Pichia pastoris, Saccharomyces carlsbergensis* or *Candida albicans* are concerned.

12. Cell according to one of Claims 1 to 11, **characterized in that** at least one interference promoter is positioned downstream of the reporter gene and of the first promoter and in an orientation opposite to the latter (DI).

13. Cell according to one of Claims 1 to 11, **characterized in that** at least one interference promoter is positioned downstream of the reporter gene and of the first promoter and in the same orientation as the latter (nDI).

14. Cell according to one of Claims 1 to 11, **characterized in that** at least one interference promoter is positioned upstream of the reporter gene and of the first promoter and in the same orientation as the latter (UI).

15. Cell according to one of Claims 1 to 11, **characterized in that** at least one interference promoter is positioned on both sides of the first promoter and of the reporter gene, the interference promoter(s) situated downstream of the first promoter and of the reporter gene having a convergent orientation with respect to the first promoter and the interference promoter(s) positioned upstream of the first promoter and of the reporter gene having an orientation identical to that of the first promoter (UDI).

16. Cell according to one of Claims 1 to 11 **characterized in that** at least one interference promoter is positioned on both sides of the first promoter and of the reporter gene, the interference promoter(s) situated downstream of the first promoter and of the reporter gene having a paired orientation with respect to the first promoter and the interference promoter(s) positioned upstream of the first promoter and of the reporter gene having an orientation identical to that of the first promoter (nUDI).

17. Cell according to one of Claims 1 to 16, **characterized in that** the reporter gene is a gene essential to the survival of the cell.

18. Cell according to Claim 17, **characterized in that** the reporter gene is a gene indispensable to the primary metabolism, to cell division, to protein synthesis, to DNA synthesis or RNA synthesis.

19. Cell according to one of Claims 1 to 16, **characterized in that** the reporter gene is not in itself alone essential to the survival of the cell, but is essential to the survival of the cell when its transcription is inhibited in association with one or more reporter genes of the same type, the expression of which is or is not controlled by the transcriptional interference system.

20. Cell according to one of Claims 1 to 19, **characterized in that** the inducible interference promoter(s) comprise a sequence capable of interacting with the DNA-binding domain (DBD) of the chimeric protein (X-DBD).

21. Cell according to one of Claims 1 to 20, **characterized in that** the inducible interference promoter(s) comprise a sequence capable of interacting with a protein having a DNA-binding domain (DBD) chosen from the group formed by: GAL4 UAS, LexAop, cIop and TetRop and that the DNA-binding domain of the chimeric protein X-DBD is the corresponding DBD (respectively GAL4, LexA, cI or TetR).

22. Cell according to one of Claims 1 to 21, **characterized in that** the transcription activation domain (AD) of the chimeric protein Y-AD is chosen from the following group formed by the transcription activation domains of proteins: B42, VP16 and GAL4p.

23. Cell according to one of Claims 1 to 22, **characterized in that** the interference DNA construct is bordered at its ends by one or more unidirectional or bidirectional transcription terminators.

24. Process for identification of a compound inhibiting the interaction of a first protein X with a second protein Y, comprising the following steps:
a) culture cells according to one of Claims 1 to 23,
b) incubate said cells in the presence of the compound to be tested,
c) compare the expression of the reporter gene in the presence and in the absence of said compound, an increase in the expression of the reporter gene being the indication that the compound to be tested is an inhibitor of the interaction of the protein X with the partner protein Y expressed by the cultured cells.

25. Use of cells according to one of Claims 1 to 23 for the identification of compounds inhibiting protein-protein interaction.

26. Use of cells according to one of Claims 1 to 23 for the screening of cDNA banks or of banks of peptides in order to identify peptides or protein factors specifically abrogating a protein-protein interaction.

27. Kit for the setting up of a double-hybrid system comprising:
- A first DNA construct comprising:
- A reporter gene placed under the control of a first promoter,
- One or more inducible promoter(s), chosen and positioned so that its (their) activation involves a transcriptional interference of the first promoter, leading to a detectable decrease in the expression of the reporter gene,
- A second DNA construct coding for:
- A first chimeric protein (Y-AD) formed of a transcription activation domain (AD) fused to a partner protein Y capable of interacting with a partner protein X,
- A third DNA construct coding for:
- A second chimeric protein (X-DBD) formed of a first DNA-binding domain (DBD) fused to a second domain formed by a protein X capable of interacting with the protein Y, the interaction of the two chimeric proteins X-DBD and Y-AD leading to the formation of a functional transcription factor activating the interference promoter(s) when the two chimeric proteins are expressed in a host cell.

28. Kit according to Claim 27, **characterized in that** the promoter regulating the expression of the reporter gene is an inducible promoter, **in that** the protein Y is capable of interacting with two partner proteins X and Z, and **in that** said kit comprises a fourth DNA construct coding for a third chimeric protein (Z-DBD) formed of a DNA-binding domain (DBD), fused to a second domain formed by a protein Z capable of interacting with the protein Y, the interaction of the two chimeric proteins Z-DBD and Y-AD leading to the formation of a functional transcription factor activating the expression of the reporter gene.

29. Process for identification of a compound inhibiting the interaction of a first protein X with a second protein Y, but not inhibiting or inhibiting less the interaction between the protein Y and a third protein Z, comprising the following steps:
a) culture cells according to Claim 2 or 3,
b) incubate said cells in the presence of the compound to be tested,
c) compare the expression of the reporter gene in the presence and in the absence of said compound, an increase in the expression of the reporter gene being the indication that the compound to be tested is an inhibitor of the interaction of the protein X with the partner protein Y, but that this product does not inhibit or inhibits less the interaction between the protein Y and the protein Z.

## Patentansprüche

1. Zelle, die ein Interferenz-DNA-Konstrukt enthält, wobei das Konstrukt Folgendes umfasst:
- ein Reportergen, das unter die Kontrolle eines ersten Promotors gestellt ist,
- einen oder mehrere induzierbare Promotor(en), der/die als Interferenz-Promotor bezeichnet wird/werden und derart ausgewählt und positioniert ist/sind, dass seine (ihre) Aktivierung eine Interferenz der Transkription von dem ersten Promotor bewirkt, die zu einer nachweisbaren Verringerung der Expression des Reportergens führt,
wobei die Zelle außerdem Folgendes exprimiert:
- ein erstes chimäres Protein (Y-AD), bestehend aus einer Transkriptionsaktivierungsdomäne (AD), fusioniert an ein Protein Y, das mit einem Partnerprotein X wechselwirken kann,
- ein zweites chimäres Protein (X-DBD), bestehend aus einer ersten, DNA-bindenden Domäne (DBD), fusioniert an eine zweite Domäne, die aus einem Protein X besteht, das mit dem Protein Y wechselwirken kann, wobei die Wechselwirkung der beiden chimären Proteine X-DBD und Y-AD zur Bildung eines funktionellen Transkriptionsfaktors führt, der den oder die Interferenz-Promotor(en) aktiviert.

2. Zellen nach Anspruch 1, **dadurch gekennzeichnet, dass** der Promotor, der die Expression des Reportergens reguliert, ein induzierbarer Promotor ist, das Protein Y mit zwei Partnerproteinen X und Z wechselwirken kann, und **dadurch**, dass die Zelle ein drittes chimäres Protein (Z-DBD) exprimiert, bestehend aus einer DNA-bindenden Domäne (DBD), fusioniert an eine zweite Domäne, die aus einem Protein Z besteht, das mit dem Protein Y wechselwirken kann, wobei die Wechselwirkung der beiden chimären Proteine Z-DBD und Y-AD zur Bildung eines funktionellen Transkriptionsfaktors führt, der die Expression des Reportergens aktiviert.

3. Zelle nach Anspruch 2, **dadurch gekennzeichnet, dass** der induzierbare Promotor, der die Expression des Reportergens reguliert, eine Sequenz umfasst, die mit der DNA-bindenden Domäne (DBD) des chimären Proteins (Z-DBD) wechselwirken kann.

4. Zelle nach Anspruch 1, **dadurch gekennzeichnet, dass** der Promotor, der die Expression des Reportergens reguliert, ein konstitutiver Promotor ist.

5. Zelle nach einem der Ansprüche 1 bis 4, **dadurch gekennzeichnet, dass** es sich um eine Wirtszelle handelt, die mit dem Interferenz-DNA-Konstrukt und mit den DNA-Konstrukten, die für die chimären Proteine codieren, transformiert oder transfiziert ist, wobei sich diese sämtlichen Konstrukte auf einem oder mehreren nicht-integrierenden Vektor(en) befinden.

6. Zelle nach einem der Ansprüche 1 bis 4, **dadurch gekennzeichnet, dass** es sich um eine Wirtszelle handelt, die mit den DNA-Konstrukten, die für die chimären Proteine codieren, transformiert oder transfiziert ist, wobei sich diese Konstrukte auf einem oder mehreren nicht-integrierenden Vektor(en) befinden, und **dadurch**, dass das Interferenz-DNA-Konstrukt in das Genom der Zelle integriert worden ist.

7. Zelle nach einem der Ansprüche 1 bis 4, **dadurch gekennzeichnet, dass** das Interferenz-DNA-Konstrukt und die DNA-Konstrukte, die für die chimären Proteine codieren, in das Genom der Zelle integriert worden sind.

8. Zelle nach Anspruch 6 oder 7, **dadurch gekennzeichnet, dass** das Interferenz-DNA-Konstrukt an einem Locus integriert worden ist, der keine störenden genomischen Transkriptionsaktivitäten besitzt.

9. Zelle nach einem der Ansprüche 1 bis 8, **dadurch gekennzeichnet, dass** sie aus der Gruppe ausgewählt ist, die aus Säuger-, Insekten-, Pflanzen- und Hefezellen besteht.

10. Zelle nach einem der Ansprüche 1 bis 9, **dadurch gekennzeichnet, dass** es sich um Hefezellen handelt.

11. Zelle nach einem der Ansprüche 1 bis 10, **dadurch gekennzeichnet, dass** es sich um Hefen der Spezies *Saccharomyces cerevisiae, Schizosaccharomyces pombe, Kluyveromyces lactis, Pichia pastoris, Saccharomyces carlsbergensis* oder *Candida albicans* handelt.

12. Zelle nach einem der Ansprüche 1 bis 11, **dadurch gekennzeichnet, dass** mindestens ein Interferenz-Promotor stromabwärts des Reportergens und des ersten Promotors und in einer zu Letzterem entgegengesetzten Orientierung (DI) positioniert ist.

13. Zelle nach einem der Ansprüche 1 bis 11, **dadurch gekennzeichnet, dass** mindestens ein Interferenz-Promotor stromabwärts des Reportergens und des ersten Promotors und in derselben Orientierung wie Letzterer (nDI) positioniert ist.

14. Zelle nach einem der Ansprüche 1 bis 11, **dadurch gekennzeichnet, dass** mindestens ein Interferenz-Promotor stromaufwärts des Reportergens und des ersten Promotors und in derselben Orientierung wie Letzterer (UI) positioniert ist.

15. Zelle nach einem der Ansprüche 1 bis 11, **dadurch gekennzeichnet, dass** mindestens ein Interferenz-Promotor auf beiden Seiten des ersten Promotors und des Reportergens positioniert ist, wobei der oder die stromabwärts des ersten Promotors und des Reportergens befindliche(n) Interferenz-Promotor(en) eine in Bezug auf den ersten Promotor konvergente Orientierung aufweis(t/en) und der oder die stromaufwärts des ersten Promotors und des Reportergens befindliche(n) Interferenz-Promotor(en) eine zu derjenigen des ersten Promotors identische Orientierung aufweis(t/en) (UDI).

16. Zelle nach einem der Ansprüche 1 bis 11, **dadurch gekennzeichnet, dass** mindestens ein Interferenz-Promotor auf beiden Seiten des ersten Promotors und des Reportergens positioniert ist, wobei der oder die stromabwärts des ersten Promotors und des Reportergens befindliche(n) Interferenz-Promotor(en) eine Tandem-Orientierung in Bezug auf den ersten Promotor aufweis(t/en) und der oder die stromaufwärts des ersten Promotors und des Reportergens befindliche(n) Interferenz-Promotor(en) eine zu derjenigen des ersten Promotors identische Orientierung aufweis(t/en) (nUDI).

17. Zelle nach einem der Ansprüche 1 bis 16, **dadurch gekennzeichnet, dass** das Reportergen ein für das Überleben der Zelle essenzielles Gen ist.

18. Zelle nach Anspruch 17, **dadurch gekennzeichnet, dass** das Reportergen ein Gen ist, das für den Primärstoffwechsel, die Zellteilung, die Proteinsynthese, die DNA-Synthese oder die RNA-Synthese unverzichtbar ist.

19. Zelle nach einem der Ansprüche 1 bis 16, **dadurch gekennzeichnet, dass** das Reportergen selbst nicht allein für das Überleben der Zelle essenziell ist, sondern für das Überleben der Zelle essenziell ist, wenn seine Transkription gehemmt wird, in Verbindung mit einem oder mehreren Reporter-genen desselben Typs, deren Expression durch das Transkriptions-Interferenz-System gesteuert wird oder nicht.

20. Zelle nach einem der Ansprüche 1 bis 19, **dadurch gekennzeichnet, dass** der oder die induzierbare(n) Interferenz-Promotor(en) eine Sequenz umfass(t/en), die mit der DNA-bindenden Domäne (DBD) des chimären Proteins (X-DBD) wechselwirken kann.

21. Zelle nach einem der Ansprüche 1 bis 20, **dadurch gekennzeichnet, dass** der oder die induzierbare(n) Interferenz-Promotor(en) eine Sequenz umfass(t/en), die mit einem Protein wechselwirken kann, das eine DNA-bindende Domäne (DBD) besitzt und aus der Gruppe, bestehend aus: GAL4-UAS, LexAop, cIop und TetRop, ausgewählt ist, und dass die DNA-bindende Domäne des chimären Proteins X-DBD die entsprechende DBD (GAL4, LexA, cI beziehungsweise TetR) ist.

22. Zelle nach einem der Ansprüche 1 bis 21, **dadurch gekennzeichnet, dass** die Transkriptionsaktivierungsdomäne (AD) des chimären Proteins Y-AD aus der Gruppe ausgewählt ist, die aus den Transkriptionsaktivierungsdomänen der folgenden Proteine besteht: B42, VP16 und GAL4p.

23. Zelle nach einem der Ansprüche 1 bis 22, **dadurch gekennzeichnet, dass** das Interferenz-DNA-Konstrukt an seinen Enden von einem oder mehreren uni- oder bidirektionalen Transkriptionsterminatoren flankiert ist.

24. Verfahren zur Identifikation einer Verbindung, welche die Wechselwirkung zwischen einem ersten Protein X und einem zweiten Protein Y hemmt, das folgende Schritte umfasst:
a) Züchten der Zellen nach einem der Ansprüche 1 bis 23,
b) Inkubieren dieser Zellen in Gegenwart einer zu testenden Verbindung,
c) Vergleichen der Expression des Reportergens in Gegenwart und in Abwesenheit der Verbindung, wobei eine Erhöhung der Expression des Reportergens ein Anzeichen dafür ist, dass die zu testende Verbindung ein Inhibitor der Wechselwirkung zwischen dem Protein X und dem Partnerprotein Y ist, die durch die gezüchteten Zellen exprimiert werden.

25. Verwendung von Zellen nach einem der Ansprüche 1 bis 23 zur Identifikation von Verbindungen, welche die Protein-Protein-Wechselwirkung hemmen.

26. Verwendung von Zellen nach einem der Ansprüche 1 bis 23 für das Screening von cDNA-Banken oder Peptidbanken, um Peptide oder Proteinfaktoren zu identifizieren, die spezifisch eine Protein-Protein-Wechselwirkung unterbinden.

27. Kit zur Einrichtung eines Doppel-Hybrid-Systems, umfassend:
- ein erstes DNA-Konstrukt, umfassend:
- ein Reportergen, das unter die Kontrolle eines ersten Promotors gestellt ist,
- einen oder mehrere induzierbare Promotor(en), der/die derart ausgewählt und positioniert ist/sind, dass seine (ihre) Aktivierung eine Interferenz der Transkription von dem ersten Promotor bewirkt, die zu einer nachweisbaren Verringerung der Expression des Reportergens führt,
- ein zweites DNA-Konstrukt, das für Folgendes codiert:
- ein erstes chimäres Protein (Y-AD), bestehend aus einer Transkriptionsaktivierungsdomäne (AD), fusioniert an ein Partnerprotein Y, das mit einem Partnerprotein X wechselwirken kann,
- ein drittes DNA-Konstrukt, das für Folgendes codiert:
- ein zweites chimäres Protein (X-DBD), bestehend aus einer ersten, DNA-bindenden Domäne (DBD), fusioniert an eine zweite Domäne, die aus einem Protein X besteht, das mit dem Protein Y wechselwirken kann, wobei die Wechselwirkung der beiden chimären Proteine X-DBD und Y-AD zur Bildung eines funktionellen Transkriptionsfaktors führt, der den oder die Interferenz-Promotor(en) aktiviert, wenn die beiden chimären Proteine in einer Wirtszelle exprimiert werden.

28. Kit nach Anspruch 27, **dadurch gekennzeichnet, dass** der Promotor, der die Expression des Reportergens reguliert, ein induzierbarer Promotor ist, **dadurch**, dass das Protein Y mit zwei Partnerproteinen X und Z wechselwirken kann, und **dadurch**, dass das Kit ein viertes DNA-Konstrukt umfasst, das für ein drittes chimäres Protein (Z-DBD) codiert, bestehend aus einer DNA-bindenden Domäne (DBD), fusioniert an eine zweite Domäne, die aus einem Protein Z besteht, das mit dem Protein Y wechselwirken kann, wobei die Wechselwirkung der beiden chimären Proteine Z-DBD und Y-AD zur Bildung eines funktionellen Transkriptionsfaktors führt, der die Expression des Reportergens aktiviert.

29. Verfahren zur Identifikation einer Verbindung, welche die Wechselwirkung zwischen einem ersten Protein X und einem zweiten Protein Y hemmt, welche aber die Wechselwirkung zwischen dem Protein Y und einem dritten Protein Z nicht oder weniger hemmt, das folgende Schritte umfasst:
a) Züchten der Zellen nach Anspruch 2 oder 3,
b) Inkubieren dieser Zellen in Gegenwart der zu testenden Verbindung,
c) Vergleichen der Expression des Reportergens in Gegenwart und in Abwesenheit der Verbindung, wobei eine Erhöhung der Expression des Reportergens ein Anzeichen dafür ist, dass die zu testende Verbindung ein Inhibitor der Wechselwirkung zwischen dem Protein X und dem Partnerprotein Y ist, aber dass dieses Produkt die Wechselwirkung zwischen dem Protein Y und dem Protein Z nicht oder weniger hemmt.


<110> AVENTIS PHARMA SA.
<120> Système double hybride basé sur la mise sous silence de gène par interférence transcriptionnelle
<130> IDM04003
<160> 38
<170> PatentIn version 3.1
<210> 1
   <211> 37
   <212> DNA
   <213> Artificial Sequence
<220>
   <223> oligonucleotide RB43
<400> 1
   aaaatctaga ggcgccatat ccttttgttg tttccgg 37
<210> 2
   <211> 35
   <212> DNA
   <213> Artificial Sequence
<220>
   <223> Oligonucleotide RB44
<400> 2
   aaaaacgcgt ggcgcccatc tttcaggagg cttgc 35
<210> 3
   <211> 27
   <212> DNA
   <213> Artificial Sequence
<220>
   <223> Oligonucleotide RB75
<400> 3
   cgcgtggggg gttaattaaa aaaaagc 27
<210> 4
   <211> 27
   <212> DNA
   <213> Artificial Sequence
<220>
   <223> oligonucleotide RB76
<400> 4
   ggccgctttt ttttaattaa cccccca 27
<210> 5
   <211> 29
   <212> DNA
   <213> Artificial Sequence
<220>
   <223> Oligonucleotide RB45
<400> 5
   aaaaacgcgt acagagcaga aagccctag 29
<210> 6
   <211> 52
   <212> DNA
   <213> Artificial Sequence
<220>
   <223> Oligonucleotide RB46
<400> 6
   aaaaaagcgg ccgcggcgcg ccttaattaa ctacataaga acacctttgg tg 52
<210> 7
   <211> 31
   <212> DNA
   <213> Artificial Séquence
<220>
   <223> oligonucleotide RB51
<400> 7
   aaaaggcgcg cctaattccg ggcgaatttc t 31
<210> 8
   <211> 28
   <212> DNA
   <213> Artificial sequence
<220>
   <223> Oligonucleotide RB52
<400> 8
   aaaagagctc tgcatgccgg tagaggtg 28
<210> 9
   <211> 93
   <212> DNA
   <213> Artificial sequence
<220>
   <223> oligonucleotide RB98
<400> 9
   cgatcgcgtt tgtacagaaa aaaaagaaaa atttgaaata taaataacgt tcttaatact 60
   aacataacta ttaaaaaaaa taaataggga ccg 93
<210> 10
   <211> 95
   <212> DNA
   <213> Artificial sequence
<220>
   <223> oligonucleotide RB99
<400> 10
   aattcggtcc ctatttattt tttttaatag ttatgttagt attaagaacg ttatttatat 60
   ttcaaatttt tctttttttt ctgtacaaac gcgat 95
<210> 11
   <211> 34
   <212> DNA
   <213> Artificial Sequence
<220>
   <223> Oligonucleotide RB49
<400> 11
   aaaaggcgcg ccgtaaagag ccccattatc ttag 34
<210> 12
   <211> 33
   <212> DNA
   <213> Artificial sequence
<220>
   <223> Oligonucleotide RB50
<400> 12
   aaaaggcgcg cctttgagat ccgggttttt tct 33
<210> 13
   <211> 94
   <212> DNA
   <213> Artificial Sequence
<220>
   <223> Oligonucleotide RB81
<400> 13
   ccggggcggc cgcaacgttt ggtggtggcg accatcctcc aaaatcggat ctggttccgc 60
   gttgagtagc tgaataagtg aataggcggc cgct 94
<210> 14
   <211> 94
   <212> DNA
   <213> Artificial Sequence
<220>
   <223> Oligonucleotide RB82
<400> 14
   ctagagcggc cgcctattca cttattcagc tactcaacgc ggaaccagat ccgattttgg 60
   aggatggtcg ccaccaccaa acgttgcggc cgcc 94
<210> 15
   <211> 32
   <212> DNA
   <213> Artificial Sequence
<220>
   <223> Oligonucleotide RB55
<400> 15
   aaaacccggg gtaaagagcc ccattatctt ag 32
<210> 16
   <211> 33
   <212> DNA
   <213> Artificial sequence
<220>
   <223> Oligonucleotide RB56
<400> 16
   aaaacccggg cattttgaga tccgggtttt ttc 33
<210> 17
   <211> 34
   <212> DNA
   <213> Artificial Sequence
<220>
   <223> Oligonucleotide RB83
<400> 17
   aaaaaagagc tctactaaac tcacaaatta gagc 34
<210> 18
   <211> 41
   <212> DNA
   <213> Artificial Sequence
<220>
   <223> Oligonucleotide RB84
<400> 18
   aaaaaagaat tcgcggccgc aaatatactg gggaaccagt c 41
<210> 19
   <211> 41
   <212> DNA
   <213> Artificial Sequence
<220>
   <223> Oligonucleotide RB85
<400> 19
   aaaaaacgta cggcggccgc gataaggaga atccatacaa g 41
<210> 20
   <211> 32
   <212> DNA
   <213> Artificial sequence
<220>
   <223> oligonucleotide RB86
<400> 20
   aaaaaacgta cgtttatgga ccctgaaacc ac 32
<210> 21
   <211> 25
   <212> DNA
   <213> Artificial Sequence
<220>
   <223> oligonucleotide RB38
<400> 21
   ggggtaatta atcagcgaag cgatg 25
<210> 22
   <211> 19
   <212> DNA
   <213> Artificial Sequence
<220>
   <223> oligonucleotide RB61
<400> 22
   cgatgtatgg gtttggttg 19
<210> 23
   <211> 19
   <212> DNA
   <213> Artificial Sequence
<220>
   <223> Oligonucleotide RB66
<400> 23
   tcttgcgaga tgatcccgc 19
<210> 24
   <211> 22
   <212> DNA
   <213> Artificial Sequence
<220>
   <223> Oligonucleotide RB68
<400> 24
   taagcgtatt actgaaagtt cc 22
<210> 25
   <211> 23
   <212> DNA
   <213> Artificial Sequence
<220>
   <223> Oligonucleotide RB103
<400> 25
   gcaatgaaag agcagagcga gag 23
<210> 26
   <211> 25
   <212> DNA
   <213> Artificial Sequence
<220>
   <223> oligonucleotide RB109
<400> 26
   gttatcagat attatcaggt gggaa 25
<210> 27
   <211> 33
   <212> DNA
   <213> Artificial Sequence
<220>
   <223> Oligonucleotide RB250
<400> 27
   agatcgaatt caaggcggcc ggggaggccg agg 33
<210> 28
   <211> 32
   <212> DNA
   <213> Artificial Sequence
<220>
   <223> Oligonucleotide RB251
<400> 28
   gcaggtcgac tcatggggta tgggccccca gc 32
<210> 29
   <211> 55
   <212> DNA
   <213> Artificial sequence
<220>
   <223> oligonucleotide RB136
<400> 29
   ttgtaaaacg acggccagtg aattccgtac gatatccttt tgttgtttcc gggtg 55
<210> 30
   <211> 24
   <212> DNA
   <213> Artificial Sequence
<220>
   <223> oligonucleotide RB137
<400> 30
   agttgattgt atgcttggta tagc 24
<210> 31
   <211> 9728
   <212> DNA
   <213> Artificial Sequence
<220>
   <223> Vecteur pRB34
<400> 31
   tcgcgcgttt cggtgatgac ggtgaaaacc tctgacacat gcagctcccg gagacggtca      60
   cagcttgtct gtaagcggat gccgggagca gacaagcccg tcagggcgcg tcagcgggtg     120
   ttggcgggtg tcggggctgg cttaactatg cggcatcaga gcagattgta ctgagagtgc     180
   accatatgcg gtgtgaaata ccgcacagat gcgtaaggag aaaataccgc atcaggcgcc     240
   attcgccatt caggctgcgc aactgttggg aagggcgatc ggtgcgggcc tcttcgctat     300
   tacgccagct ggcgaaaggg ggatgtgctg caaggcgatt aagttgggta acgccagggt     360
   tttcccagtc acgacgttgt aaaacgacgg ccagtgaatt ccgtacgata tccttttgtt     420
   gtttccgggt gtacaatatg gacttcctct tttctggcaa ccaaacccat acatcgggat     480
   tcctataata ccttcgttgg tctccctaac atgtaggtgg cggaggggag atatacaata     540
   gaacagatac cagacaagac ataatgggct aaacaagact acaccaatta cactgcctca     600
   ttgatggtgg tacataacga actaatactg tagccctaga cttgatagcc atcatcatat     660
   cgaagtttca ctaccctttt tccatttgcc atctattgaa gtaataatag gcgcatgcaa     720
   cttcttttct ttttttttct tttctctctc ccccgttgtt gtctcaccat atccgcaatg 780
   acaaaaaaat gatggaagac actaaaggaa aaaattaacg acaaagacag caccaacaga 840
   tgtcgttgtt ccagagctga tgaggggtat ctcgaagcac acgaaacttt ttccttcctt 900
   cattcacgca cactactctc taatgagcaa cggtatacgg ccttccttcc agttacttga 960
   atttgaaata aaaaaaagtt tgctgtcttg ctatcaagta taaatagacc tgcaattatt 1020
   aatcttttgt ttcctcgtca ttgttctcgt tccctttctt ccttgtttct ttttctgcac 1080
   aatatttcaa gctataccaa gcatacaatc aactccaagc tttgcaaaga tggataaagc 1140
   ggaattaatt cccgagcctc caaaaaagaa gagaaaggtc gaattgggta ccgccgccaa 1200
   ttttaatcaa agtgggaata ttgctgatag ctcattgtcc ttcactttca ctaacagtag 1260
   caacggtccg aacctcataa caactcaaac aaattctcaa gcgctttcac aaccaattgc 1320
   ctcctctaac gttcatgata acttcatgaa taatgaaatc acggctagta aaattgatga 1380
   tggtaataat tcaaaaccac tgtcacctgg ttggacggac caaactgcgt ataacgcgtt 1440
   tggaatcact acagggatgt ttaataccac tacaatggat gatgtatata actatctatt 1500
   cgatgatgaa gataccccac caaacccaaa aaaagagatc gaattcaagg cggccgggga 1560
   ggccgaggag gaggaggagg atgatgatga tgaagaggag gaggaggact tatcttctcc 1620
   cccagggctg cctgagcccc tggagagtgt ggaggcccct cccaggcccc aagcccttac 1680
   agatggcccc cgggaacaca gcaagagtgc cagcctcctg tttggcatgc ggaacagtgc 1740
   agccagtgat gaggactcaa gctgggctac cttatcccag ggcagcccct cctatggctc 1800
   cccagaggac acagattcct tctggaaccc caacgccttc gagacggatt ccgacctgcc 1860
   ggctggatgg atgagggtcc aggacacctc agggacctat tactggcaca tcccaacagg 1920
   gaccacccag tgggaacccc ccggccgggc ctccccctca caggggagca gcccccaaga 1980
   ggagtcccag ctcacctgga caggttttgc tcatggagaa ggctttgagg atggagaatt 2040
   ttggaaggat gaacccagtg atgaggcccc aatggagctg ggactgaagg aacctgagga 2100
   ggggacgttg accttcccag ctcagagcct cagcccagag ccgttgcccc aagaggagga 2160
   gaagcttccc ccacggaata ccaacccagg gatcaagtgt ttcgccgtgc gctccctagg 2220
   ctgggtagag atgaccgagg aggagctggc ccctggacgc agcagtgtgg cagtcaacaa 2280
   ttgcatccgt cagctctctt accacaaaaa caacctgcat gaccccatgt ctgggggctg 2340
   gggggaagga aaggatctgc tactgcagct ggaggatgag acactaaagc tagtggagcc 2400
   acagagccag gcactgctgc acgcccaacc catcatcagc atccgcgtgt ggggcgtcgg 2460
   gcgggacagt ggaagggact ttgcctacgt agctcgtgat aagctgaccc agatgctcaa 2520
   gtgccacgtg tttcgctgtg aggcacctgc caagaacatc gccaccagcc tgcatgagat 2580
   ctgctctaag atcatggccg aacggcgtaa tgcccgctgc ttggtaaatg gactctccct 2640
   ggaccactct aaacttgtgg atgtcccttt ccaagtggaa ttcccagcgc ctaagaatga 2700
   gttggtccag aagttccaag tctattacct ggggaatgta cctgttgcta aacctgttgg 2760
   ggtagatgtg attaatgggg ccctcgagtc agtcctgtcc tccagcagcc gtgaacaatg 2820
   gaccccaagt catgtcagtg tggcccctgc taccctcacc atcttgcacc agcagacaga 2880
   ggcagtgctg ggagagtgtc gggtgcgttt cctctccttc ctggccgtgg gcagagatgt 2940
   ccacacgttt gcattcatca tggctgccgg cccagcctcc ttctgctgcc acatgttctg 3000
   gtgcgagccc aatgctgcca gcctctcaga ggctgtgcag gctgcgtgca tgcttcgcta 3060
   ccagaagtgt ctggatgccc gttcccaggc ctccacctcc tgcctcccag caccccctgc 3120
   tgagtctgtg gcacggcgtg tagggtggac tgtccgcagg ggtgttcagt cgctgtgggg 3180
   ctccctgaag cccaaacggc tgggggccca taccccatga gtcgacctgc agagatctat 3240
   gaatcgtaga tactgaaaaa ccccgcaagt tcacttcaac tgtgcatcgt gcaccatctc 3300
   aatttctttc atttatacat cgttttgcct tcttttatgt aactatactc ctctaagttt 3360
   caatcttggc catgtaacct ctgatctata gaatttttta aatgactaga attaatgccc 3420
   atcttttttt tggacctaaa ttcttcatga aaatatatta cgagggctta ttcagaagct 3480
   ttggacttct tcgccagagg tttggtcaag tctccaatca aggttgtcgg cttgtctacc 3540
   ttgccagaaa tttacgaaaa gatggaaaag ggtcaaatcg ttggtagata cgttgttgac 3600
   acttctaaat aagcgaattt cttatgattt atgattttta ttattaaata agttataaaa 3660
   aaaataagtg tatacaaatt ttaaagtgac tcttaggttt taaaacgaaa attcttgttc 3720
   ttgagtaact ctttcctgta ggtcaggttg ctttctcagg tatagcatga ggtcgctctt 3780
   attgaccaca cctctaccgg catgcaagct tggcgtaatc atggtcatag ctgtttcctg 3840
   tgtgaaattg ttatccgctc acaattccac acaacatacg agccggaagc ataaagtgta 3900
   aagcctgggg tgcctaatga gtgagctaac tcacattaat tgcgttgcgc tcactgcccg 3960
   ctttccagtc gggaaacctg tcgtgccagc agatctatta cattatgggt ggtatgttgg 4020
   aataaaaatc aactatcatc tactaactag tatttacgtt actagtatat tatcatatac 4080
   ggtgttagaa gatgacgcaa atgatgagaa atagtcatct aaattagtgg aagctgaaac 4140
   gcaaggattg ataatgtaat aggatcaatg aatattaaca tataaaatga tgataataat 4200
   atttatagaa ttgtgtagaa ttgcagattc ccttttatgg attcctaaat cctcgaggag 4260
   aacttctagt atatctacat acctaatatt attgccttat taaaaatgga atcccaacaa 4320
   ttacatcaaa atccacattc tcttcaaaat caattgtcct gtacttcctt gttcatgtgt 4380
   gttcaaaaac gttatattta taggataatt atactctatt tctcaacaag taattggttg 4440
   tttggccgag cggtctaagg cgcctgattc aagaaatatc ttgaccgcag ttaactgtgg 4500
   gaatactcag gtatcgtaag atgcaagagt tcgaatctct tagcaaccat tatttttttc 4560
   ctcaacataa cgagaacaca caggggcgct atcgcacaga atcaaattcg atgactggaa 4620
   attttttgtt aatttcagag gtcgcctgac gcatatacct ttttcaactg aaaaattggg 4680
   agaaaaagga aaggtgagag ccgcggaacc ggcttttcat atagaataga gaagcgttca 4740
   tgactaaatg cttgcatcac aatacttgaa gttgacaata ttatttaagg acctattgtt 4800
   ttttccaata ggtggttagc aatcgtctta ctttctaact tttcttacct tttacatttc 4860
   agcaatatat atatatatat ttcaaggata taccattcta atgtctgccc ctaagaagat 4920
   cgtcgttttg ccaggtgacc acgttggtca agaaatcaca gccgaagcca ttaaggttct 4980
   taaagctatt tctgatgttc gttccaatgt caagttcgat ttcgaaaatc atttaattgg 5040
   tggtgctgct atcgatgcta caggtgtccc acttccagat gaggcgctgg aagcctccaa 5100
   gaaggttgat gccgttttgt taggtgctgt gggtggtcct aaatggggta ccggtagtgt 5160
   tagacctgaa caaggtttac taaaaatccg taaagaactt caattgtacg ccaacttaag 5220
   accatgtaac tttgcatccg actctctttt agacttatct ccaatcaagc cacaatttgc 5280
   taaaggtact gacttcgttg ttgtcagaga attagtggga ggtatttact ttggtaagag 5340
   aaaggaagac gatggtgatg gtgtcgcttg ggatagtgaa caatacaccg ttccagaagt 5400
   gcaaagaatc acaagaatgg ccgctttcat ggccctacaa catgagccac cattgcctat 5460
   ttggtccttg gataaagcta atgttttggc ctcttcaaga ttatggagaa aaactgtgga 5520
   ggaaaccatc aagaacgaat tccctacatt gaaggttcaa catcaattga ttgattctgc 5580
   cgccatgatc ctagttaaga acccaaccca cctaaatggt attataatca ccagcaacat 5640
   gtttggtgat atcatctccg atgaagcctc cgttatccca ggttccttgg gtttgttgcc 5700
   atctgcgtcc ttggcctctt tgccagacaa gaacaccgca tttggtttgt acgaaccatg 5760
   ccacggttct gctccagatt tgccaaagaa taaggtcaac cctatcgcca ctatcttgtc 5820
   tgctgcaatg atgttgaaat tgtcattgaa cttgcctgaa gaaggtaagg ccattgaaga 5880
   tgcagttaaa aaggttttgg atgcaggtat cagaactggt gatttaggtg gttccaacag 5940
   taccacggaa gtcggtgatg ctgtcgccga agaagttaag aaaatccttg cttaaaaaga 6000
   ttctcttttt ttatgatatt tgtacataaa ctttataaat gaaattcata atagaaacga 6060
   cacgaaatta caaaatggaa tatgttcata gggtagacga aactatatac gcaatctaca 6120
   tacatttatc aagaaggaga aaaaggagga tgtaaaggaa tacaggtaag caaattgata 6180
   ctaatggctc aacgtgataa ggaaaaagaa ttgcacttta acattaatat tgacaaggag 6240
   gagggcacca cacaaaaagt taggtgtaac agaaaatcat gaaactatga ttcctaattt 6300
   atatattgga ggattttctc taaaaaaaaa aaaatacaac aaataaaaaa cactcaatga 6360
   cctgaccatt tgatggagtt taagtcaata ccttcttgaa ccatttccca taatggtgaa 6420
   agttccctca agaattttac tctgtcagaa acggccttaa cgacgtagtc gacctcctct 6480
   tcagtactaa atctaccaat accaaatctg atggaagaat gggctaatgc atcatcctta 6540
   cccagcgcat gtaaaacata agaaggttct agggaagcag atgtacaggc tgaacccgag 6600
   gataatgcga tatcccttag tgccatcaat aaagattctc cttccacgta ggcgaaagaa 6660
   acgttaacac accctggata acgatgatct ggagatccgt tcaacgtggt atgttcagcg 6720
   gataatagac ctttgactaa tttatcggat agtcttttga tgtgagcttg gtcgttgtca 6780
   aattctttct tcatcaatct cgcagcttca ccaaatcccg ctaccaatgg gggggccaaa 6840
   gtaccagatc tgctgcatta atgaatcggc caacgcgcgg ggagaggcgg tttgcgtatt 6900
   gggcgctctt ccgcttcctc gctcactgac tcgctgcgct cggtcgttcg gctgcggcga 6960
   gcggtatcag catcgatgaa tggaaattac gtactttcaa cgcaagagca agacaaaaga 7020
   aagcctaaaa ctgtcttttc gtgtcggtcg tccaatatca tcgtaaacgt gtgtagagcc 7080
   ttaaattcta atttctaact ttaaattttt tttgctatta taatactaat ttctaacttt 7140
   caacctattt tacatcttcg gaaaacaaaa actatttttt ctttaatttc tttttttact 7200
   ttctattttt aatttatata tttatattaa aaaatttaaa ttatattatt tttatagcac 7260
   gtgatgaaaa gaaatatagt cttcaaggtt atattataca ggtttatata ttattttact 7320
   tatttttgtt gttctttttc ttggagtaat ttagcattaa caacttcgac ccatcgcgat 7380
   gacgaaattg cacgtttaaa ggagaagtcg gaatcgttgc aagaagaaat aaattagttt 7440
   gacgttttat tccttttctt ctgcacagaa gtgtgtatat atatatttat atatagtcga 7500
   tgtgcaaaag cctacattat ttttcttttg atttttattt ttttgttcaa gtaggttcat 7560
   gccgacagta aatccttatg ccctacctaa atataaacac ccaaatatat atatatatat 7620
   atatatatat aaataataaa aacggaaagg aaatagaata ataataataa tactacacgt 7680
   ttacgtatgt aactcattct attattcaga ctttgatatt aatagaatta ttactactag 7740
   gtttcctcac ttcaaaatct ataaatatac cgtcatgctc taatcgatgc tcactcaaag 7800
   gtcggtaata cggttatcca cagaatcagg ggataacgca ggaaagaaca tgtgagcaaa 7860
   aggccagcaa aaggccagga accgtaaaaa ggccgcgttg ctggcgtttt tccataggct 7920
   ccgcccccct gacgagcatc acaaaaatcg acgctcaagt cagaggtggc gaaacccgac 7980
   aggactataa agataccagg cgtttccccc tggaagctcc ctcgtgcgct ctcctgttcc 8040
   gaccctgccg cttaccggat acctgtccgc ctttctccct tcgggaagcg tggcgctttc 8100
   tcatagctca cgctgtaggt atctcagttc ggtgtaggtc gttcgctcca agctgggctg 8160
   tgtgcacgaa ccccccgttc agcccgaccg ctgcgcctta tccggtaact atcgtcttga 8220
   gtccaacccg gtaagacacg acttatcgcc actggcagca gccactggta acaggattag 8280
   cagagcgagg tatgtaggcg gtgctacaga gttcttgaag tggtggccta actacggcta 8340
   cactagaagg acagtatttg gtatctgcgc tctgctgaag ccagttacct tcggaaaaag 8400
   agttggtagc tcttgatccg gcaaacaaac caccgctggt agcggtggtt tttttgtttg 8460
   caagcagcag attacgcgca gaaaaaaagg atctcaagaa gatcctttga tcttttctac 8520
   ggggtctgac gctcagtgga acgaaaactc acgttaaggg attttggtca tgagattatc 8580
   aaaaaggatc ttcacctaga tccttttaaa ttaaaaatga agttttaaat caatctaaag 8640
   tatatatgag taaacttggt ctgacagtta ccaatgctta atcagtgagg cacctatctc 8700
   agcgatctgt ctatttcgtt catccatagt tgcctgactc cccgtcgtgt agataactac 8760
   gatacgggag ggcttaccat ctggccccag tgctgcaatg ataccgcgag acccacgctc 8820
   accggctcca gatttatcag caataaacca gccagccgga agggccgagc gcagaagtgg 8880
   tcctgcaact ttatccgcct ccatccagtc tattaattgt tgccgggaag ctagagtaag 8940
   tagttcgcca gttaatagtt tgcgcaacgt tgttgccatt gctacaggca tcgtggtgtc 9000
   acgctcgtcg tttggtatgg cttcattcag ctccggttcc caacgatcaa ggcgagttac 9060
   atgatccccc atgttgtgca aaaaagcggt tagctccttc ggtcctccga tcgttgtcag 9120
   aagtaagttg gccgcagtgt tatcactcat ggttatggca gcactgcata attctcttac 9180
   tgtcatgcca tccgtaagat gcttttctgt gactggtgag tactcaacca agtcattctg 9240
   agaatagtgt atgcggcgac cgagttgctc ttgcccggcg tcaatacggg ataataccgc 9300
   gccacatagc agaactttaa aagtgctcat cattggaaaa cgttcttcgg ggcgaaaact 9360
   ctcaaggatc ttaccgctgt tgagatccag ttcgatgtaa cccactcgtg cacccaactg 9420
   atcttcagca tcttttactt tcaccagcgt ttctgggtga gcaaaaacag gaaggcaaaa 9480
   tgccgcaaaa aagggaataa gggcgacacg gaaatgttga atactcatac tcttcctttt 9540
   tcaatattat tgaagcattt atcagggtta ttgtctcatg agcggataca tatttgaatg 9600
   tatttagaaa aataaacaaa taggggttcc gcgcacattt ccccgaaaag tgccacctga 9660
   cgtctaagaa accattatta tcatgacatt aacctataaa aataggcgta tcacgaggcc 9720
   ctttcgtc 9728
<210> 32
   <211> 147
   <212> DNA
   <213> Artificial Sequence
<220>
   <223> Oligonucleotide RB252
<400> 32
   aattcaagaa acagtacaca tccattcatc atggtgtggt ggaggttgac gccgctgtca 60
   ccccagagga gcgccacctg tccaagatgc agcagaacgg ctacgaaaat ccaacctaca 120
   agttctttga gcagatgcag aactagg 147
<210> 33
   <211> 147
   <212> DNA
   <213> Artificial Sequence
<220>
   <223> Oligonucleotide RB253
<400> 33
   tcgacctagt tctgcatctg ctcaaagaac ttgtaggttg gattttcgta gccgttctgc 60
   tgcatcttgg acaggtggcg ctcctctggg gtgacagcgg cgtcaacctc caccacacca 120
   tgatgaatgg atgtgtactg tttcttg 147
<210> 34
   <211> 5879
   <212> DNA
   <213> Artificial Sequence
<220>
   <223> vecteur pRB35
<400> 34
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcaggcgcc 240
   attcgccatt caggctgcgc aactgttggg aagggcgatc ggtgcgggcc tcttcgctat 300
   tacgccagct ggcgaaaggg ggatgtgctg caaggcgatt aagttgggta acgccagggt 360
   tttcccagtc acgacgttgt aaaacgacgg ccagtgaatt cgagctcggt acccggggat 420
   cctctagagt cgacctgcag gcatgccggt agaggtgtgg tcaataagag cgacctcatg 480
   ctatacctga gaaagcaacc tgacctacag gaaagagtta ctcaagaata agaattttcg 540
   ttttaaaacc taagagtcac tttaaaattt gtatacactt atttttttta taacttattt 600
   aataataaaa atcataaatc ataagaaatt cgcccggaat tagcttggct gcaggtcgac 660
   ctagttctgc atctgctcaa agaacttgta ggttggattt tcgtagccgt tctgctgcat 720
   cttggacagg tggcgctcct ctggggtgac agcggcgtca acctccacca caccatgatg 780
   aatggatgtg tactgtttct tgaattccgg cgatacagtc aactgtcttt gacctttgtt 840
   actactctct tccgatgatg atgtcgcact tattctatgc tgtctcaatg ttagaggcat 900
   atcagtctcc actgaagcca atctatctgt gacggcatct ttattcacat tatcttgtac 960
   aaataatcct gttaacaatg cttttatatc ctgtaaagaa tccattttca aaatcatgtc 1020
   aaggtcttct cgaggaaaaa tcagtagaaa tagctgttcc agtctttcta gccttgattc 1080
   cacttctgtc agatgtgccc tagtcagcgg agaccttttg gttttgggag agtagcgaca 1140
   ctcccagttg ttcttcagac acttggcgca cttcggtttt tctttggagc acttgagctt 1200
   tttaagtcgg caaatatcgc atgcttgttc gatagaagac agtagcttca tctttcagga 1260
   ggcttgcttc aagcttggag ttgattgtat gcttggtata gcttgaaata ttgtgcagaa 1320
   aaagaaacaa ggaagaaagg gaacgagaac aatgacgagg aaacaaaaga ttaataattg 1380
   caggtctatt tatacttgat agcaagacag caaacttttt tttatttcaa attcaagtaa 1440
   ctggaaggaa ggccgtatac cgttgctcat tagagagtag tgtgcgtgaa tgaaggaagg 1500
   aaaaagtttc gtgtgcttcg agatacccct catcagctct ggaacaacga catctgttgg 1560
   tgctgtcttt gtcgttaatt ttttccttta gtgtcttcca tcattttttt gtcattgcgg 1620
   atatggtgag acaacaacgg gggagagaga aaagaaaaaa aaagaaaaga agttgcatgc 1680
   gcctattatt acttcaatag atggcaaatg gaaaaagggt agtgaaactt cgatatgatg 1740
   atggctatca agtctagggc tacagtatta gttcgttatg taccaccatc aatgaggcag 1800
   tgtaattggt gtagtcttgt ttagcccatt atgtcttgtc tggtatctgt tctattgtat 1860
   atctcccctc cgccacctac atgttaggga gaccaacgaa ggtattatag gaatcccgat 1920
   gtatgggttt ggttgccaga aaagaggaag tccatattgt acacccggaa acaacaaaag 1980
   gatatcgtac gcatagctgt ttcctgtgtg aaattgttat ccgctcacaa ttccacacaa 2040
   catacgagcc ggaagcataa agtgtaaagc ctggggtgcc taatgagtga gctaactcac 2100
   attaattgcg ttgcgctcac tgcccgcttt ccagtcggga aacctgtcgt gccagcagat 2160
   ctgggcaagt gcacaaacaa tacttaaata aatactactc agtaataacc tatttcttag 2220
   catttttgac gaaatttgct attttgttag agtcttttac accatttgtc tccacacctc 2280
   cgcttacatc aacaccaata acgccattta atctaagcgc atcaccaaca ttttctggcg 2340
   tcagtccacc agctaacata aaatgtaaac tttcggggct ctcttgcctt ccaacccagt 2400
   cagaaatcga gttccaatcc aaaagttcac ctgtcccacc tgcttctgaa tcaaacaagg 2460
   gaataaacga atgaggtttc tgtgaagctg cactgagtag tatgttgcag tcttttggaa 2520
   atacgagtct tttaataact ggcaaaccga ggaactcttg gtattcttgc cacgactcat 2580
   ctccatgcag ttggacgata tcaatgccgt aatcattgac cagagccaaa acatcctcct 2640
   taggttgatt acgaaacacg ccaaccaagt atttcggagt gcctgaacta tttttatatg 2700
   cttttacaag acttgaaatt ttccttgcaa taaccgggtc aattgttctc tttctattgg 2760
   gcacacatat aatacccagc aagtcagcat cggaatctag agcacattct gcggcctctg 2820
   tgctctgcaa gccgcaaact ttcaccaatg gaccagaact acctgtgaaa ttaataacag 2880
   acatactcca agctgccttt gtgtgcttaa tcacggtata tatactcacg tgctcaatag 2940
   tcaccaatgc cctccctctt ggccctctcc ttttcttttt tcgaccgaat taattcagat 3000
   ctgctgcatt aatgaatcgg ccaacgcgcg gggagaggcg gtttgcgtat tgggcgctct 3060
   tccgcttcct cgctcactga ctcgctgcgc tcggtcgttc ggctgcggcg agcggtatca 3120
   gcatcgatga atggaaatta cgtactttca acgcaagagc aagacaaaag aaagcctaaa 3180
   actgtctttt cgtgtcggtc gtccaatatc atcgtaaacg tgtgtagagc cttaaattct 3240
   aatttctaac tttaaatttt ttttgctatt ataatactaa tttctaactt tcaacctatt 3300
   ttacatcttc ggaaaacaaa aactattttt tctttaattt ctttttttac tttctatttt 3360
   taatttatat atttatatta aaaaatttaa attatattat ttttatagca cgtgatgaaa 3420
   agaaatatag tcttcaaggt tatattatac aggtttatat attattttac ttatttttgt 3480
   tgttcttttt cttggagtaa tttagcatta acaacttcga cccatcgcga tgacgaaatt 3540
   gcacgtttaa aggagaagtc ggaatcgttg caagaagaaa taaattagtt tgacgtttta 3600
   ttccttttct tctgcacaga agtgtgtata tatatattta tatatagtcg atgtgcaaaa 3660
   gcctacatta tttttctttt gatttttatt tttttgttca agtaggttca tgccgacagt 3720
   aaatccttat gccctaccta aatataaaca cccaaatata tatatatata tatatatata 3780
   taaataataa aaacggaaag gaaatagaat aataataata atactacacg tttacgtatg 3840
   taactcattc tattattcag actttgatat taatagaatt attactacta ggtttcctca 3900
   cttcaaaatc tataaatata ccgtcatgct ctaatcgatg ctcactcaaa ggtcggtaat 3960
   acggttatcc acagaatcag gggataacgc aggaaagaac atgtgagcaa aaggccagca 4020
   aaaggccagg aaccgtaaaa aggccgcgtt gctggcgttt ttccataggc tccgcccccc 4080
   tgacgagcat cacaaaaatc gacgctcaag tcagaggtgg cgaaacccga caggactata 4140
   aagataccag gcgtttcccc ctggaagctc cctcgtgcgc tctcctgttc cgaccctgcc 4200
   gcttaccgga tacctgtccg cctttctccc ttcgggaagc gtggcgcttt ctcatagctc 4260
   acgctgtagg tatctcagtt cggtgtaggt cgttcgctcc aagctgggct gtgtgcacga 4320
   accccccgtt cagcccgacc gctgcgcctt atccggtaac tatcgtcttg agtccaaccc 4380
   ggtaagacac gacttatcgc cactggcagc agccactggt aacaggatta gcagagcgag 4440
   gtatgtaggc ggtgctacag agttcttgaa gtggtggcct aactacggct acactagaag 4500
   gacagtattt ggtatctgcg ctctgctgaa gccagttacc ttcggaaaaa gagttggtag 4560
   ctcttgatcc ggcaaacaaa ccaccgctgg tagcggtggt ttttttgttt gcaagcagca 4620
   gattacgcgc agaaaaaaag gatctcaaga agatcctttg atcttttcta cggggtctga 4680
   cgctcagtgg aacgaaaact cacgttaagg gattttggtc atgagattat caaaaaggat 4740
   cttcacctag atccttttaa attaaaaatg aagttttaaa tcaatctaaa gtatatatga 4800
   gtaaacttgg tctgacagtt accaatgctt aatcagtgag gcacctatct cagcgatctg 4860
   tctatttcgt tcatccatag ttgcctgact ccccgtcgtg tagataacta cgatacggga 4920
   gggcttacca tctggcccca gtgctgcaat gataccgcga gacccacgct caccggctcc 4980
   agatttatca gcaataaacc agccagccgg aagggccgag cgcagaagtg gtcctgcaac 5040
   tttatccgcc tccatccagt ctattaattg ttgccgggaa gctagagtaa gtagttcgcc 5100
   agttaatagt ttgcgcaacg ttgttgccat tgctacaggc atcgtggtgt cacgctcgtc 5160
   gtttggtatg gcttcattca gctccggttc ccaacgatca aggcgagtta catgatcccc 5220
   catgttgtgc aaaaaagcgg ttagctcctt cggtcctccg atcgttgtca gaagtaagtt 5280
   ggccgcagtg ttatcactca tggttatggc agcactgcat aattctctta ctgtcatgcc 5340
   atccgtaaga tgcttttctg tgactggtga gtactcaacc aagtcattct gagaatagtg 5400
   tatgcggcga ccgagttgct cttgcccggc gtcaatacgg gataataccg cgccacatag 5460
   cagaacttta aaagtgctca tcattggaaa acgttcttcg gggcgaaaac tctcaaggat 5520
   cttaccgctg ttgagatcca gttcgatgta acccactcgt gcacccaact gatcttcagc 5580
   atcttttact ttcaccagcg tttctgggtg agcaaaaaca ggaaggcaaa atgccgcaaa 5640
   aaagggaata agggcgacac ggaaatgttg aatactcata ctcttccttt ttcaatatta 5700
   ttgaagcatt tatcagggtt attgtctcat gagcggatac atatttgaat gtatttagaa 5760
   aaataaacaa ataggggttc cgcgcacatt tccccgaaaa gtgccacctg acgtctaaga 5820
   aaccattatt atcatgacat taacctataa aaataggcgt atcacgaggc cctttcgtc 5879
<210> 35
   <211> 174
   <212> DNA
   <213> Artificial Sequence
<220>
   <223> oligonucleotide RB200
<400> 35
   ggccgcagtg aaccattgtt caatatcgtc cggggacagc atcaaatcat ccattgcttg 60
   ggacggcaag ggggacagaa cgttgttttc aggaagtagt ttccataggt ctgaaaatgt 120
   ttcctgactc agagggggct cgacgctagg atctgactgc ggctcctcca tctg 174
<210> 36
   <211> 174
   <212> DNA
   <213> Artificial Sequence
<220>
   <223> Oligonucleotide RB201
<400> 36
   gatccagatg gaggagccgc agtcagatcc tagcgtcgag ccccctctga gtcaggaaac 60
   attttcagac ctatggaaac tacttcctga aaacaacgtt ctgtccccct tgccgtccca 120
   agcaatggat gatttgatgc tgtccccgga cgatattgaa caatggttca ctgc 174
<210> 37
   <211> 44
   <212> DNA
   <213> Artificial Sequence
<220>
   <223> Oligonucleotide RB202
<400> 37
   cccgggaatt cagatccata tgtgcaatac caacatgtct gtac 44
<210> 38
   <211> 35
   <212> DNA
   <213> Artificial Sequence
<220>
   <223> Oligonucleotide RB203
<400> 38
   acttagagct ctaggggaaa taagttagca caatc 35